(19) Europäisches Patentamt
European Patent Office
Office européen des brevets

(11) EP 4 048 675 B1

(12) **EUROPÄISCHE PATENTSCHRIFT**

(45) Veröffentlichungstag und Bekanntmachung des Hinweises auf die Patenterteilung:
**08.04.2026 Patentblatt 2026/15**

(21) Anmeldenummer: **20792669.2**

(22) Anmeldetag: **22.10.2020**

(51) Internationale Patentklassifikation (IPC):
***C07D 491/06*** (2006.01) ***C07D 493/06*** (2006.01)
***C07D 495/06*** (2006.01) ***C09K 11/06*** (2006.01)

(52) Gemeinsame Patentklassifikation (CPC):
**C07D 493/06; C07D 491/06; C07D 495/06; C09K 11/06; H10K 85/626; H10K 85/654; H10K 85/657; H10K 85/6572; H10K 85/6574;**
H10K 50/11; H10K 2101/10; H10K 2101/90; Y02E 10/549

(86) Internationale Anmeldenummer:
**PCT/EP2020/079691**

(87) Internationale Veröffentlichungsnummer:
**WO 2021/078831 (29.04.2021 Gazette 2021/17)**

(54) **IN EINER ORGANISCHEN ELEKTRONISCHEN VORRICHTUNG EINSETZBARE VERBINDUNGEN**
COMPOUNDS THAT CAN BE USED IN AN ORGANIC ELECTRONIC DEVICE
COMPOSÉS POUVANT ÊTRE UTILISÉS DANS UN DISPOSITIF ÉLECTRONIQUE ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.10.2019 EP 19205306**

(43) Veröffentlichungstag der Anmeldung:
**31.08.2022 Patentblatt 2022/35**

(73) Patentinhaber: **Merck Patent GmbH**
**64293 Darmstadt (DE)**

(72) Erfinder:
- **STOESSEL, Philipp**
  **64293 DARMSTADT (DE)**
- **LACKNER, Aaron**
  **64293 DARMSTADT (DE)**
- **MEKIC, Amel**
  **9471 BUCHS (CH)**

(74) Vertreter: **Merck Patent Association**
**Merck Patent GmbH**
**64271 Darmstadt (DE)**

(56) Entgegenhaltungen:
**WO-A1-2010/151006**

- **NATALIYA N KARAUSH-KARMAZIN ET AL: "Impact of heteroatoms (S, Se, and Te) on the aromaticity of heterocirculenes", NEW JOURNAL OF CHEMISTRY, vol. 43, no. 30, 2 July 2019 (2019-07-02), GB, pages 12178 - 12190, XP055699588, ISSN: 1144-0546, DOI: 10.1039/ C9NJ02468A**
- **CARLOS A. MANZANO ET AL: "Heterocyclic Aromatics in Petroleum Coke, Snow, Lake Sediments, and Air Samples from the Athabasca Oil Sands Region", ENVIRONMENTAL SCIENCE & TECHNOLOGY, vol. 51, no. 10, 28 April 2017 (2017-04-28), US, pages 5445 - 5453, XP055586056, ISSN: 0013-936X, DOI: 10.1021/ acs.est.7b01345**

EP 4 048 675 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung beschreibt Verbindungen gemäß Anspruch 12 und 13, insbesondere zur Verwendung in elektronischen Vorrichtungen. Die Erfindung betrifft ferner ein Verfahren zur Herstellung der erfindungsgemäßen Verbindungen gemäß Anspruch 18. Die vorliegende Erfindung beschreibteine elektronische Vorrichtung gemäß Anspruch 1.

Der Aufbau organischer Elektrolumineszenzvorrichtungen, in denen organische Halbleiter als funktionelle Materialien eingesetzt werden, ist beispielsweise in US 4539507, US 5151629, EP 0676461, WO 98/27136 und WO 2010/151006 A1 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei Elektrolumineszenzvorrichtungen, insbesondere auch bei Elektrolumineszenzvorrichtungen, die Phosphoreszenz zeigen, immer noch Verbesserungsbedarf, beispielsweise im Hinblick auf Effizienz, Betriebsspannung und Lebensdauer. Ferner sind organische Elektrolumineszenzvorrichtungen bekannt, die fluoreszierende Emitter oder Emitter umfassen, die TADF (thermally activated delayed fluorescence) zeigen.

In Nataliya N Karaush-Karmazin et al, New Journal of Chemistry, Bd 43, Nr. 30, 2019 und Carlos A. Manzano et al, Environmental Science & Technology Bd. 51, Nr. 10, 2017 werden spezielle cyclische Verbindungen beschrieben.

Die Eigenschaften organischer elektrolumineszierender Vorrichtungen werden nicht nur durch die eingesetzten Emitter bestimmt. Hier sind insbesondere auch die anderen verwendeten Materialien, wie Host-/Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Generell besteht bei diesen Materialien, beispielsweise für die Verwendung als Matrixmaterialien, Lochtransportmaterialien oder Elektronentransportmaterialien noch Verbesserungsbedarf, insbesondere in Bezug auf die Lebensdauer, aber auch in Bezug auf die Effizienz und die Betriebsspannung der Vorrichtung. Ferner sollten die Verbindungen eine hohe Farbreinheit aufweisen.

Eine weitere Aufgabe der vorliegenden Erfindung ist die Bereitstellung von Verbindungen, welche sich für den Einsatz in einer organischen elektronischen Vorrichtung, insbesondere in einer organischen Elektrolumineszenzvorrichtung, als fluoreszierende Emitter oder Emitter eignen, die TADF (thermally activated delayed fluorescence) zeigen, und welche bei Verwendung in dieser Vorrichtung zu guten Device-Eigenschaften führen, sowie die Bereitstellung der entsprechenden elektronischen Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung, eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochtransportmaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot und gelb phosphoreszierende Elektrolumineszenzvorrichtungen eignen.

Weiterhin sollten die Verbindungen, insbesondere bei ihrem Einsatz als Matrixmaterialien, als Lochtransportmaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene elektronische Vorrichtungen und Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, sowie die entsprechenden bevorzugten Ausfürhungsformen sind daher Gegenstand der vorliegenden Erfindung.

Gegenstand der vorliegenden Erfindung ist daher eine elektronische Vorrichtung, enthaltend mindestens eine Verbindung, umfassend mindestens eine Struktur der Formel (Ia), vorzugsweise (Ib), (Ic), (Id), (Ie) und/oder (If),

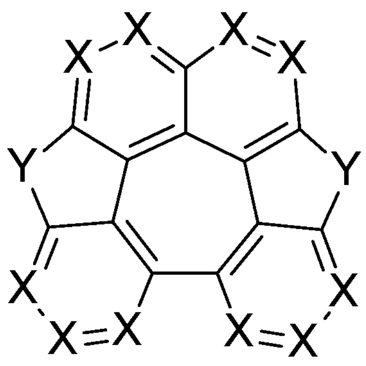

Formel (Ia)

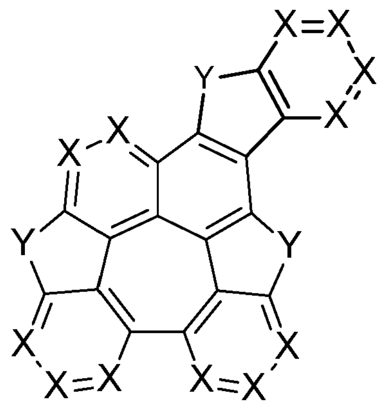

Formel (Ib)

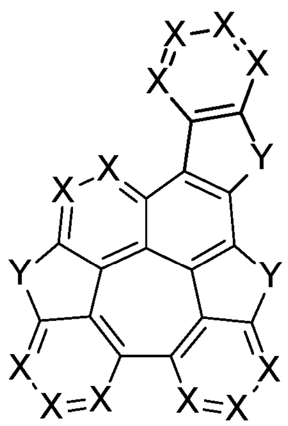

Formel (Ic)

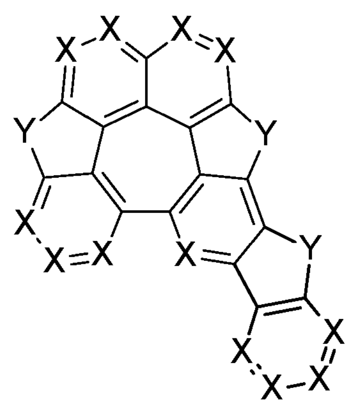

Formel (Id)

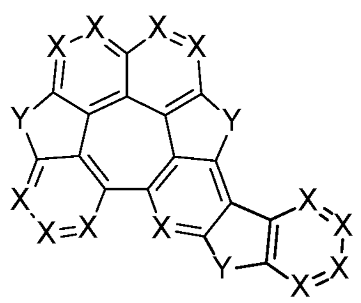

Formel (Ie)

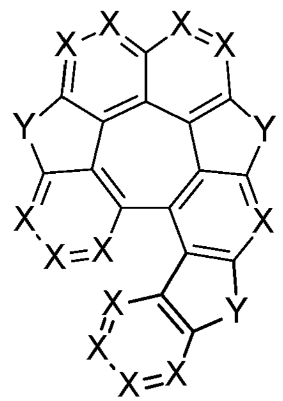

Formel (If),

wobei in den Formeln (Ia) bis (If) gilt:

X ist bei jedem Auftreten gleich oder verschieden CR, wobei höchstens 2 der Gruppen CR, für die X steht, ungleich der Gruppe CH ist;

Y ist bei jedem Auftreten gleich oder verschieden eine Brücke, ausgewählt aus O, S, B(R), C=O, N(R) und N(Ar), besonders bevorzugt O, S, N(Ar);

R ist bei jedem Auftreten gleich oder verschieden H, D, F, CN, $N(Ar)_2$, $N(R^1)_2$, $Si(Ar)_3$, $Si(R^1)_3$, $B(Ar)_2$, $B(R^1)_2$, eine geradkettige Alkyl-, oder Alkoxygruppe mit 1 bis 40 C-Atomen oder eine verzweigte oder cyclische Alkyl-, oder Alkoxygruppe mit 3 bis 20 C-Atomen, wobei die Alkyl-, oder Alkoxygruppe jeweils mit einem oder mehreren Resten $R^1$ substituiert sein kann, oder ein aromatisches oder heteroaromatisches Ringsystem mit 5 bis 60 aromatischen Ringatomen, das jeweils durch einen oder mehrere Reste $R^1$ substituiert sein kann; dabei können zwei Reste R auch miteinander ein Ringsystem bilden;

Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann, dabei können zwei Reste Ar, welche an dasselbe Si-Atom, N-Atom oder B-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, $N(R^1)$, $P(R^1)$ und $P(=O)R^1$, miteinander verbrückt sein;

$R^1$ ist bei jedem Auftreten gleich oder verschieden H, D, F, CN, $N(Ar^1)_2$, $N(R^2)_2$, $B(Ar^1)_2$, $B(R^2)_2$, $Si(Ar^1)_3$, $Si(R^2)_3$, eine geradkettige Alkyl-, oder Alkoxygruppe mit 1 bis 40 C-Atomen oder eine verzweigte oder cyclische Alkyl-, oder Alkoxygruppe mit 3 bis 40 C-Atomen, die jeweils mit einem oder mehreren Resten $R^2$ substituiert sein kann, wobei ein oder mehrere H-Atome durch D ersetzt sein können, oder ein aromatisches oder heteroaromatisches Ringsystem mit 5 bis 60 aromatischen Ringatomen, das jeweils durch einen oder mehrere Reste $R^2$ substituiert sein kann; dabei können zwei oder mehrere, vorzugsweise benachbarte Reste $R^1$ miteinander ein Ringsystem bilden; dabei können einer oder mehrere Reste $R^1$ mit einem weiteren Teil der Verbindung ein Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann, dabei können zwei Reste $Ar^1$, welche an dasselbe Si-Atom, N-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, $N(R^2)$, $P(R^2)$ und $P(=O)R^2$, miteinander verbrückt sein;

$R^2$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D ersetzt sein können und das durch ein oder mehrere Alkylgruppen mit jeweils 1 bis 4 Kohlenstoffatomen substituiert sein kann; dabei können zwei oder mehrere, vorzugsweise benachbarte Substituenten $R^2$ miteinander ein Ringsystem bilden.

Gegenstand der vorliegenden Erfindung sind auch Verbindungen gemäß Anspruch 12 und Anspruch 13.

Vorzugsweise können die zuvor genannten Verbindungen als aktive Verbindung in elektronischen Vorrichtungen eingesetzt werden. Aktive Verbindungen sind generell die organischen oder anorganischen Materialien, welche beispielsweise in einer organischen elektronischen Vorrichtung, insbesondere in einer organischen Elektrolumineszenzvorrichtung zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Hierbei sind organische Materialien bevorzugt.

Vorzugsweise ist eine erfindungsgemäße Verbindung gemäß Anspruch 12 oder 13 eine rein organische Verbindung. Eine rein organische Verbindung ist eine Verbindung, die nicht mit einem Metallatom in Verbindung steht, also weder mit einem Metallatom eine Koordinationsverbindung bildet, noch mit einem Metallatom eine kovalente Bindung ausbildet. Hierbei umfasst eine rein organische Verbindung vorzugsweise kein Metallatom, welches in Phosphoreszenzemittern eingesetzt werden. Diese Metalle, wie Kupfer, Molybdän, usw. insbesondere Rhenium, Ruthenium, Osmium, Rhodium, Iridium, Palladium, werden später ausführlich dargestellt.

Die Verbindung, die einer organischen elektronischen Vorrichtung als aktive Verbindung einsetzbar ist, kann bevorzugt ausgewählt sein aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Excitonenblockiermaterialien, Elektroneninjektionsmaterialien, Lochtransportmaterialien, Lochinjektionsmaterialien, n-Dotanden, p-Dotanden, Wide-Band-Gap-Materialien, Elektronenblockiermaterialien und/oder Lochblockiermaterialien. Hierbei sind fluoreszierende Emitter, Emitter, die TADF (thermally activated delayed fluorescence) zeigen, Hostmaterialien, Elektronentransportmaterialien, Excitonenblockiermaterialien, Elektroneninjektionsmaterialien, Lochtransportmaterialien, Lochinjektionsmaterialien, n-Dotanden, p-Dotanden, Wide-Band-Gap-Materialien, Elektronenblockiermaterialien und/oder Lochblockiermaterialien bevorzugt.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

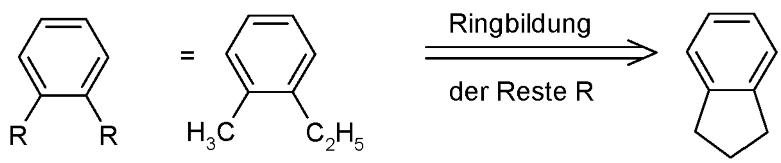


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

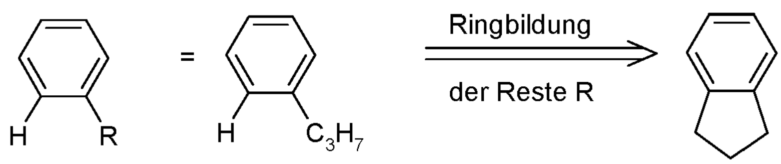


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Falls zwei oder mehrere, vorzugsweise benachbarte Reste R, $R^1$ und/oder $R^2$ miteinander ein Ringsystem bilden, so kann ein monocyclisches oder polycyclisches, aliphatisches, aromatisches oder heteroaromatisches Ringsystem entstehen.

Eine Arylgruppe im Sinne dieser Erfindung enthält 6 bis 60 C-Atome, vorzugsweise 6 bis 40 C-Atome, besonders bevorzugt 6 bis 30 C-Atome; eine Heteroarylgruppe im Sinne dieser Erfindung enthält 2 bis 60 C-Atome, vorzugsweise 2 bis 40 C-Atome, besonders bevorzugt 2 bis 30 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome, besonders bevorzugt 6 bis 30 C-Atome im Ringsystem. Ein heteroaromatisches Ringsystem im Sinne dieser Erfindung enthält 1 bis 60 C, vorzugsweise 1 bis 40 C-Atome, besonders bevorzugt 1 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin,

ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60, vorzugsweise 5 - 40 aromatischen Ringatomen, besonders bevorzugt 5 bis 30 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausgestaltung kann die erfindungsgemäße elektronische Vorrichtung mindestens eine Verbindung umfassend eine Struktur der Formeln (IIa), (IIb), (IIc), (IId), (IIe) und/oder (IIf) enthalten, vorzugsweise weist die Verbindung eine Struktur der genannten Formeln auf,

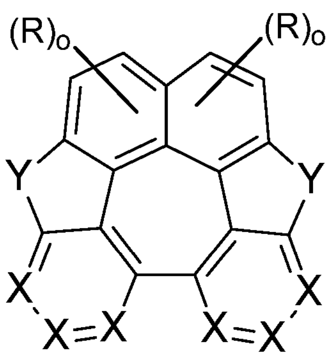

Formel (IIa)

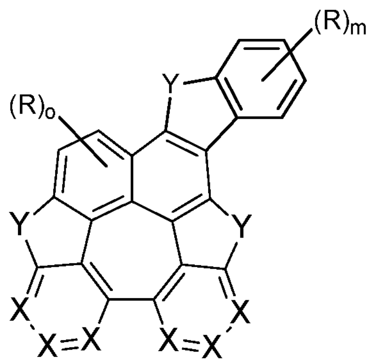

Formel (IIb)

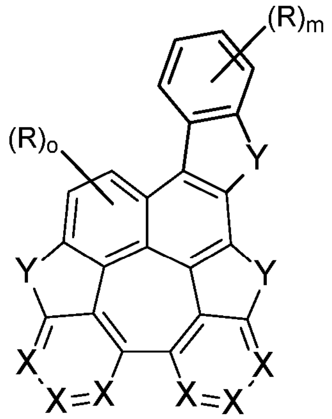

Formel (IIc)

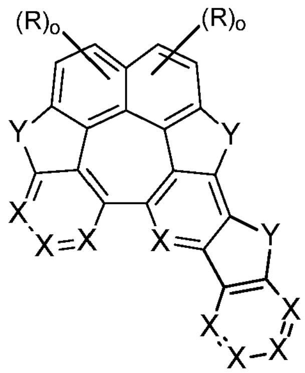

Formel (IId)

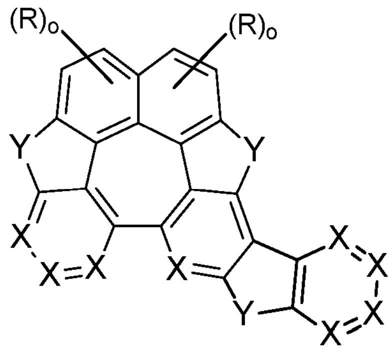

Formel (IIe)

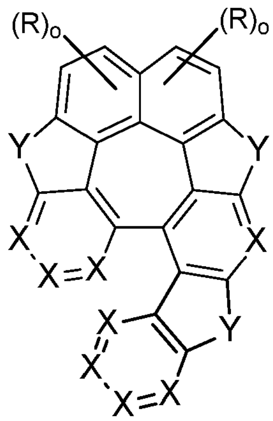

Formel (IIf),

wobei die Reste X, Y und R die zuvor genannte Bedeutung haben, der Index m gleich oder verschieden 0, 1, 2, 3 oder 4, bevorzugt 0, 1, 2 oder 3, vorzugsweise 0, 1 oder 2, besonders bevorzugt 0 oder 1 ist und der Index o gleich oder verschieden 0, 1 oder 2, vorzugsweise 0 oder 1 ist, wobei die Summe der Indices o beziehungsweise o und m vorzugsweise 1 oder 2, besonders bevorzugt 1 ist.

Vorzugsweise kann vorgesehen sein, dass die elektronische Vorrichtung mindestens eine Verbindung umfassend eine Struktur der Formeln (IIIa), (IIIb), (IIIc), (IIId), (IIIe) und/oder (IIIf) enthält, vorzugsweise weist die Verbindung eine Struktur der genannten Formeln auf,

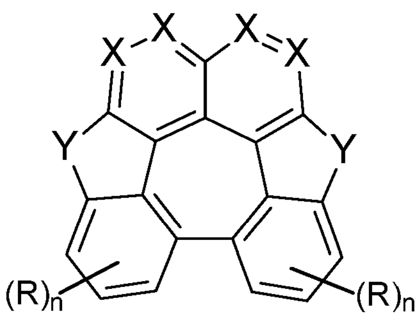

Formel (IIIa)

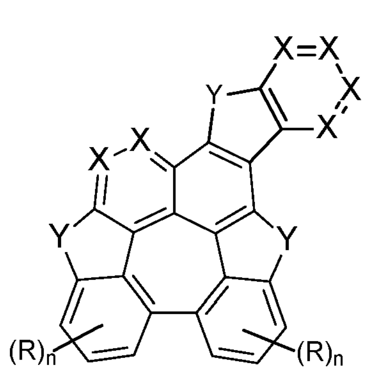

Formel (IIIb)

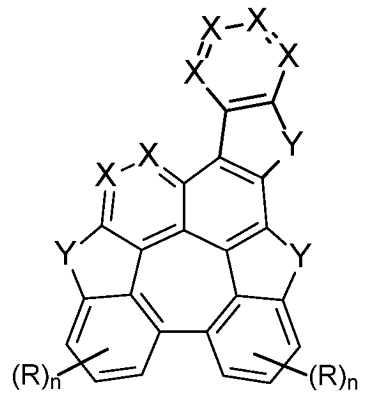

Formel (IIIc)

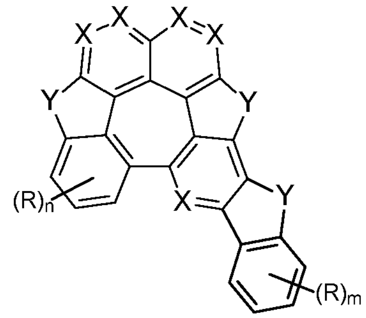

Formel (IIId)

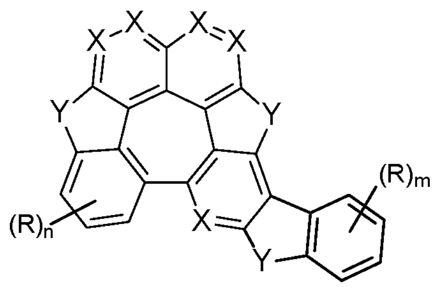

Formel (IIIe)

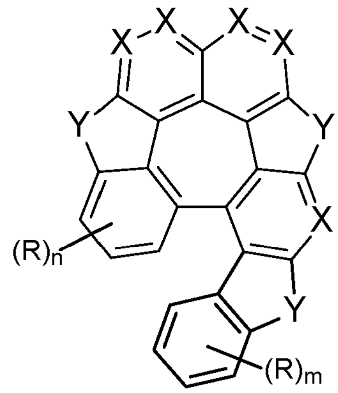

Formel (IIIf),

wobei die Reste X, Y und R die zuvor genannte Bedeutung haben, der Index m gleich oder verschieden 0, 1, 2, 3 oder 4, bevorzugt 0, 1, 2 oder 3, vorzugsweise 0, 1 oder 2, besonders bevorzugt 0 oder 1 ist und der Index n gleich oder verschieden 0, 1, 2 oder 3, vorzugsweise 0, 1 oder 2, besonders bevorzugt 0 oder 1 ist, wobei die Summe der Indices n beziehungsweise n und m vorzugsweise 1 oder 2, besonders bevorzugt 1 ist.

Es ist vorgesehen, dass in Formeln (Ia) bis (If), (IIa) bis (IIf) und/oder (IIIa) bis (IIIf) höchstens 2 der Gruppen CR, für die X steht, ungleich der Gruppe CH ist.

In einer weiteren Ausführungsform der elektronischen Vorrichtung istvorgesehen, dass die elektronische Vorrichtung mindestens eine Verbindung umfassend mindestens eine Struktur der Formeln (IVa), (IVb), (IVc), (IVd), (IVe) und/oder (IVf) enthält, vorzugsweise weist die Verbindung eine Struktur der genannten Formeln auf,

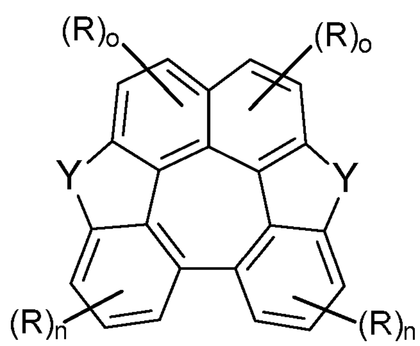

Formel (IVa)

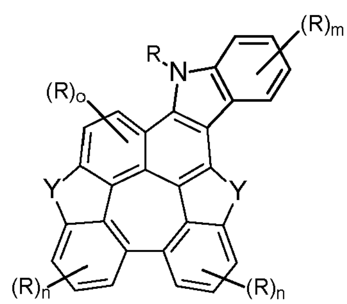

Formel (IVb)

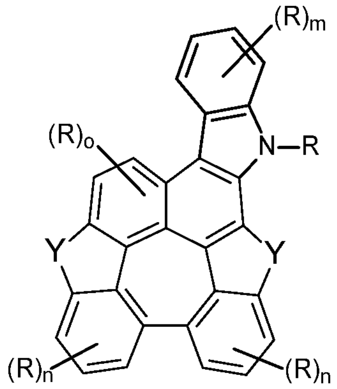

Formel (IVc)

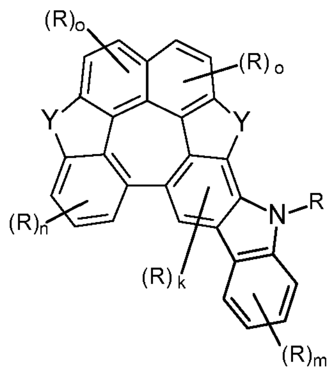

Formel (IVd)

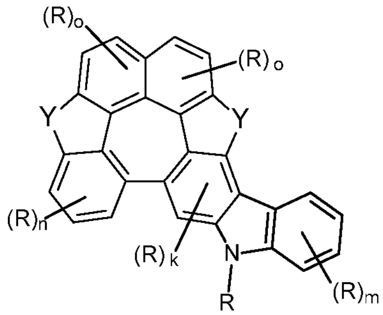

Formel (IVe)

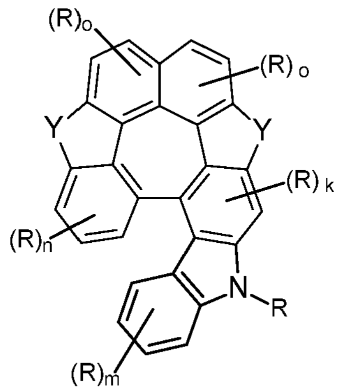

Formel (IVf),

wobei die Reste Y und R die zuvor, insbesondere für Formel (Ia) bis (If) genannte Bedeutung haben, der Index k gleich oder verschieden 0 oder 1, vorzugsweise 0 ist, der Index o gleich oder verschieden 0, 1 oder 2, vorzugsweise 0 oder 1 ist, der Index n gleich oder verschieden 0, 1, 2 oder 3, vorzugsweise 0, 1 oder 2, besonders bevorzugt 0 oder 1 ist und der Index m gleich oder verschieden 0, 1, 2, 3 oder 4, bevorzugt 0, 1, 2 oder 3, vorzugsweise 0, 1 oder 2, besonders bevorzugt 0 oder 1 ist, wobei die Summe der Indices k, m, n und o vorzugsweise 1 oder 2, besonders bevorzugt 1 ist.

Vorzugsweise kann vorgesehen sein, dass die Summe der Indices k, m, n und o in den Formeln (IIa) bis (IIf), (IIIa) bis (IIIf) und/oder (IVa) bis (IVf) höchstens 6, vorzugsweise höchstens 4, besonders bevorzugt höchstens 2 ist. Weiterhin ist bevorzugt, dass die Summe der Indices o und n in den Formeln (IIa) bis (IIf), (IIIa) bis (IIIf) und/oder (IVa) bis (IVf) mindestens 1 ist. Besonders bevorzugt beträgt die Summe der Indices o und n in den Formeln (IIa) bis (IIf), (IIIa) bis (IIIf) und/oder (IVa) bis (IVf) 1 oder 2 und speziell bevorzugt genau 1.

Weiterhin kann vorgesehen sein, dass die elektronische Vorrichtung mindestens eine Verbindung umfassend eine Struktur der Formeln (Va), (Vb), (Vc), (Vd), (Ve), (Vf), (Vg), (Vh), (Vi), (Vj) und/oder (Vk) umfasst, vorzugsweise weist die Verbindung eine Struktur der genannten Formeln auf,

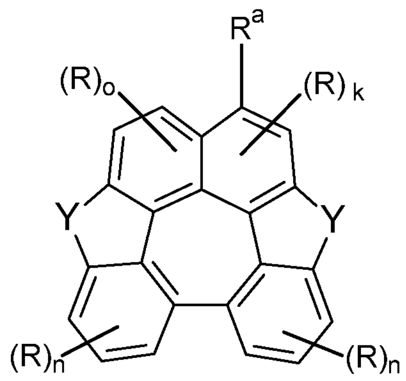

Formel (Va)

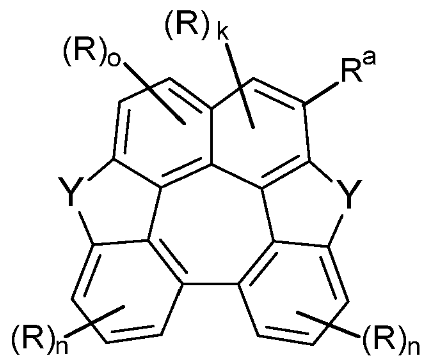

Formel (Vb)

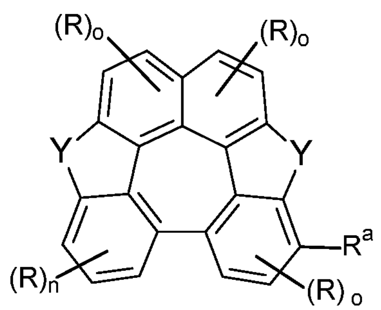

Formel (Vc)

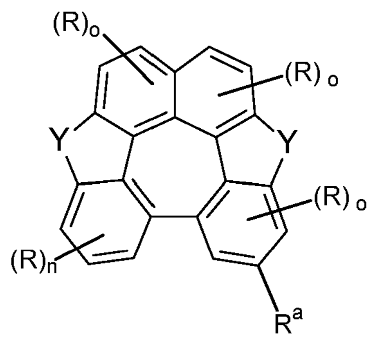

Formel (Vd)

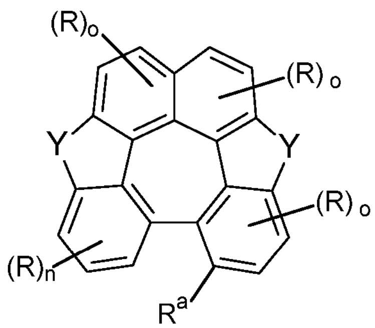

Formel (Ve)

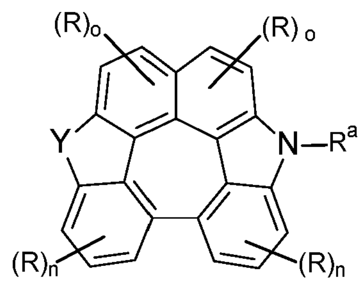

Formel (Vf)

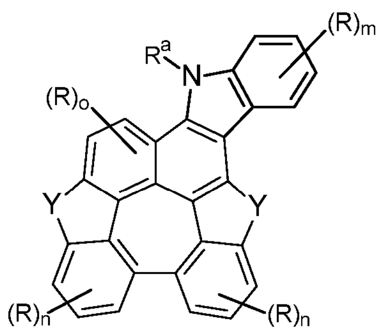

Formel (Vg)

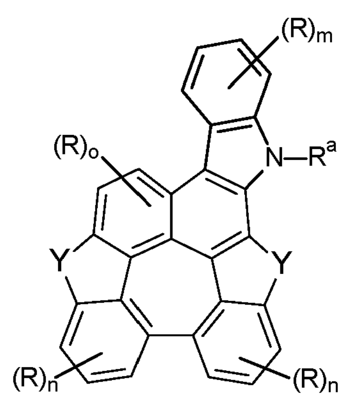

Formel (Vh)

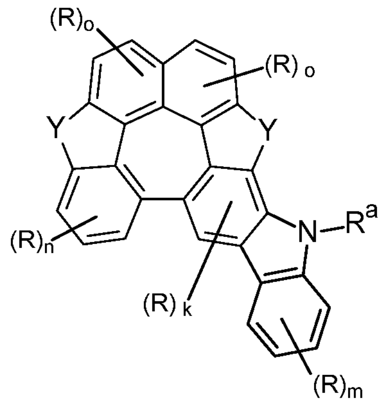

Formel (Vi)

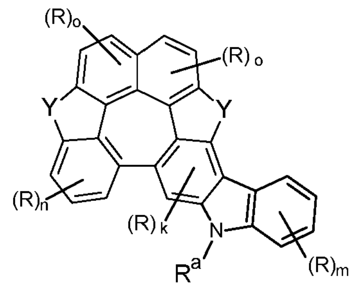

Formel (Vj)

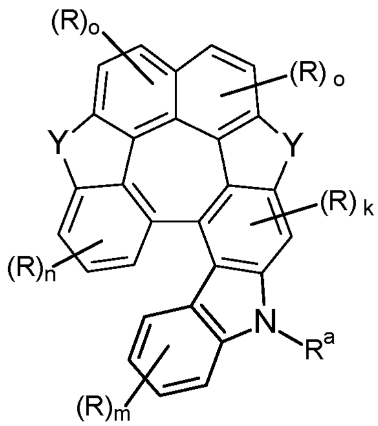

Formel (Vk),

wobei die Reste Y und R die zuvor genannte Bedeutung haben und weiterhin gilt:

$R^a$ ist bei jedem Auftreten gleich oder verschieden F, CN, $N(Ar)_2$, $N(R^1)_2$, $Si(Ar)_3$, $Si(R^1)_3$, $B(Ar)_2$, $B(R^1)_2$, eine geradkettige Alkyl-, oder Alkoxygruppe mit 1 bis 20 C-Atomen oder eine verzweigte oder cyclische Alkyl-, oder Alkoxygruppe mit 3 bis 40 C-Atomen, wobei die Alkyl-, oder Alkoxygruppe jeweils mit einem oder mehreren Resten $R^1$ substituiert sein kann, oder ein aromatisches oder heteroaromatisches Ringsystem mit 5 bis 60 aromatischen Ringatomen, das jeweils durch einen oder mehrere Reste $R^1$ substituiert sein kann; dabei können Reste $R^a$ auch mit einem Rest R ein Ringsystem bilden, wobei die Reste Ar und $R^1$ die zuvor genannte Bedeutung haben;

k ist gleich oder verschieden 0 oder 1, vorzugsweise 0,

o ist gleich oder verschieden 0, 1 oder 2, vorzugsweise 0 oder 1;

n ist gleich oder verschieden 0, 1, 2 oder 3, vorzugsweise 0, 1 oder 2, besonders bevorzugt 0 oder 1; und

m ist gleich oder verschieden 0, 1, 2, 3 oder 4, vorzugsweise 0, 1 oder 2, besonders bevorzugt 0 oder 1.

Vorzugsweise kann vorgesehen sein, dass in Formeln (Va) bis (Vk) die Summe der Indices k, m, n und o höchstens 6, vorzugsweise höchstens 4, besonders bevorzugt höchstens 2, speziell bevorzugt höchstens 1 beträgt und ganz besonders bevorzugt 0 ist.

In einer bevorzugten Ausführungsform kann vorgesehen sein, dass die zwei Reste Y in den zuvor und nachfolgend dargelegten Formeln gleich sind. Ferner kann in einer weiteren bevorzugten Ausführungsform vorgesehen sein, dass die zwei Reste Y in den zuvor und nachfolgend dargelegten Formeln verschieden sind.

Vorzugsweise ist mindestens einer der Reste R und/oder $R^a$ ausgewählt aus der Gruppe der Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene, Anthracene, Benzanthracene, Fluoradene.

Fermer kann vorzugsweise vorgesehen sein, dass ein direkt an ein Stickstoffatom gebundener Rest R keine

Gruppe darstellt, die ausgewählt ist aus F, CN, $N(Ar)_2$, $N(R^1)_2$, wobei $R^1$ die zuvor, insbesondere für Formel (Ia) bis (If) genannte Bedeutung hat. Demgemäß stellt in den Formeln (Vf) bis (Vk) die Gruppe $R^a$ bevorzugt keinen F-, CN-, $N(Ar)_2$-, $N(R^1)_2$-Rest dar, wobei $R^1$ die zuvor, insbesondere für Formel (Ia) bis (If) genannte Bedeutung hat. In einer weiterhin bevorzugten Ausführungsform weisen die Strukturen der Formeln (Ia) bis (If) sowie die zuvor und nachfolgend dargelegten bevorzugten Ausgestaltungen dieser Strukturen keine N-N-Bindung auf.

Ferner kann vorgesehen sein, dass mindestens einer der Reste R und/oder $R^a$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 9,9'-Diaryl-Fluorenyl 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imidazolyl, Benzimidazolyl, Benzoxazolyl, Benzthiazolyl, 1-, 2-, 3- oder 4-Carbazolyl, 1- oder 2-Napthyl, Anthracenyl, vorzugsweise 9-Anthracenyl, Trans- und cis-Indenofluorenyl, Indenocarbazolyl, Indolocarbazolyl, Spirocarbazolyl, 5-Aryl-Phenanthridin-6-on-yl, 9,10-Dehydrophenanthrenyl, Fluoranthenyl, Tolyl, Mesityl, Phenoxytolulyl, Anisolyl, Triarylaminyl, Bis-triarylaminyl, Tristriarylaminyl, Hexamethylindanyl, Tetralinyl, Monocycloalkyl, Biscycloalkyl, Tricycloalkyl, Alkyl, wie z.B. tert-Butyl, Methyl, Propyl, Alkoxyl, Alkylsulfanyl, Alkylaryl, Triarylsilyl, Trialkylsilyl, Xanthenyl, 10-Aryl-Phenoxazinyl, Phenanthrenyl und/oder Triphenylenyl, die jeweils durch einen oder mehrere Reste substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen- Gruppen besonders bevorzugt sind.

Wenn die zuvor und nachfolgend dargelegten Strukturen durch Substituenten R und/oder $R^a$ substituiert sind, dann sind diese Substituenten R und/oder $R^a$ bevorzugt gewählt aus der Gruppe bestehend aus H, D, F, CN, $N(Ar)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten $R^1$ substituiert sein kann, einem aromatischen oder heteroaromatischen Ringsystem mit 5 bis 24 aromatischen Ringatomen, das jeweils mit einem oder mehreren Resten $R^1$ substituiert sein kann, bevorzugt aber unsubstituiert ist; dabei können optional zwei Substituenten R und/oder $R^a$, die vorzugsweise an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann, wobei die Gruppe Ar die zuvor, insbesondere für Formel (Ia) bis (If) genannte Bedeutung aufweist.

Besonders bevorzugt sind diese Substituenten R und/oder $R^a$ ausgewählt aus der Gruppe bestehend aus H, D, F, CN, $N(Ar)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, die jeweils mit einem oder mehreren Resten $R^1$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, wobei Ar die zuvor dargelegte Bedeutung aufweisen kann.

Besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^1$ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, Triazinyl, Chinazolinyl, Chinoxalinyl, Chinolinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^1$ substituiert sein können, bevorzugt aber unsubstituiert sind.

Ganz besonders bevorzugt sind die Substituenten $R^a$ ausgewählt aus der Gruppe bestehend aus einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^1$ substituiert sein kann, bevorzugt aber unsubstituiert ist. Beispiele für geeignete Substituenten $R^a$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, Triazinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^1$ substituiert sein können, bevorzugt aber unsubstituiert sind.

Weiterhin kann vorgesehen sein, dass die Substitutenten R und/oder $R^a$ der zuvor und nachfolgend dargelegten Strukturen, vorzugsweise Strukturen gemäß den Formeln (Ia) bis (If), (IIa) bis (IIf), (IIIa) bis (IIIf), (IVa) bis (IVf) und/oder (Va) bis (Vk) untereinander kein kondensiertes aromatisches oder heteroaromatisches Ringsystem, vorzugsweise kein kondensiertes Ringsystem bilden.

Gemäß einer weiteren Ausführungsform kann vorgesehen sein, dass die elektronische Vorrichtung mindestens eine Verbindung enthält, die eine Lochtransportgruppe umfasst, wobei vorzugsweise mindestens eine der zuvor dargelegten Gruppen R und/oder $R^a$, die unter anderem in einer Struktur gemäß den Formeln (Ia) bis (If), (IIa) bis (IIf), (IIIa) bis (IIIf), (IVa) bis (IVf) und/oder (Va) bis (Vk) enthalten sein können, eine Lochtransportgruppe umfasst, vorzugsweise darstellt. Lochtransportgruppen sind in der Fachwelt bekannt, wobei diese vorzugsweise Triarylamin- oder Carbazolgruppen umfassen.

Vorzugsweise kann vorgesehen sein, dass die Lochtransportgruppe eine Gruppe umfasst, bevorzugt für eine Gruppe steht, die ausgewählt ist aus den Formeln (H-1) bis (H-3),

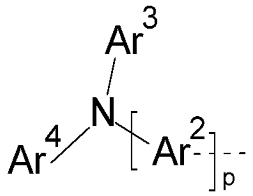

Formel (H-1)

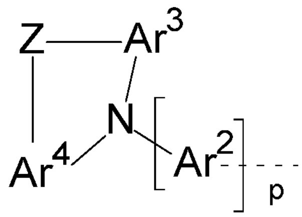

Formel (H-2)

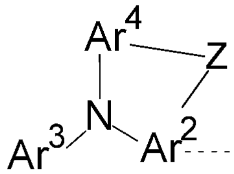

Formel (H-3),

wobei die gestrichelte Bindung die Anbindungsposition markiert und die Symbole die folgende Bedeutung aufweisen;

$Ar^2$, $Ar^3$, $Ar^4$ ist jeweils unabhängig ein aromatisches Ringsystem mit 6 bis 40 C-Atomen oder ein heteroaromatisches Ringsystem mit 3 bis 40 C-Atomen, welche jeweils durch einen oder mehrere Reste $R^1$ substituiert sein kann;

p ist 0 oder 1;

Z steht für eine Bindung oder $C(R^1)_2$, $Si(R^1)_2$, C=O, $NR^1$, N-$Ar^1$, $BR^1$, $PR^1$, $PO(R^1)$, SO, $SO_2$, Se, O oder S, vorzugsweise für eine Bindung oder $C(R^1)_2$, N-$Ar^1$, O oder S;

wobei die Symbole $Ar^1$ und $R^1$ die zuvor, insbesondere für Formel (Ia) bis (If) genannte Bedeutung aufweisen. Hierbei ist das Vorhandensein einer N-N-Bindung vorzugsweise ausgeschlossen.

Weiterhin kann vorgesehen sein, dass die Lochtransportgruppe eine Gruppe umfasst, bevorzugt für eine Gruppe steht, die ausgewählt ist aus den Formeln (H-4) bis (H-26),

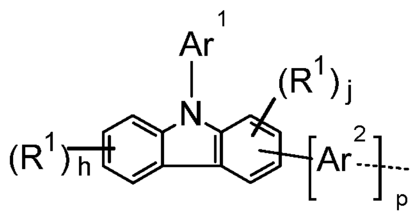

Formel (H-4)

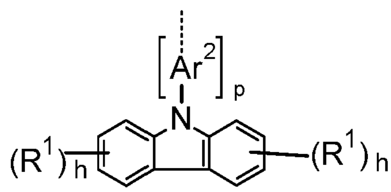

Formel (H-5)

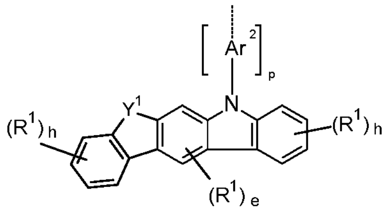

Formel (H-6)

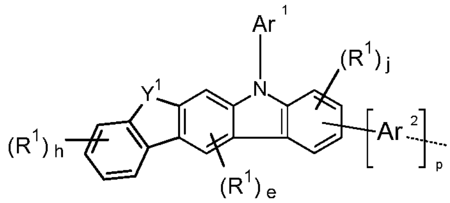

Formel (H-7)

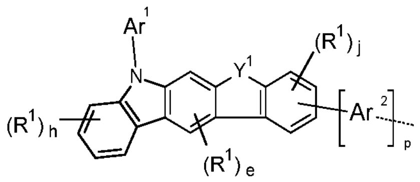

Formel (H-8)

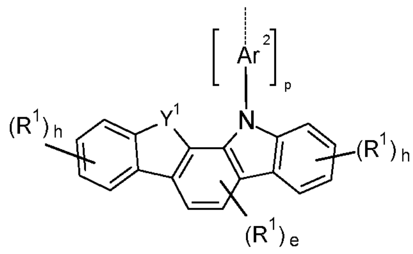

Formel (H-9)

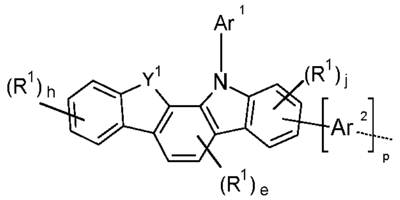

Formel (H-10)

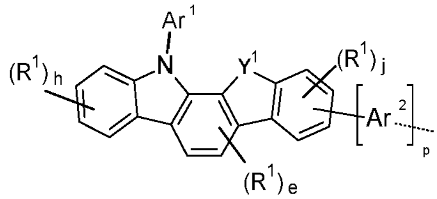

Formel (H-11)

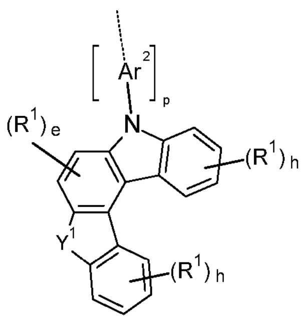

Formel (H-12)

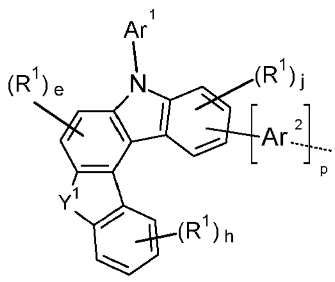

Formel (H-13)

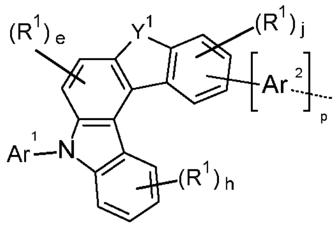

Formel (H-14)

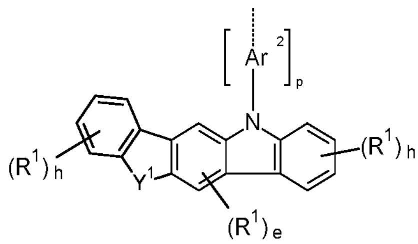

Formel (H-15)

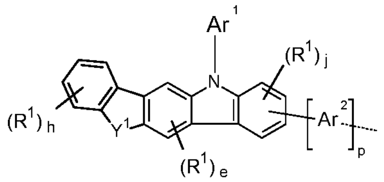

Formel (H-16)

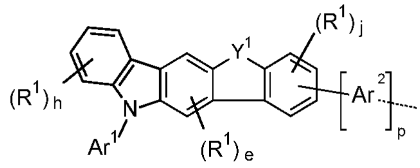

Formel (H-17)

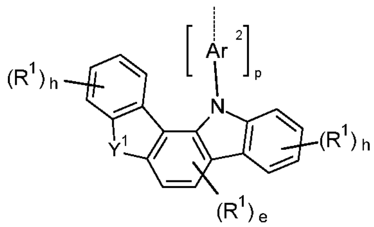

Formel (H-18)

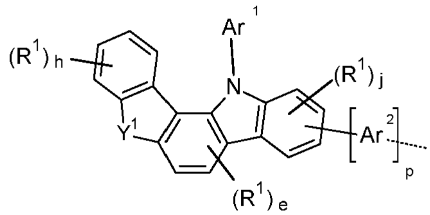

Formel (H-19)

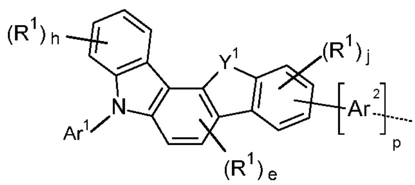

Formel (H-20)

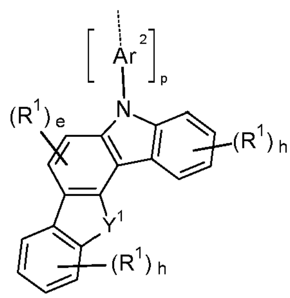

Formel (H-21)

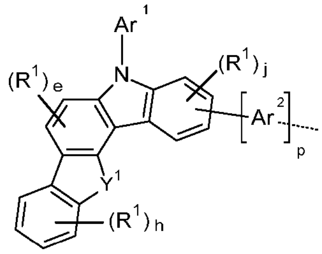

Formel (H-22)

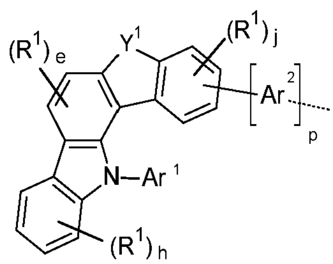

Formel (H-23)

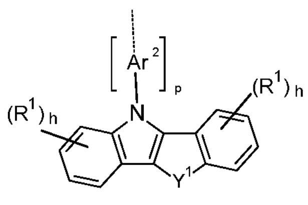

Formel (H-24)

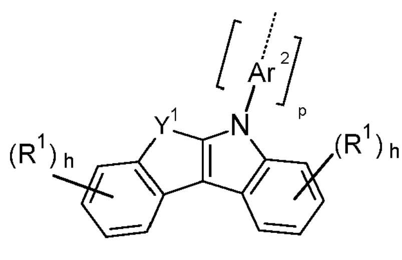

Formel (H-25)

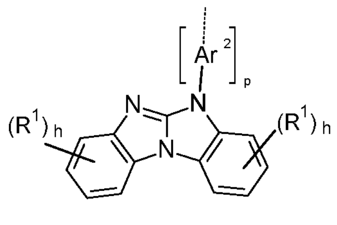

Formel (H-26),

wobei $Y^1$ O, S, $C(R^1)_2$, $NR^1$ oder $NAr^1$ darstellt, die gestrichelte Bindung die Anbindungsposition markiert, e 0, 1 oder 2 ist, j 0, 1, 2 oder 3 ist, h gleich oder verschieden bei jedem Auftreten 0, 1, 2, 3 oder 4 ist, p 0 oder 1 ist, $Ar^1$ und $R^1$ die zuvor, insbesondere für Formel (Ia) bis (If) und $Ar^2$ die zuvor, insbesondere für Formel (H-1) oder (H-2) genannten Bedeutungen aufweisen. Hierbei ist das Vorhandensein einer N-N-Bindung vorzugsweise ausgeschlossen.

Aus der obigen Formulierung ist ersichtlich, dass, falls der Index p = 0 ist, die entsprechende Gruppe $Ar^2$ nicht vorhanden ist und eine Bindung gebildet wird.

Bevorzugt kann die Gruppe $Ar^2$ mit dem aromatischen oder heteroaromatischen Rest oder dem Stickstoffatom, an den die Gruppe $Ar^2$ gemäß den Formeln (H-1) bis (H-26) gebunden sein kann, eine durchgängige Konjugation ausbilden.

In einer weiteren bevorzugten Ausführungsform steht $Ar^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (Ia) bis (If) genannte Bedeutung aufweisen kann. Besonders bevorzugt steht $Ar^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formel (Ia) bis (If) genannte Bedeutung aufweisen kann.

Weiterhin bevorzugt steht das unter anderem in Formeln (H-1) bis (H-26) dargelegte Symbol $Ar^2$ für einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (H-1) bis (H-26) dargelegte Gruppe $Ar^2$ ein aromatisches Ringsystem mit höchstens zwei kondensierten aromatischen und/oder heteroaromatischen 6-Ringen, vorzugsweise kein kondensiertes aromatisches oder heteroaromatisches Ringsystem mit kondensierten 6-Ringen umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt. Besonders bevorzugt sind Strukturen, die keine Kondensation aufweisen, wie beispielsweise Phenyl-, Biphenyl-, Terphenyl- und/oder Quaterphenyl-Strukturen.

Ferner kann vorgesehen sein, dass die unter anderem in Formeln (H-1) bis (H-26) dargelegte Gruppe $Ar^2$ höchstens 1 Stickstoffatom, bevorzugt höchstens 2 Heteroatome, insbesondere bevorzugt höchstens ein Heteroatom und besonders bevorzugt kein Heteroatom aufweist.

In einer weiteren bevorzugten Ausführungsform der Erfindung steht $Ar^3$ und/oder $Ar^4$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (Ia) bis (If) dargestellte Bedeutung aufweisen kann.

Gemäß einer weiteren Ausführungsform kann vorgesehen sein, dass die erfindungsgemäße Vorrichtung mindestens eine Verbindung umfasst, die einen Elektronentransportgruppe-umfassenden Rest umfasst, wobei vorzugsweise mindestens eine der zuvor dargelegten Gruppen R und/oder $R^a$, die unter anderem in einer Struktur gemäß den Formeln (Ia) bis (If), (IIa) bis (IIf), (IIIa) bis (IIIf), (IVa) bis (IVf) und/oder (Va) bis (Vk) enthalten sein können, einen Elektronentransportgruppe-umfassenden Rest umfasst, vorzugsweise darstellt. Elektronentransportgruppen sind in der Fachwelt weithin bekannt und fördern die Fähigkeit von Verbindungen, Elektronen zu transportieren und/oder zu leiten.

Weiterhin zeigen erfindungsgemäße Vorrichtungen enthaltend mindestens eine Verbindung überraschende Vorteile, wenn diese Verbindung mindestens eine Struktur umfasst, die aus der Gruppe Pyridine, Pyrimidine, Pyrazine, Pyridazine, Triazine, Chinazoline, Chinoxaline, Chinoline, Isochinoline, Imidazole und/oder Benzimidazole ausgewählt ist, wobei Pyrimidine, Triazine und Chinazoline besonders bevorzugt sind. Diese Strukturen fördern im Allgemeinen die

Fähigkeit von Verbindungen, Elektronen zu transportieren und/oder zu leiten.

In einer bevorzugten Ausgestaltung der vorliegenden Erfindung kann vorgesehen sein, dass der Elektronentransportgruppe-umfassende Rest für eine Gruppe steht, die durch die Formel (QL) darstellbar ist,

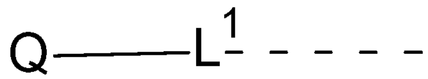

Formel (QL),

worin L’ eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, Q eine Elektronentransportgruppe ist, wobei $R^1$ die zuvor, insbesondere für Formel (Ia) bis (If) genannte Bedeutung aufweist, und die gestrichelte Bindung die Anbindungsposition markiert.

Bevorzugt kann die Gruppe $L^1$ mit der Gruppe Q und dem Atom, bevorzugt dem Kohlen- oder Stickstoffatom, an den die Gruppe $L^1$ gemäß Formel (QL) gebunden ist, eine durchgängige Konjugation ausbilden. Eine durchgängige Konjugation der aromatischen beziehungsweise heteroaromatischen Systeme wird ausgebildet, sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen der elektronentransportierenden Gruppe Q und dem Atom, über das die Gruppe der Formel (QL) an weitere Strukturelemente einer erfindungsgemäßen Verbindung bindet, liegt. Im Gegensatz hierzu kann bei einer zweiten Spirobifluorenstruktur eine durchgängige Konjugation ausgebildet werden, falls die Verbindung zwischen der Gruppe Q und dem aromatischen oder heteroaromatischen Rest, an den die Gruppe L’ gemäß Formel (QL) gebunden ist, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der Gruppe Q und dem aromatischen oder heteroaromatischen Rest, an den die Gruppe L’ gemäß Formel (QL) gebunden ist, über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen.

In einer weiteren bevorzugten Ausführungsform der Erfindung steht $L^1$ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (Ia) bis (If) genannte Bedeutung aufweisen kann.

Besonders bevorzugt steht L’ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor, insbesondere für Formel (Ia) bis (If) genannte Bedeutung aufweisen kann.

Weiterhin bevorzugt steht das unter anderem in Formel (QL) dargelegte Symbol L’ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (QL) dargelegte Gruppe $L^1$ ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme $L^1$ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^1$ substituiert sein können, bevorzugt aber unsubstituiert sind.

Ferner kann vorgesehen sein, dass die in Formel (QL) dargelegte Gruppe $L^1$ höchstens 1 Stickstoffatom, bevorzugt höchstens 2 Heteroatome, insbesondere bevorzugt höchstens ein Heteroatom und besonders bevorzugt kein Heteroatom aufweist.

Vorzugsweise kann die unter anderem in Formel (QL) dargelegte Gruppe Q beziehungsweise die Elektronentransportgruppe ausgewählt sein aus Strukturen der Formeln (Q-1), (Q-2), (Q-4), (Q-4), (Q-5), (Q-6), (Q-7), (Q-8), (Q-9) und/oder (Q-10),

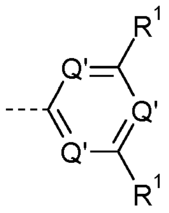

Formel (Q-1)

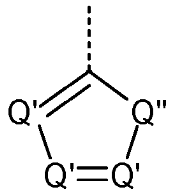

Formel (Q-2)

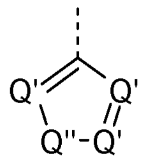

Formel (Q-3)

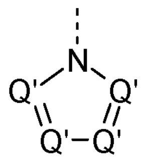

Formel (Q-4)

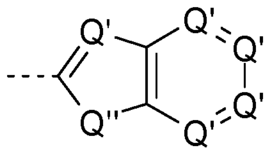

Formel (Q-5)

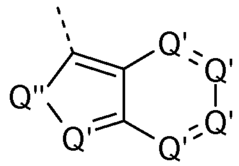

Formel (Q-6)

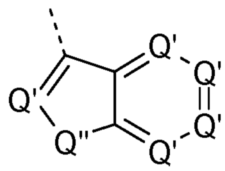

Formel (Q-7)

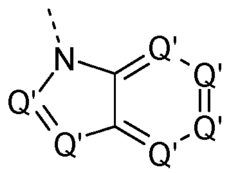

Formel (Q-8)

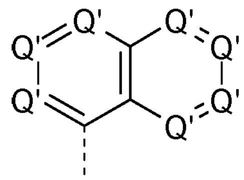

Formel (Q-9)

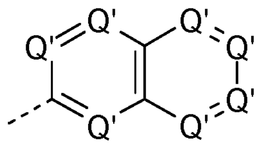

Formel (Q-10),

wobei die gestrichelte Bindung die Anbindungsposition markiert,

Q' bei jedem Auftreten gleich oder verschieden $CR^1$ oder N darstellt, und
Q" $NR^1$, O oder S darstellt;

wobei wenigstens ein Q' gleich N und

$R^1$ wie zuvor, insbesondere in Formel (Ia) bis (If) definiert ist.

Weiterhin kann die unter anderem in Formel (QL) dargelegte Gruppe Q beziehungsweise die Elektronentransportgruppe vorzugsweise ausgewählt sein aus einer Struktur der Formeln (Q-11), (Q-12), (Q-13), (Q-14) und/oder (Q-15),

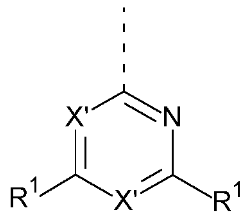

Formel (Q-11)

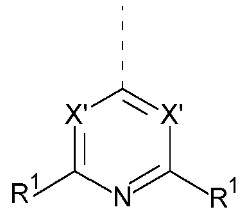

Formel (Q-12)

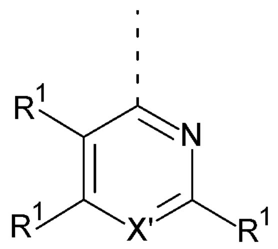

Formel (Q-13)

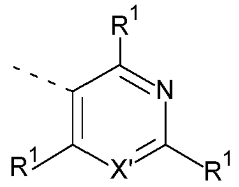

Formel (Q-14)

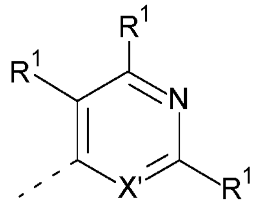

Formel (Q-15),

wobei das Symbol $R^1$ die zuvor für Formel (Ia) bis (If) genannte Bedeutung aufweist, X' N oder $CR^1$ ist und die gestrichelte Bindung die Anbindungsposition markiert, wobei X' vorzugsweise ein Stickstoffatom darstellt.

In einer weiteren Ausführungsform kann die in Formel (QL) dargelegte Gruppe Q beziehungsweise die Elektronentransportgruppe ausgewählt sein aus Strukturen der Formeln (Q-16), (Q-17), (Q-18), (Q-19), (Q-20), (Q-21) und/oder (Q-22),

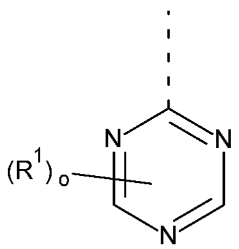

Formel (Q-16)

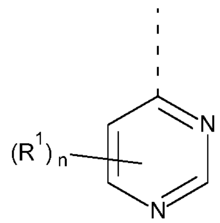

Formel (Q-17)

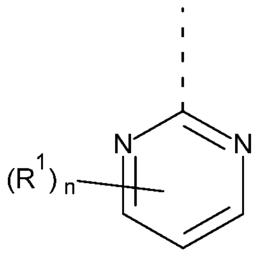

Formel (Q-18)

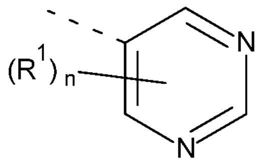

Formel (Q-19)

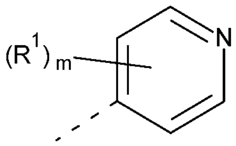

Formel (Q-20)

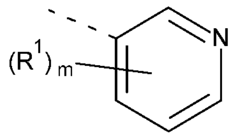

Formel (Q-21)

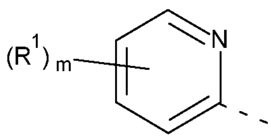

Formel (Q-22),

worin das Symbol $R^1$ die zuvor für Formel (Ia) bis (If) dargelegte Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0, 1 oder 2, vorzugsweise 1 oder 2 ist. Hierbei sind die Strukturen der Formeln (Q-16), (Q-17), (Q-18) und (Q-19) bevorzugt.

In einer weiteren Ausführungsform kann die in Formel (QL) dargelegte Gruppe Q beziehungsweise die Elektronentransportgruppe ausgewählt sein aus Strukturen der Formeln (Q-23), (Q-24) und/oder (Q-25),

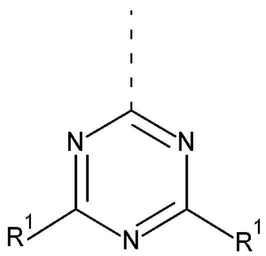

Formel (Q-23)

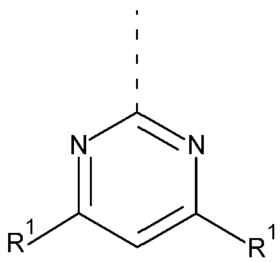

Formel (Q-24)

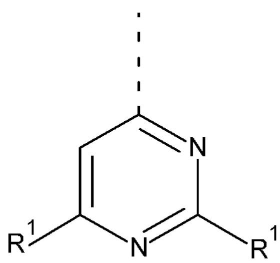

Formel (Q-25),

worin das Symbol $R^1$ die zuvor für Formel (Ia) bis (If) dargelegte Bedeutung aufweist und die gestrichelte Bindung die Anbindungsposition markiert.

In einer weiteren Ausführungsform kann die in Formel (QL) dargelegte Gruppe Q beziehungsweise die Elektronentransportgruppe ausgewählt sein aus Strukturen der Formeln (Q-26), (Q-27), (Q-28), (Q-29) und/oder (Q-30),

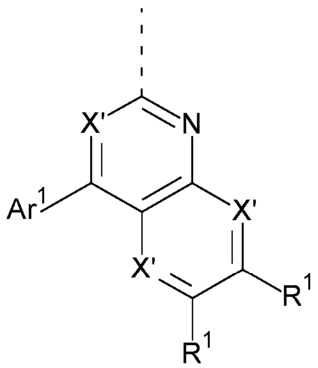

Formel (Q-26)

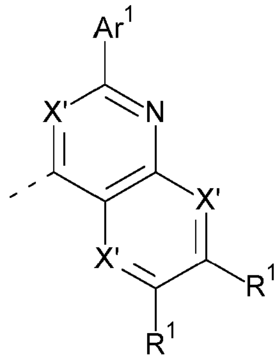

Formel (Q-27)

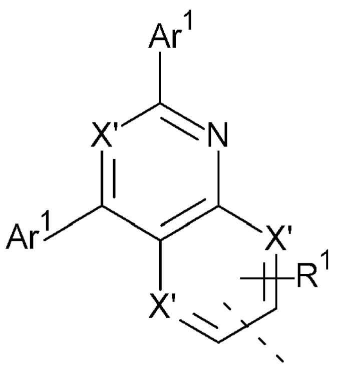

Formel (Q-28)

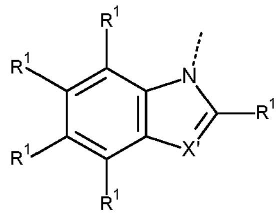

Formel (Q-29)

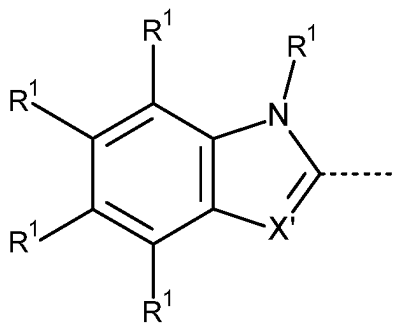

Formel (Q-30),

wobei die Symbole $Ar^1$ und $R^1$ die zuvor für Formel (Ia) bis (If) genannte Bedeutung aufweisen, X' N oder $CR^1$ ist und die gestrichelte Bindung die Anbindungsposition markiert. Vorzugsweise stellt in den Strukturen der Formeln (Q-26), (Q-27) und (Q-28) genau ein X' ein Stickstoffatom dar.

Vorzugsweise kann die in Formel (QL) dargelegte Gruppe Q beziehungsweise die Elektronentransportgruppe ausgewählt sein aus Strukturen der Formeln (Q-31), (Q-32), (Q-33), (Q-34), (Q-35), (Q-36), (Q-37), (Q-38), (Q-39), (Q-40), (Q-41), (Q-42), (Q-43) und/oder (Q-44),

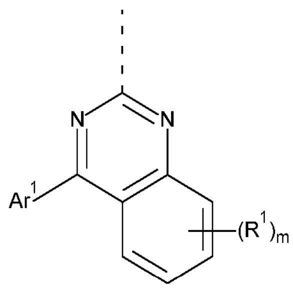

Formel (Q-31)

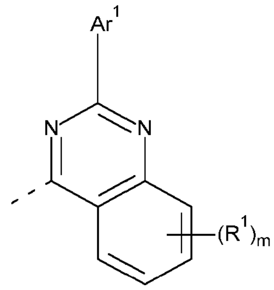

Formel (Q-32)

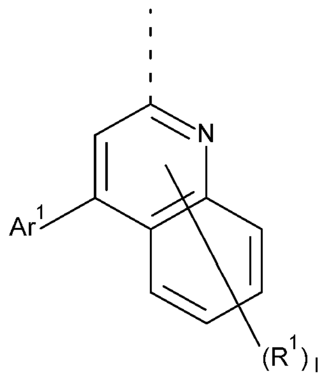

Formel (Q-33)

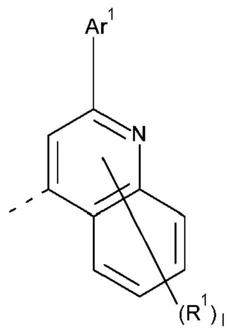

Formel (Q-34)

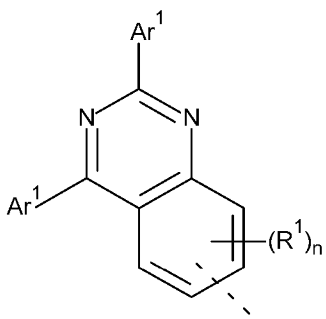

Formel (Q-35)

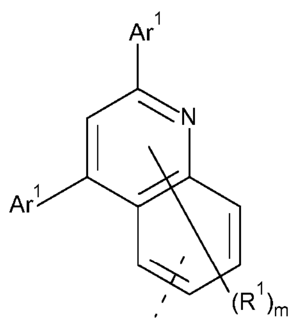

Formel (Q-36)

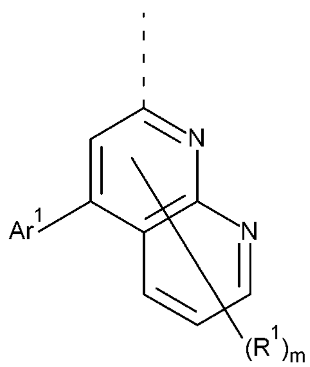

Formel (Q-37)

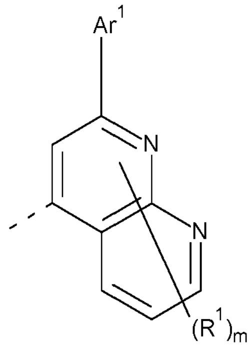

Formel (Q-38)

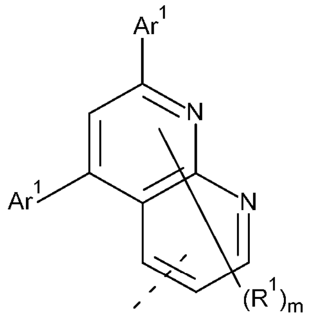

Formel (Q-39)

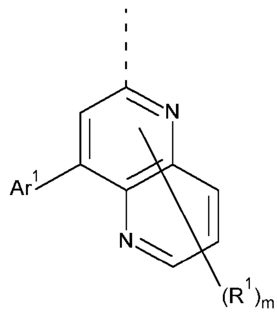

Formel (Q-40)

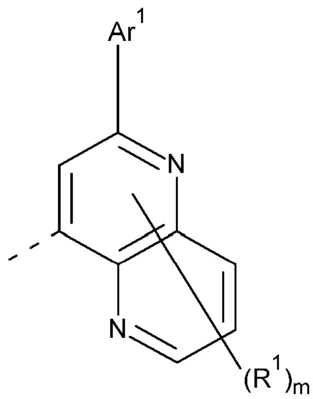

Formel (Q-41)

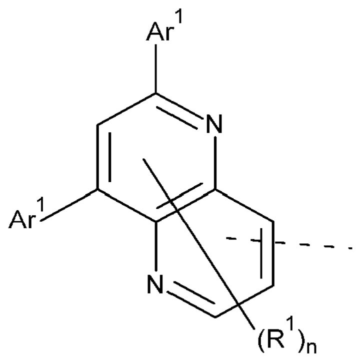

Formel (Q-42)

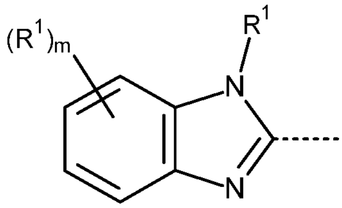

Formel (Q-43)

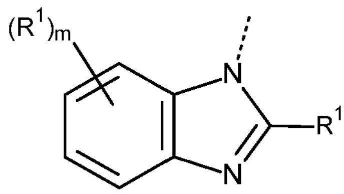

Formel (Q-44),

worin die Symbole $Ar^1$ und $R^1$ die zuvor für Formel (Ia) bis (If) dargelegte Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1, n 0, 1, 2 oder 3, vorzugsweise 0, 1 oder 2 und l 1, 2, 3, 4 oder 5, vorzugsweise 0, 1 oder 2 ist.

In einer weiteren bevorzugten Ausführungsform der Erfindung steht $Ar^1$ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste $R^2$ substituiert sein kann, bevorzugt aber unsubstituiert ist, wobei $R^2$ die zuvor, insbesondere in Formel (Ia) bis (If) dargestellte Bedeutung aufweisen kann.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise ein C- oder N-Atom der zuvor dargestellten Gruppen (H-1) bis (H-26) oder (Q-26) bis (Q-44).

Mit Vorteil stellt $Ar^1$ in den Formeln (H-1) bis (H-26) oder (Q-26) bis (Q-44) ein aromatisches Ringsystem mit 6 bis 12 aromatischen Ringatomen dar, welches mit einem oder mehreren Resten $R^2$ substituiert sein kann, vorzugsweise aber unsubstituiert ist, wobei $R^2$ die zuvor, insbesondere für Formel (Ia) bis (If) dargestellte Bedeutung aufweisen kann.

Bevorzugt bilden die Reste $R^1$ oder $R^2$ in den Formeln (H-1) bis (H-26) oder (Q-1) bis (Q-44) mit den Ringatomen der Arylgruppe oder Heteroarylgruppe $Ar^1$, $Ar^2$, $Ar^3$ und/oder $Ar^4$, an die die Reste $R^1$ oder $R^2$ gebunden sind, kein kondensiertes Ringsystem. Dies schließt die Bildung eines kondensierten Ringsystems mit möglichen Substituenten $R^2$ ein, die an die Reste $R^1$ gebunden sein können.

Ferner kann vorgesehen sein, dass die Gruppe Ar, $Ar^1$, $Ar^2$, $Ar^3$ und/oder $Ar^4$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imimdazolyl, Benzimidazolyl, Benzoxazolyl, Benzthiazolyl, 1-, 2-, 3- oder 4-Carbazolyl, Indenocarbazolyl, 1- oder 2-Napthyl, Anthracenyl, vorzugsweise 9-Anthracenyl, Phenanthrenyl und/oder Triphenylenyl, die jeweils durch einen oder mehrere Reste $R^1$ und/oder $R^2$ substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen-Gruppen besonders bevorzugt sind.

In einer bevorzugten Ausführungsform kann vorgesehen sein, dass mindestens zwei Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) jeweils eine Lochtransportgruppe umfassen, vorzugsweise darstellen.

Ferner kann vorgesehen sein, dass mindestens einer der Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) zwei Lochtransportgruppen umfasst. Hierbei kann eine Lochtransportgruppe als Rest $R^1$ angesehen werden, wobei in diesem Fall die in den Strukturen der Formeln (H-1) bis (H-26) dargelegten Substituenten $R^1$ durch Reste $R^2$ zu ersetzen sind.

Ferner kann vorgesehen sein, dass mindestens einer der Reste $R^1$ in einer Struktur gemäß einer der Formeln (Vg) bis (Vk) eine Lochtransportgruppe umfasst, wobei in diesem Fall die in den Strukturen der Formeln (H-1) bis (H-26) dargelegten Substituenten $R^1$ durch Reste $R^2$ zu ersetzen sind.

In einer bevorzugten Ausführungsform kann vorgesehen sein, dass mindestens zwei Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) jeweils einen Elektronentransportgruppe-umfassenden Rest umfassen, vorzugsweise darstellen.

Ferner kann vorgesehen sein, dass mindestens einer der Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) zwei Elektronentransportgruppe-umfassende Reste umfasst. Hierbei kann ein Elektronentransportgruppe-umfassender Rest als Rest $R^1$ angesehen werden, wobei in diesem Fall die in den Strukturen der Formeln (QL) und/oder (Q-1) bis (Q-44) dargelegten Substituenten $R^1$ durch Reste $R^2$ zu ersetzen sind.

Ferner kann vorgesehen sein, dass mindestens einer der Reste $R^1$ in einer Struktur gemäß einer der Formeln (Vg) bis (Vk) einen Elektronentransportgruppe-umfassende Rest umfasst, wobei in diesem Fall die in den Strukturen der Formeln (H-1) bis (H-26) dargelegten Substituenten $R^1$ durch Reste $R^2$ zu ersetzen sind, wobei in diesem Fall die in den Strukturen der Formeln (QL) und/oder (Q-1) bis (Q-44) dargelegten Substituenten $R^1$ durch Reste $R^2$ zu ersetzen sind.

In einer weiteren Ausgestaltung kann vorgesehen sein, dass mindestens einer der Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) eine Lochtransportgruppe umfasst, vorzugsweise darstellt, und mindestens einer der Reste R und/oder $R^a$ einen Elektronentransportgruppe-umfassenden Rest umfasst, vorzugsweise darstellt.

Ferner kann vorgesehen sein, dass mindestens einer der Reste R und/oder $R^a$ in einer Struktur gemäß den Formeln (Ia) bis (If), (IIa) bis (IIf), (IIIa) bis (IIIf), (IVa) bis (IVf) und/oder (Va) bis (Vk) sowohl einen Elektronentransportgruppe-umfassenden Rest umfasst als auch eine Lochtransportgruppe. Hierbei kann ein Elektronentransportgruppe-umfassender Rest oder eine Lochtransportgruppe als Rest $R^1$ angesehen werden, wobei in diesem Fall die in den Strukturen der Formeln (QL), (Q-1) bis (Q-44) oder (H-1) bis (H-26) dargelegten Substituenten $R^1$ durch Reste $R^2$ zu ersetzen sind.

In einer weiteren Ausgestaltung kann vorgesehen sein, dass mindestens einer der Reste R und/oder $R^a$ mindestens eine Gruppe umfasst, die zu mit Wide-Band-Gap -Materialien führt. Der Begriff "Gruppe, die zu mit Wide-Band-Gap -Materialien führt" legt dar, dass die Verbindungen als Wide-Band-Gap-Materialien eingesetzt werden können, so dass die Verbindungen entsprechende Gruppen aufweisen. Wide-Band-Gap-Materialien werden später ausführlicher dargelegt.

Ferner kann vorgesehen sein, dass mindestens einer der Reste R und/oder $R^a$ mindestens eine Gruppe umfasst, die zu Materialien führt, die als Hostmaterial eingesetzt werden. Der Begriff "Gruppe, die zu Materialien führt, die als Hostmaterial eingesetzt werden" legt dar, dass die Verbindungen als Hostmaterialien eingesetzt werden können, so dass die Verbindungen entsprechende Gruppen aufweisen. Hostmaterialien werden später ausführlicher dargelegt.

In einer weiteren Ausgestaltung kann vorgesehen sein, dass die Verbindung ein kondensiertes aromatisches oder heteroaromatisches Ringsystem mit mindestens 2, vorzugsweise drei kondensierten Ringen umfasst, welches gegebenenfalls substituiert sein kann.

Vorzugsweise umfasst mindestens einer der Reste R und/oder $R^a$ in Strukturen der Formeln (Ia) bis (If), (IIa) bis (IIf), (IIIa) bis (IIIf), (IVa) bis (IVf) und/oder (Va) bis (Vk) mindestens ein aromatisches oder heteroaromatisches Ringsystem

mit zwei, vorzugsweise mit drei kondensierten aromatischen oder heteroaromatischen Ringen.

Bevorzugt kann vorgesehen sein, dass das aromatische oder heteroaromatische Ringsystem mit zwei, vorzugsweise mit drei kondensierten aromatischen oder heteroaromatischen Ringen ausgewählt ist aus den Gruppen der Formeln (Ar-1) bis (Ar-11),

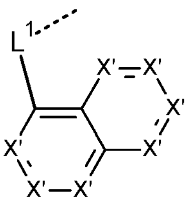
(Ar-1)

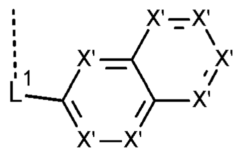
(Ar-2)

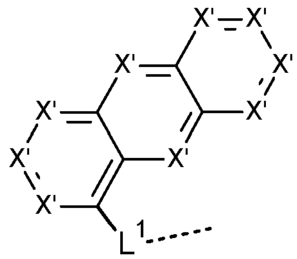
(Ar-3)

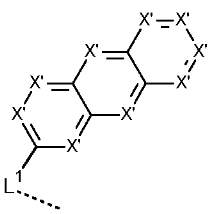
(Ar-4)

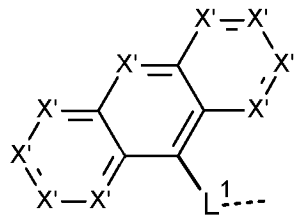
(Ar-5)

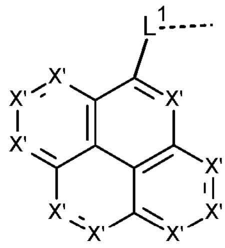
(Ar-6)

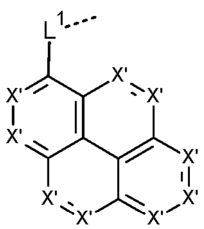
(Ar-7)

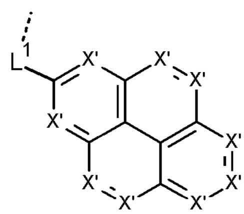
(Ar-8)

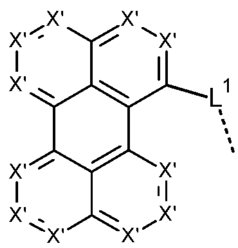
(Ar-9)

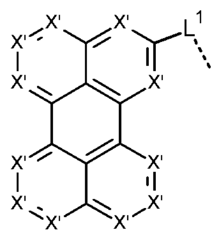
(Ar-10)

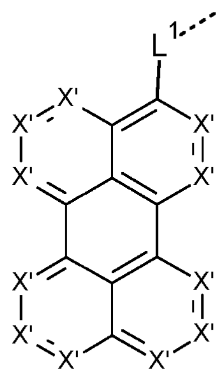
(Ar-11),

wobei X' N oder $CR^1$, vorzugsweise $CR^1$ ist, L' eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, wobei $R^1$ die zuvor, insbesondere für Formel (Ia) bis (If) dargelegte Bedeutung hat und die gestrichelte Bindung die Anbindungsposition markiert.

Ganz besonders bevorzugt kann vorgesehen sein, dass das aromatische oder heteroaromatische Ringsystem mit zwei, vorzugsweise mit drei kondensierten aromatischen oder heteroaromatischen Ringen ausgewählt ist aus den Gruppen der Formeln (Ar’-1) bis (Ar’-11),

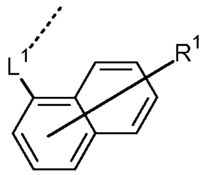
(Ar‘-1)

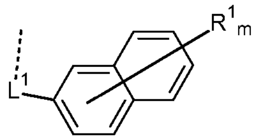
(Ar‘-2)

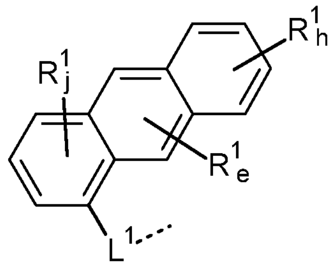
(Ar‘-3)

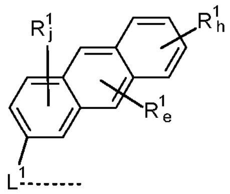
(Ar‘-4)

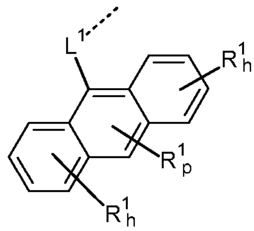
(Ar‘-5)

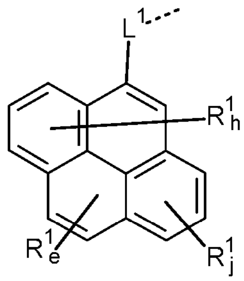
(Ar‘-6)

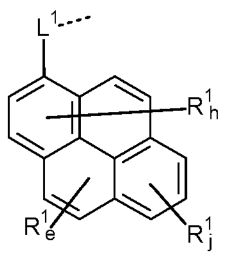
(Ar‘-7)

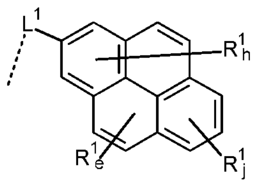
(Ar‘-8)

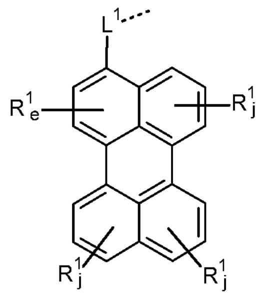
(Ar‘-9)

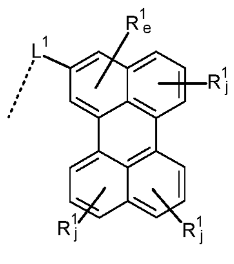
(Ar‘-10)

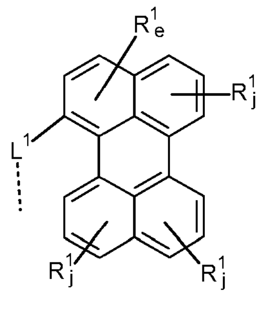
(Ar‘-11),

wobei L’ eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, wobei $R^1$ die zuvor, insbesondere für Formel (Ia) bis (If) dargelegte Bedeutung hat, die gestrichelte Bindung die Anbindungsposition markiert und für die Indices gilt:

p ist 0 oder 1;
e ist 0, 1 oder 2, vorzugsweise 0 oder 1;
j ist bei jedem Auftreten unabhängig 0, 1, 2 oder 3, vorzugsweise 0,1 oder 2, besonders bevorzugt vorzugsweise 0 oder 1;
h ist bei jedem Auftreten unabhängig 0, 1, 2, 3 oder 4, vorzugsweise 0,1 oder 2, besonders bevorzugt vorzugsweise 0 oder 1;
i ist bei jedem Auftreten unabhängig 0, 1 oder 2;
m ist eine ganze Zahl im Bereich von 0 bis 7, vorzugsweise 0, 1, 2, 3, 4, 5 oder 6, besonders bevorzugt 0, 1, 2, 3 oder 4, speziell bevorzugt 0, 1 oder 2.

Vorzugsweise beträgt die Summe der Indices p, e, i, j, h und m in den Strukturen der Formel (Ar'-1) bis (Ar'-11) jeweils höchstens 3, vorzugsweise höchstens 2 und besonders bevorzugt höchstens 1.

Die zuvor dargelegten Strukturen der Formeln (Ar-1) bis (Ar-11) und/oder (Ar'-1) bis (Ar'-11) sind insbesondere bevorzugte Reste für Verbindungen, die sich zur Verwendung als fluoreszierender Emitter oder als blaue OLED-Materialien eignen.

In einer bevorzugten Variante der vorliegenden Erfindung stellt eine der zuvor dargelegten Strukturen der Formeln (Ar-1) bis (Ar-11) und/oder (Ar'-1) bis (Ar'-11) einen Rest $R^a$ in einer Struktur der Formeln (Vb) bis (Vk) dar, vorzugsweise (Vc) bis (Ve).

In einer weiterhin bevorzugten Ausführungsform der vorliegenden Erfindung stellt die Gruppe L' in den zuvor dargelegten Strukturen der Formeln (Ar-1) bis (Ar-11) und/oder (Ar'-1) bis (Ar'-11) ein aromatisches oder heteroaromatisches Ringsystem mit 5 bis 40, bevorzugt 5 bis 30 aromatischen Ringatomen dar, welches durch einen oder mehrere Reste $R^1$ substituiert sein kann, wobei $R^1$ die zuvor, insbesondere für Formel (Ia) bis (If) dargelegte Bedeutung hat.

Wenn $X^1$ für $CR^1$ steht bzw. wenn die aromatische und/oder heteroaromatische Gruppe durch Substituenten $R^1$ substituiert ist, dann sind diese Substituenten $R^1$ bevorzugt gewählt aus der Gruppe bestehend aus H, D, F, CN, $N(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten $R^2$ substituiert sein kann, wobei ein oder mehrere H-Atome durch D ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, ; dabei können optional zwei Substituenten $R^1$, die vorzugsweise an benachbarte Kohlenstoffatome gebunden sind, ein monocyclisches oder polycyclisches, aliphatisches, aromatisches oder heteroaromatisches Ringsystem bilden, wobei die Gruppe $Ar^1$ die zuvor, insbesondere für Formel (Ia) bis (If) genannte Bedeutung aufweist.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^2$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^2$ substituiert sein können, bevorzugt aber unsubstituiert sind.

Weiterhin kann vorgesehen sein, dass die Substitutenten $R^1$ eines aromatischen oder heteroaromatischen Ringssystems mit weiteren Ringatomen des aromatischen oder heteroaromatischen Ringssystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$ ein, die an die Reste $R^1$ gebunden sein können.

Weiterhin kann vorgesehen sein, dass in einer Struktur gemäß Formel (Ia) bis (If), (IIa) bis (IIf), (IIIa) bis (IIIf), (IVa) bis (IVf) und/oder (Va) bis (Vk) mindestens ein Rest $R^1$ oder $Ar^1$ für eine Gruppe steht, die ausgewählt ist aus den Formeln ($R^1$-1) bis ($R^1$- 43), beziehungsweise in einer Struktur gemäß Formel (H-1) bis (H-26), (QL), (Q-1) bis (Q-44), (Ar-1) bis (Ar-11) und/oder (Ar'-1) bis (Ar'-11) mindestens ein Rest $Ar^1$ oder $R^1$ für eine Gruppe steht, die ausgewählt ist aus den Formeln ($R^1$-1) bis ($R^1$- 43),

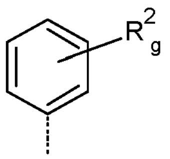

Formel ($R^1$-1)

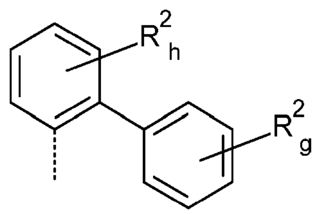

Formel ($R^1$-2)

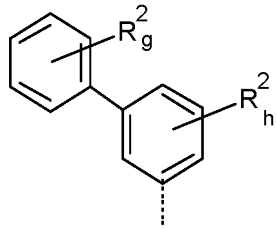

Formel ($R^1$-3)

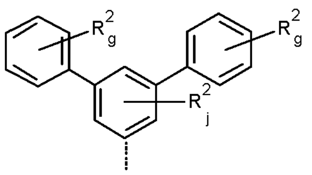

Formel ($R^1$-4)

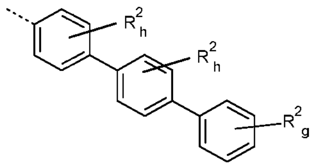

Formel ($R^1$-5)

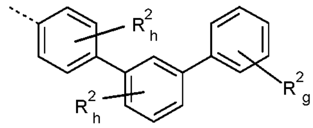

Formel ($R^1$-6)

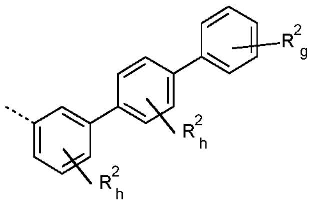

Formel ($R^1$-7)

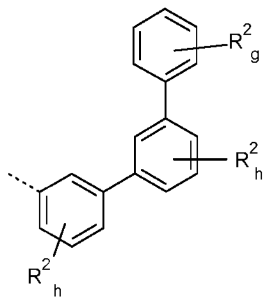

Formel ($R^1$-8)

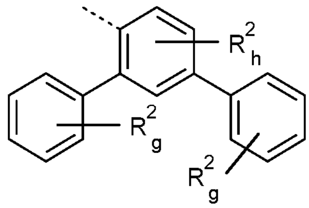

Formel ($R^1$-9)

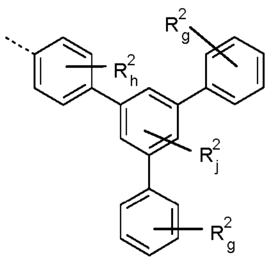

Formel ($R^1$-10)

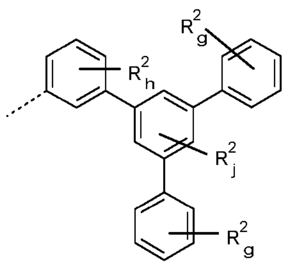

Formel ($R^1$-11)

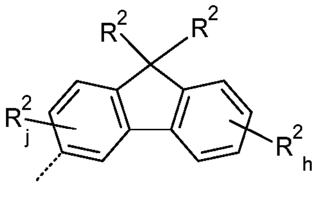

Formel ($R^1$-12)

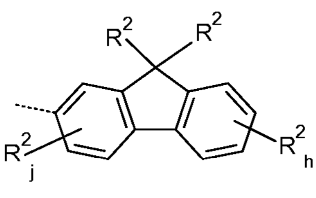

Formel ($R^1$-13)

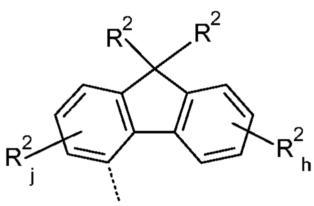

Formel ($R^1$-14)

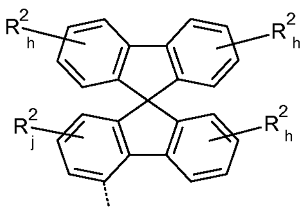

Formel ($R^1$-15)

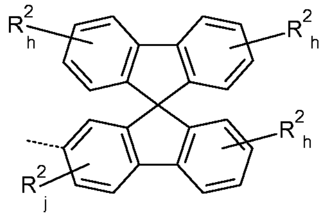

Formel ($R^1$-16)

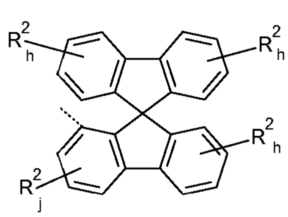

Formel ($R^1$-17)

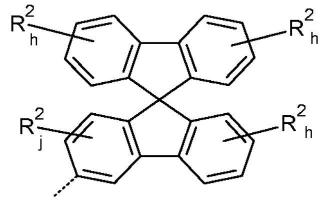

Formel ($R^1$-18)

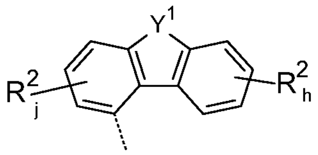

Formel ($R^1$-19)

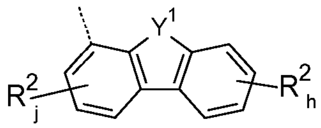

Formel ($R^1$-20)

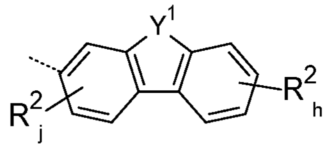

Formel ($R^1$-21)

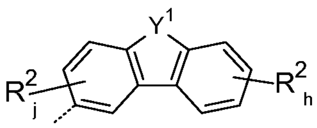

Formel ($R^1$-22)

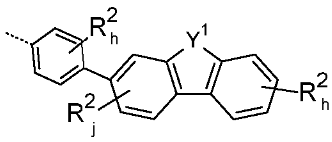

Formel ($R^1$-23)

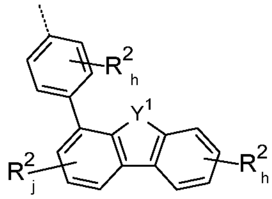

Formel ($R^1$-24)

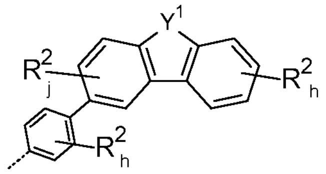

Formel ($R^1$-25)

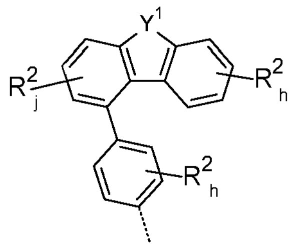

Formel ($R^1$-26)

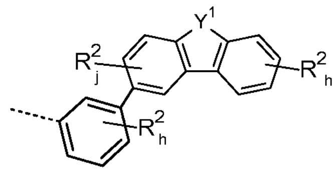

Formel ($R^1$-27)

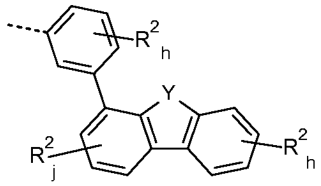

Formel ($R^1$-28)

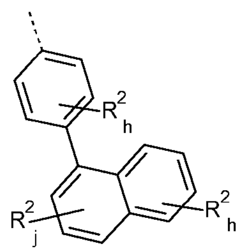

Formel ($R^1$-29)

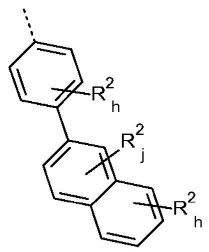

Formel ($R^1$-30)

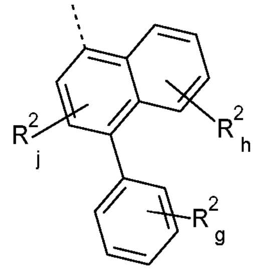

Formel ($R^1$-31)

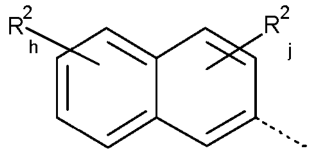

Formel ($R^1$-32)

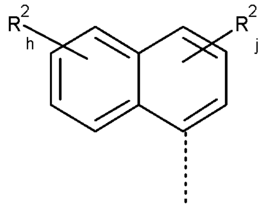

Formel ($R^1$-33)

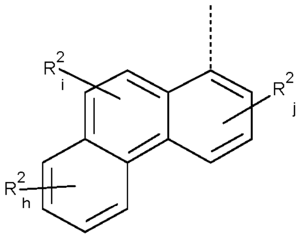

Formel ($R^1$-34)

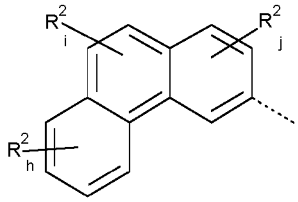

Formel ($R^1$-35)

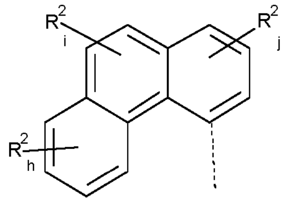

Formel ($R^1$-36)

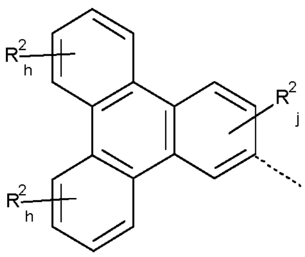

Formel ($R^1$-37)

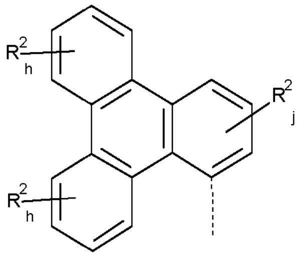

Formel ($R^1$-38)

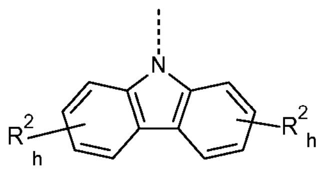

Formel ($R^1$-39)

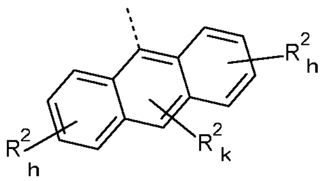

Formel ($R^1$-40)

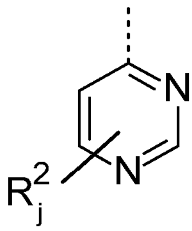

Formel ($R^1$-41)

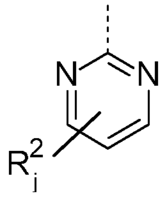

Formel ($R^1$-42)

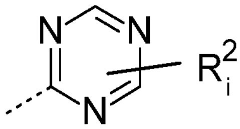

Formel ($R^1$-43),

wobei für die verwendeten Symbole gilt:

$Y^1$ ist O, S oder $NR^2$, vorzugsweise O oder S;
k ist bei jedem Auftreten unabhängig 0 oder 1;
i ist bei jedem Auftreten unabhängig 0, 1 oder 2;

j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
$R^2$ kann die zuvor genannte, insbesondere für Formel (la) bis (If) genannte Bedeutung aufweisen und

die gestrichelte Bindung markiert die Anbindungsposition.

Hierbei sind die Gruppen der Formeln $R^1$-1 bis $R^1$-28 bevorzugt, wobei die Gruppen R’-1, $R^1$-3, $R^1$-4, R’-10, R’-11, R’-12, R’-13, R’-14, R’-16, R’-17, R’-18, $R^1$-19, $R^1$-20, $R^1$-21 und/oder $R^1$-22 besonders bevorzugt sind.
Vorzugsweise kann vorgesehen sein, dass die Summe der Indices k, i, j, h und g in den Strukturen der Formel ($R^1$-1) bis ($R^1$-43) jeweils höchstens 3, vorzugsweise höchstens 2 und besonders bevorzugt höchstens 1 beträgt.
Bevorzugt bilden die Reste $R^2$ in den Formeln ($R^1$-1) bis ($R^1$-43) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem.
Die zuvor dargelegten Reste der Formeln ($R^1$-1) bis ($R^1$-43) stellen bevorzugte Reste Ar gemäß Formel (la) bis (If) beziehungsweise $Ar^3$, $Ar^4$ gemäß Formeln (H-1) bis (H-3) oder bevorzugte Ausführungsformen dieser Formeln dar, wobei in diesem Fall die in den Formeln ($R^1$-1) bis ($R^1$-43) dargelegten Gruppen $R^2$ durch Reste $R^1$ zu ersetzen sind. Die zuvor dargelegten Bevorzugungen hinsichtlich der Formeln ($R^1$-1) bis ($R^1$-43) gelten entsprechend.
Bevorzugt kann vorgesehen sein, dass die Verbindung mindestens eine verbindende Gruppe umfasst, die ausgewählt ist aus den Formeln ($L^1$-1) bis ($L^1$-73), vorzugsweise in der Struktur gemäß Formeln (H-1) bis (H-26) die Gruppe $Ar^2$ ausgewählt ist aus den Formeln ($L^1$-1) bis ($L^1$-73) oder die Elektronentransportgruppe mit weiteren Strukturelementen über eine verbindende Gruppe verbunden ist, die ausgewählt ist aus den Formeln ($L^1$-1) bis ($L^1$-73) oder der Rest $L^1$ in Formeln (QL), (Ar-1) bis (Ar-11) und/oder (Ar’-1) bis (Ar’-11) für eine Gruppe steht, die ausgewählt ist aus den Formeln ($L^1$-1) bis ($L^1$-73),

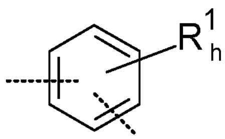
Formel ($L^1$-1)

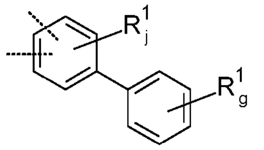
Formel ($L^1$-2)

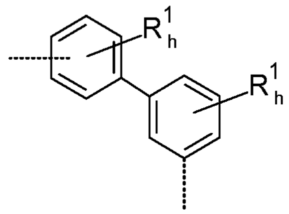
Formel ($L^1$-3)

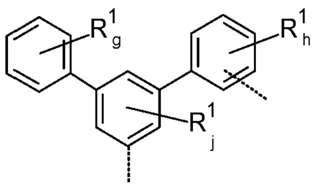
Formel ($L^1$-4)

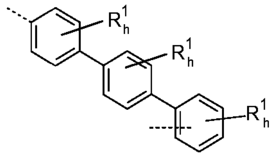
Formel ($L^1$-5)

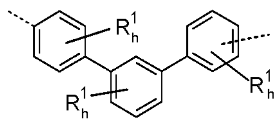
Formel ($L^1$-6)

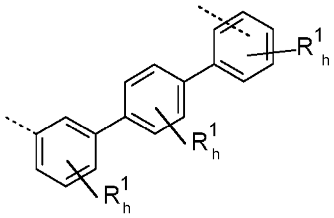
Formel ($L^1$-7)

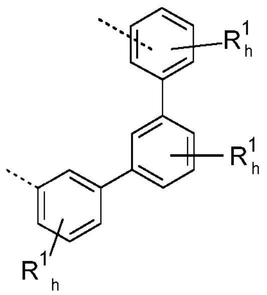
Formel ($L^1$-8)

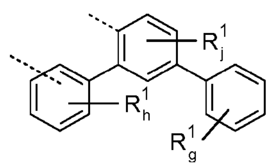
Formel ($L^1$-9)

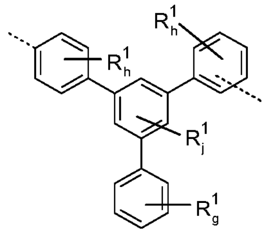

Formel ($L^1$-10)

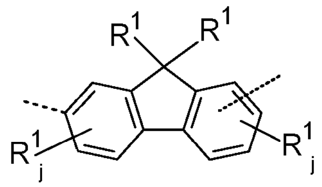

Formel ($L^1$-11)

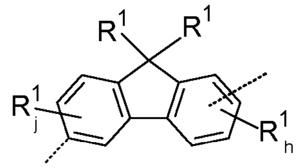

Formel ($L^1$-12)

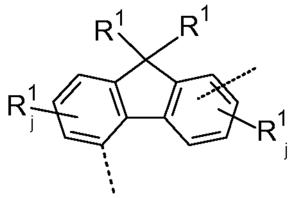

Formel ($L^1$-13)

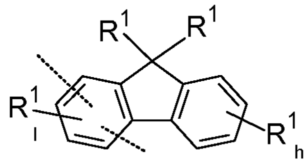

Formel ($L^1$-14)

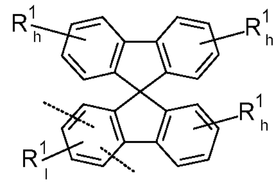

Formel ($L^1$-15)

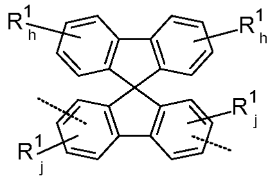

Formel ($L^1$-16)

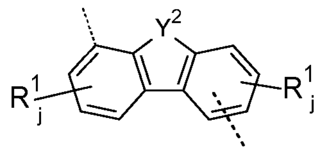

Formel ($L^1$-17)

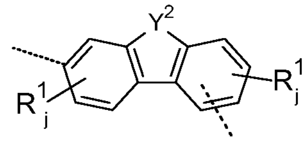

Formel ($L^1$-18)

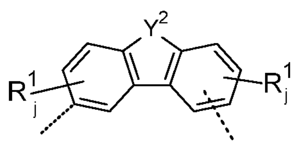

Formel ($L^1$-19)

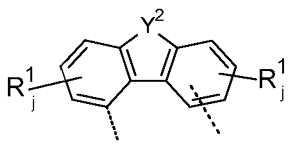

Formel ($L^1$-20)

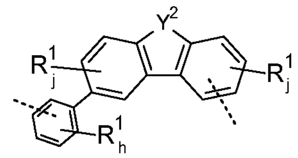

Formel ($L^1$-21)

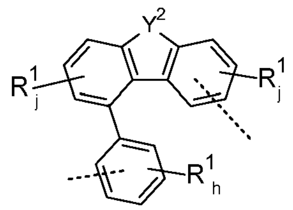

Formel ($L^1$-22)

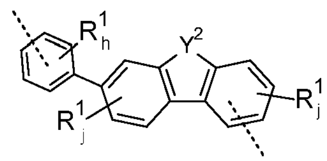

Formel ($L^1$-23)

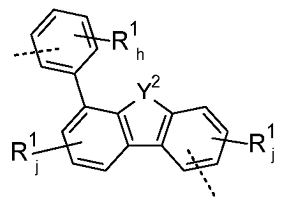

Formel ($L^1$-24)

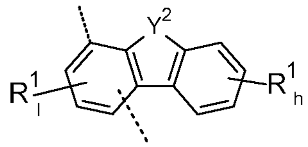

Formel ($L^1$-25)

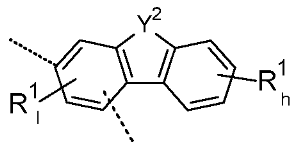

Formel ($L^1$-26)

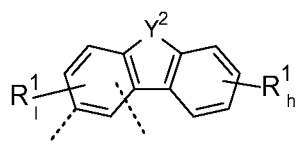

Formel ($L^1$-27)

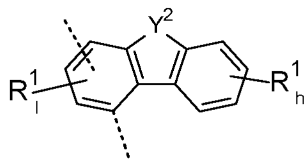

Formel ($L^1$-28)

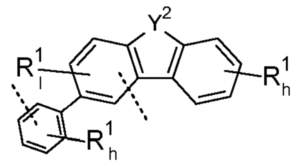

Formel ($L^1$-29)

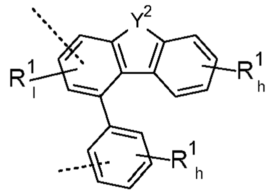

Formel ($L^1$-30)

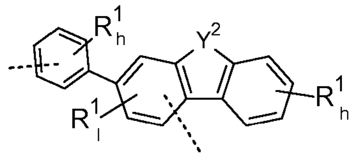

Formel ($L^1$-31)

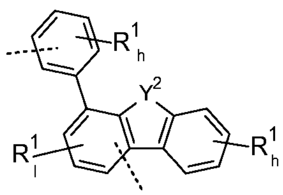

Formel ($L^1$-32)

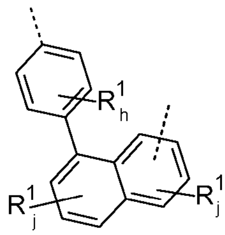

Formel ($L^1$-33)

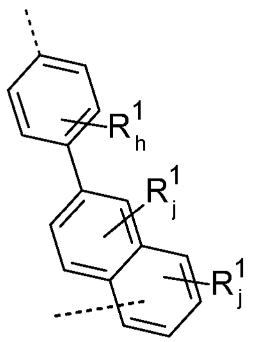

Formel ($L^1$-34)

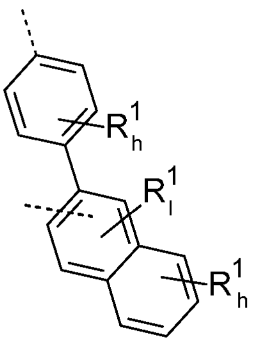

Formel ($L^1$-35)

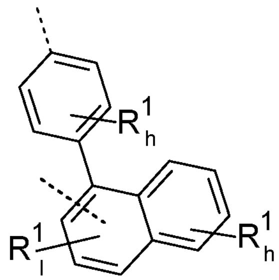

Formel ($L^1$-36)

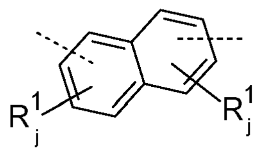

Formel ($L^1$-37)

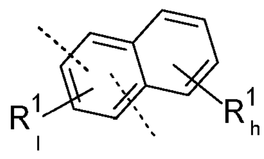

Formel ($L^1$-38)

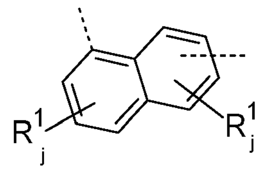

Formel ($L^1$-39)

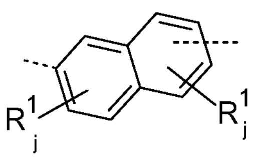

Formel ($L^1$-40)

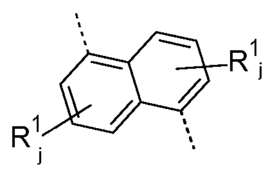

Formel ($L^1$-41)

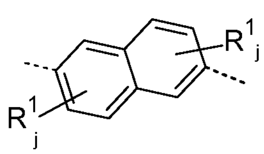

Formel ($L^1$-42)

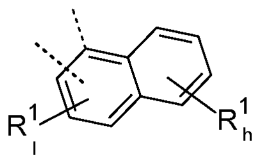

Formel ($L^1$-43)

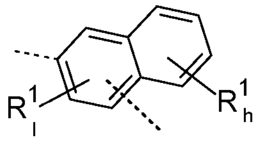

Formel ($L^1$-44)

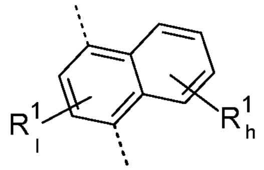

Formel ($L^1$-45)

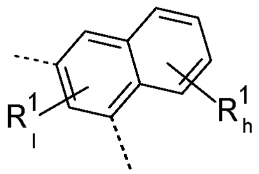

Formel ($L^1$-46)

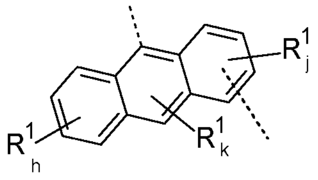

Formel ($L^1$-47)

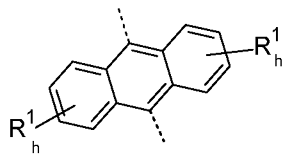

Formel ($L^1$-48)

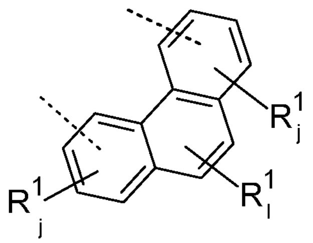

Formel ($L^1$-49)

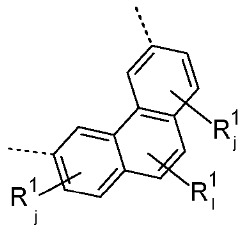

Formel ($L^1$-50)

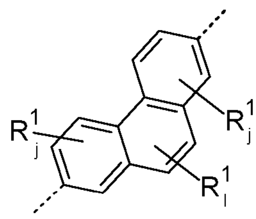

Formel ($L^1$-51)

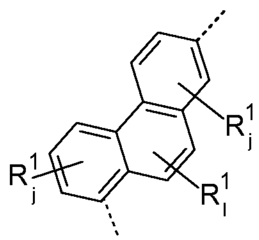

Formel ($L^1$-52)

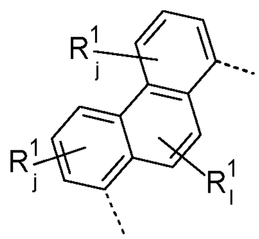

Formel ($L^1$-53)

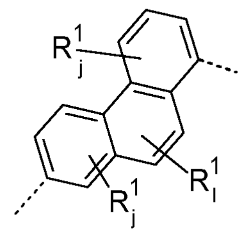

Formel ($L^1$-54)

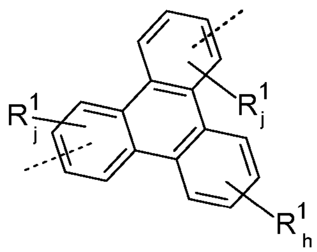

Formel ($L^1$-55)

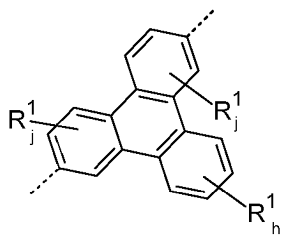

Formel ($L^1$-56)

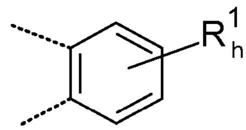

Formel ($L^1$-57)

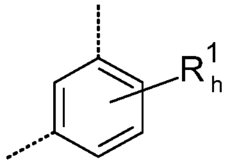

Formel ($L^1$-58)

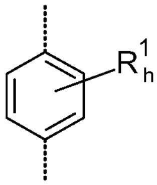

Formel ($L^1$-59)

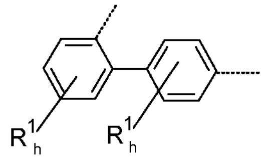

Formel ($L^1$-60)

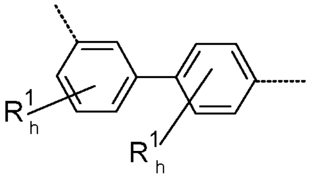

Formel ($L^1$-61)

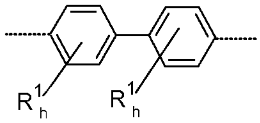

Formel ($L^1$-62)

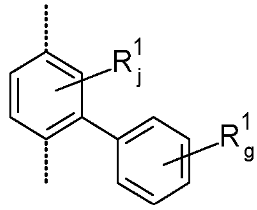

Formel ($L^1$-63)

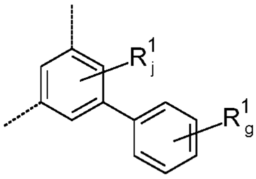

Formel ($L^1$-64)

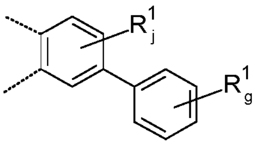

Formel ($L^1$-65)

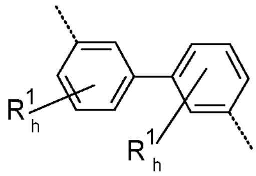

Formel ($L^1$-66)

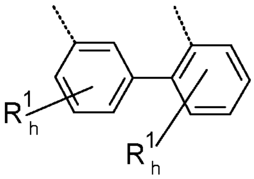

Formel ($L^1$-67)

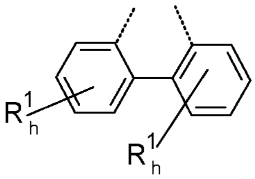

Formel ($L^1$-68)

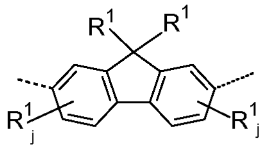

Formel ($L^1$-69)

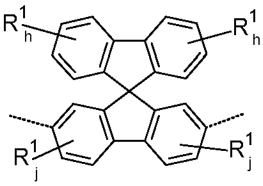

Formel ($L^1$-70)

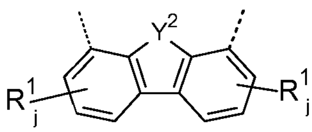

Formel ($L^1$-71)

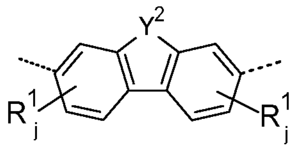

Formel ($L^1$-72)

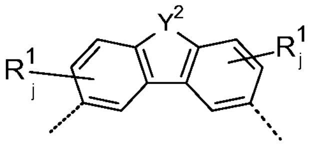

Formel ($L^1$-73),

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol $Y^2$ O, S oder $NR^1$, vorzugsweise O oder S ist; und das Symbol $R^1$

die zuvor, insbesondere für Formel (Ia) bis (If) genannte Bedeutung aufweist.

Vorzugsweise kann vorgesehen sein, dass die Summe der Indices k, l, g, h und j in den Strukturen der Formel ($L^1$-1) bis ($L^1$-73) jeweils höchstens 3, vorzugsweise höchstens 2 und besonders bevorzugt höchstens 1 beträgt. Bevorzugte erfindungsgemäße Verbindungen mit einer Gruppe der Formeln (H-1) bis (H-26) umfassen eine Gruppe $Ar^2$, die ausgewählt ist aus einer der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68). Mit Vorteil kann die Summe der Indices k, l, g, h und j in den Strukturen der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68) jeweils höchstens 3, vorzugsweise höchstens 2 und besonders bevorzugt höchstens 1 betragen.

Bevorzugte erfindungsgemäße Verbindungen mit einer Gruppe der Formel (QL) umfassen eine Gruppe $L^1$, die eine Bindung darstellt oder die ausgewählt ist aus einer der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68). Mit Vorteil kann die Summe der Indices k, l, g, h und j in den Strukturen der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68) jeweils höchstens 3, vorzugsweise höchstens 2 und besonders bevorzugt höchstens 1 betragen.

Bevorzugte erfindungsgemäße Verbindungen mit einer Gruppe der Formeln (Ar-1) bis (Ar-11) und/oder (Ar'-1) bis (Ar'-11) umfassen eine Gruppe $L^1$, die eine Bindung darstellt oder die ausgewählt ist aus einer der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68). Mit Vorteil kann die Summe der Indices k, l, g, h und j in den Strukturen der Formeln ($L^1$-1) bis ($L^1$-46) und/oder ($L^1$-57) bis ($L^1$-73), bevorzugt der Formel ($L^1$-1) bis ($L^1$-32) und/oder ($L^1$-57) bis ($L^1$-73), speziell bevorzugt der Formel ($L^1$-1) bis ($L^1$-10) und/oder ($L^1$-57) bis ($L^1$-68) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-73) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem.

Gemäß einer bevorzugten Ausgestaltung ist eine erfindungsgemäße Verbindung nach Anspruch 12 oder 13 durch mindestens eine der Strukturen gemäß Formel (Ia) bis (If), (IIa) bis (IIf), (IIIa) bis (IIIf), (IVa) bis (IVf) und/oder (Va) bis (Vk) darstellbar. Vorzugsweise weisen Verbindungen, umfassend Strukturen gemäß Formel (Ia) bis (If), (IIa) bis (IIf), (IIIa) bis (IIIf), (IVa) bis (IVf) und/oder (Va) bis (Vk), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Bei Ausgestaltung der erfindungsgemäßen Verbindungen zur Verwendung als fluoreszierender Emitter oder als blaue OLED-Materialien können bevorzugte Verbindungen entsprechende Gruppen, beispielsweise Fluoren-, Anthracen- und/oder Pyren-Gruppen enthalten, die mit Gruppen $R^1$ oder $R^2$ substituiert sein können oder die durch entsprechende Substituition der Gruppen ($L^1$-1) bis ($L^1$-73) oder ($R^1$-1) bis ($R^1$-43) mit den Substituenten $R^1$ oder $R^2$ gebildet werden.

In einer weiteren bevorzugten Ausführungsform der Erfindung ist $R^2$, beispielsweise bei einer Struktur gemäß Formeln (Ia) bis (If), (IIa) bis (IIf), (IIIa) bis (IIIf), (IVa) bis (IVf) und/oder (Va) bis (Vk) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste $R^2$ mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem.

Ferner kann vorgesehen sein, dass die erfindungsgemäße Verbindung nicht in unmittelbaren Kontakt mit einem Metallatom steht, vorzugsweise kein Ligand für einen Metallkomplex darstellt.

Besonders bevorzugt sind weiterhin Verbindungen mit Strukturen der Formel (Va), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar’-1 bis Ar’-11 | Ar’-3 bis Ar’-11 | Ar’-3 bis Ar’-5 | Ar’-5 |

Besonders bevorzugt sind weiterhin Verbindungen mit Strukturen der Formel (Vb), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar’-1 bis Ar’-11 | Ar’-3 bis Ar’-11 | Ar’-3 bis Ar’-5 | Ar’-5 |

Besonders bevorzugt sind weiterhin Verbindungen mit Strukturen der Formel (Vc), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar’-1 bis Ar’-11 | Ar’-3 bis Ar’-11 | Ar’-3 bis Ar’-5 | Ar’-5 |

Besonders bevorzugt sind weiterhin Verbindungen mit Strukturen der Formel (Vd), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |

(fortgesetzt)

| Rest $R^a$ umfasst eine der Gruppen | vorzugsweise | besonders bevorzugt | ganz besonders bevorzugt |
|---|---|---|---|
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar'-1 bis Ar'-11 | Ar'-3 bis Ar'-11 | Ar'-3 bis Ar'-5 | Ar'-5 |

Besonders bevorzugt sind weiterhin Verbindungen mit Strukturen der Formel (Ve), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar'-1 bis Ar'-11 | Ar'-3 bis Ar'-11 | Ar'-3 bis Ar'-5 | Ar'-5 |

Besonders bevorzugt sind weiterhin Verbindungen mit Strukturen der Formel (Vf), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar'-1 bis Ar'-11 | Ar'-3 bis Ar'-11 | Ar'-3 bis Ar'-5 | Ar'-5 |

Besonders bevorzugt sind weiterhin Verbindungen mit Strukturen der Formel (Vk), welche die folgenden Eigenschaften aufweisen:

| Rest $R^a$ umfasst eine der Gruppen | vorzugsweise | besonders bevorzugt | ganz besonders bevorzugt |
|---|---|---|---|
| H-1 bis H-26 | H-1 - H-11 und H-15 bis H-17 | H-1 bis H-11 | H-1 |
| H-1 bis H-26 | H-4 bis H-26 | H-4 bis H-11 und H-15 bis H-17 | H4, H-5 |
| QL | Q-11 bis Q-25, | Q-16 bis 19 und Q-23 bis Q-25 | Q-23 |
| QL | Q-26 bis Q44 | Q-33 bis Q-42 | Q-35 |
| Ar-1 bis Ar-11 | Ar-3 bis Ar-11 | Ar-3 bis Ar-5 | Ar-5 |
| Ar'-1 bis Ar'-11 | Ar'-3 bis Ar'-11 | Ar'-3 bis Ar'-5 | Ar'-5 |

Die Reste der Formeln H-1 bis H-26 in den zuvor dargelegten Tabellen sind vorzugsweise gemäß den folgenden Kriterien ausgewählt:

| $Ar^1$ (falls vorhanden) | $Ar^2$ (falls vorhanden) | $Ar^3$, $Ar^4$ |
|---|---|---|
| $R^1$-1 bis $R^1$-43 | $L^1$-1 bis $L^1$-73 | $R^1$-1 bis $R^1$-43 |
| $R^1$-1 bis $R^1$-28 | $L^1$-1 bis $L^1$-46 oder $L^1$-57 bis $L^1$-73 | $R^1$-1 bis $R^1$-28 |
| $R^1$-1 bis $R^1$-43 | $L^1$-1 bis $L^1$-4 | $R^1$-1 bis $R^1$-43 |

Die Reste der Formel QL in den zuvor dargelegten Tabellen sind vorzugsweise gemäß den folgenden Kriterien ausgewählt:

| $L^1$ | vorzugsweise | Q | vorzugsweise |
|---|---|---|---|
| Eine Bindung oder $L^1$-1 bis $L^1$-73 | Eine Bindung oder $L^1$-1 bis $L^1$-46 oder $L^1$-57 bis $L^1$-73 | Q-11 bis Q-25 | Q-16 bis 19 und Q-23 bis Q-25 |
| Eine Bindung oder $L^1$-1 bis $L^1$-73 | Eine Bindung oder $L^1$-1 bis $L^1$-46 oder $L^1$-57 bis $L^1$-73 | Q-26 bis Q44 | Q-33 bis Q-42 |

Beispiele für geeignete Verbindungen gemäß den oben aufgeführten Ausführungsformen sind die in der folgenden Tabelle aufgeführten Verbindungen:

| | |
|---|---|
| 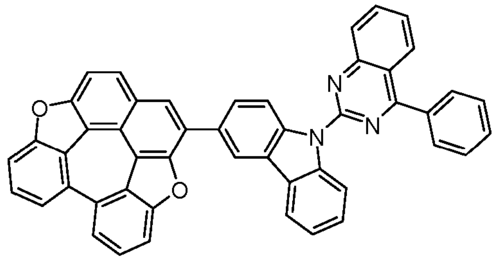 | 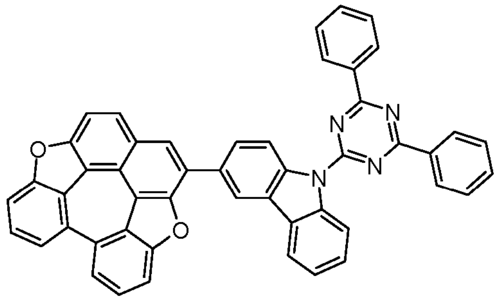 |
| 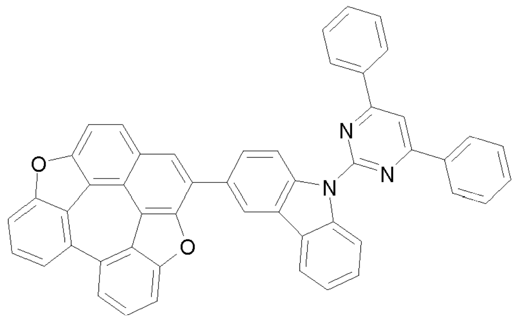 | 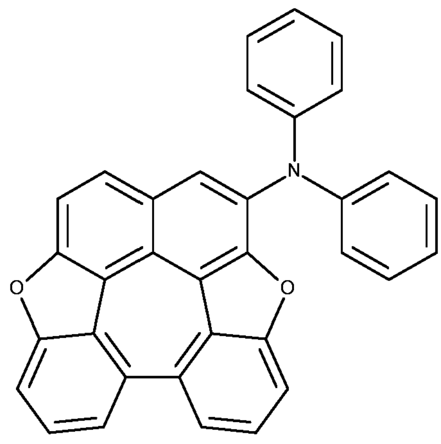 |
| 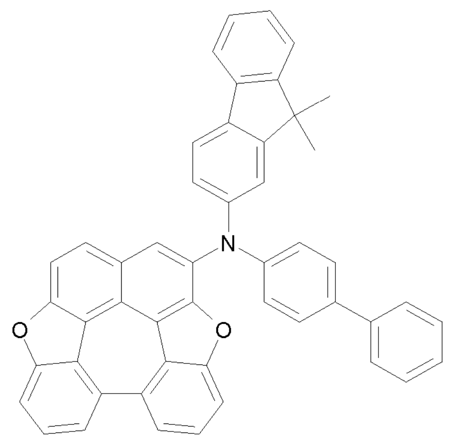 | 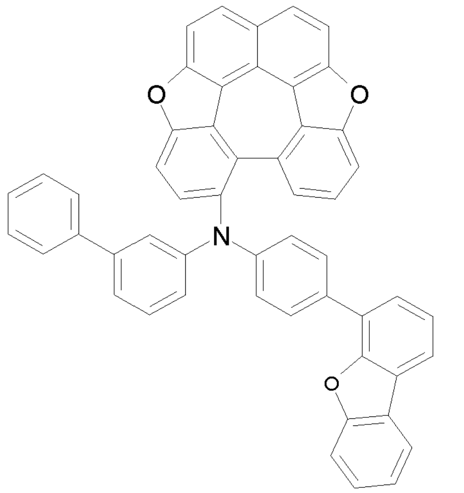 |

| | |
|---|---|
| 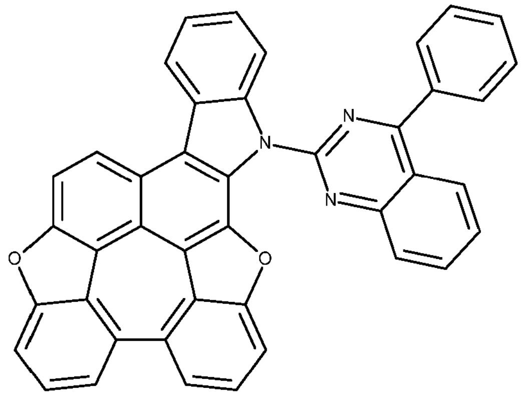 | 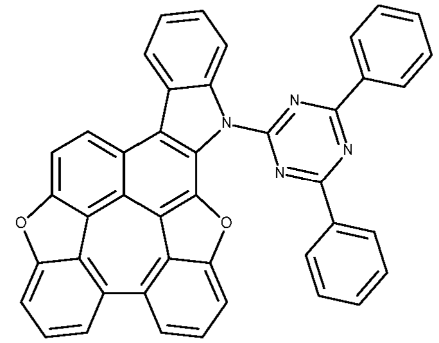 |
| 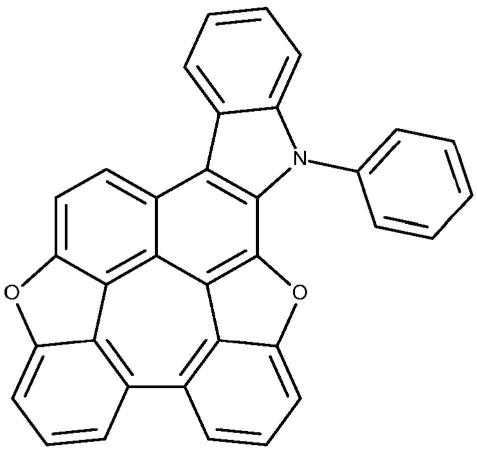 | 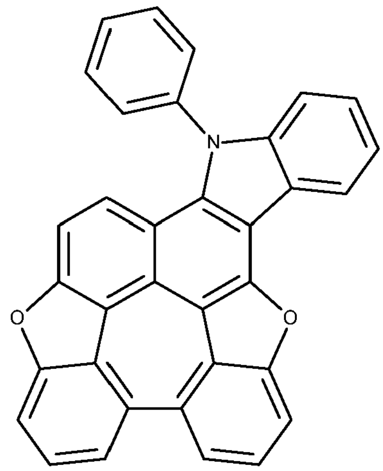 |
| 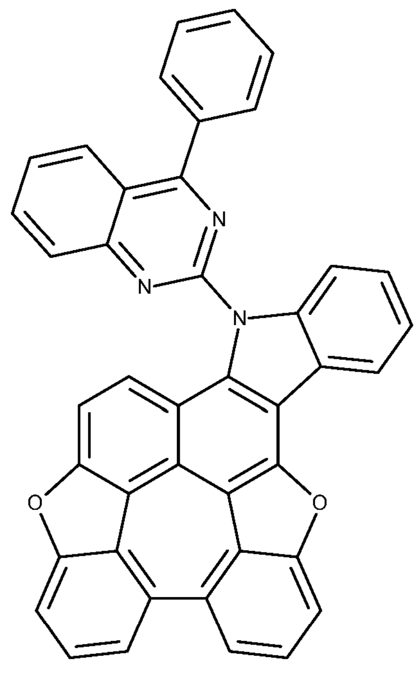 | 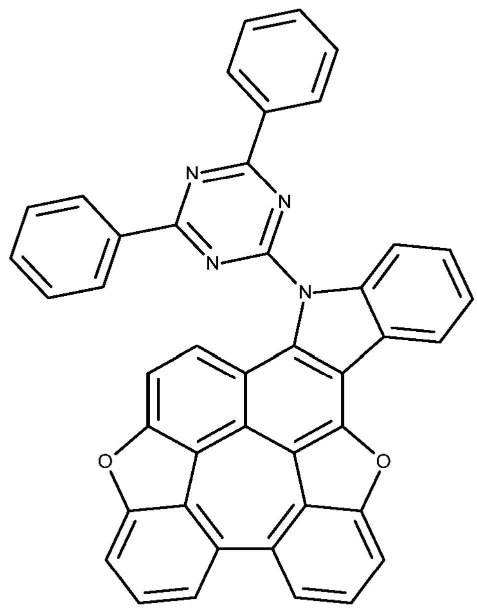 |

| | |
|---|---|
| 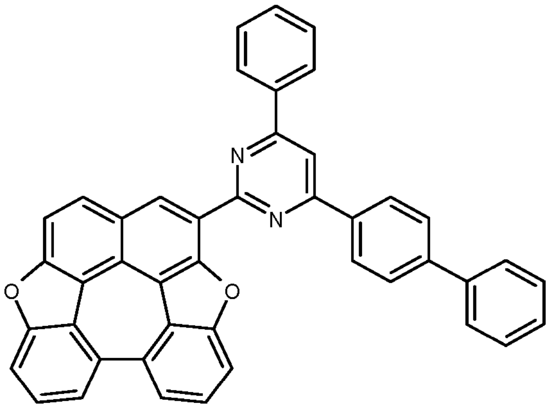 | 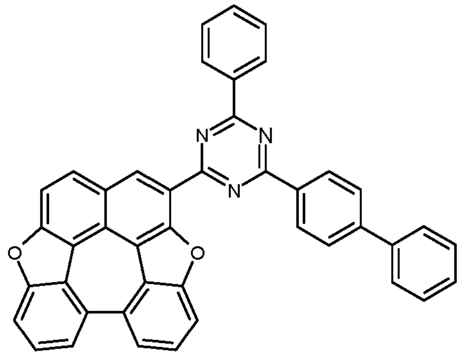 |
| 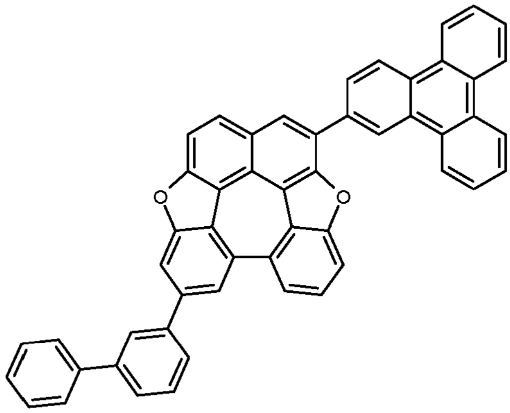 | 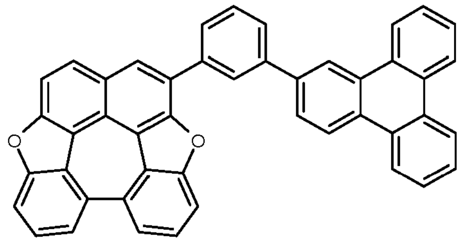 |
| 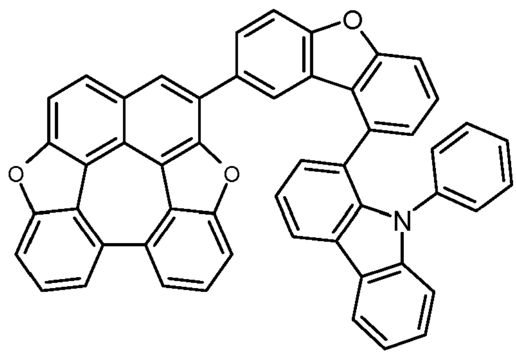 | 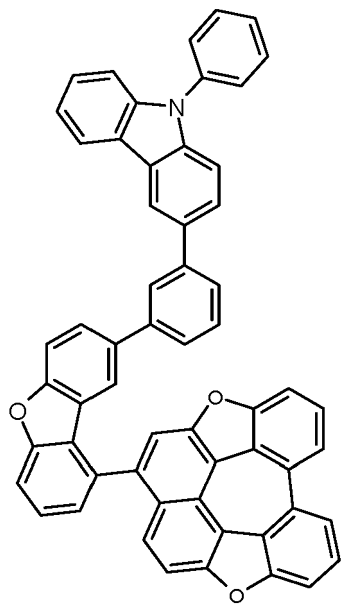 |

| | |
|---|---|
| 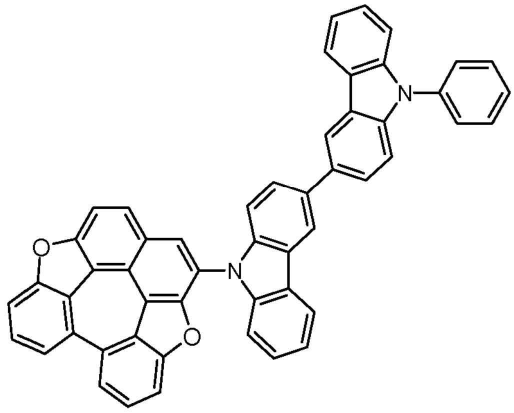 | 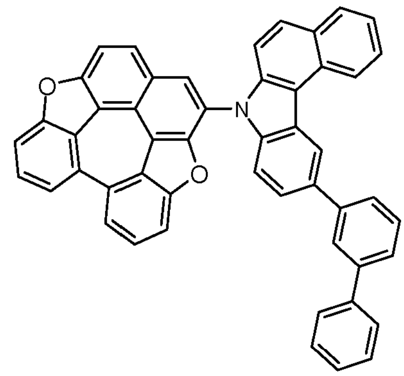 |
| 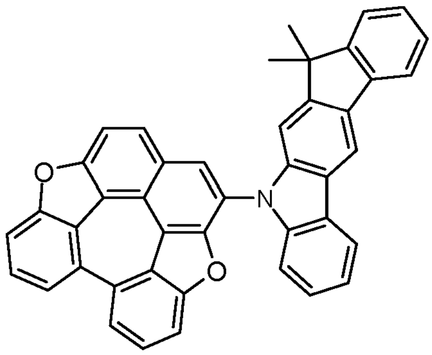 | 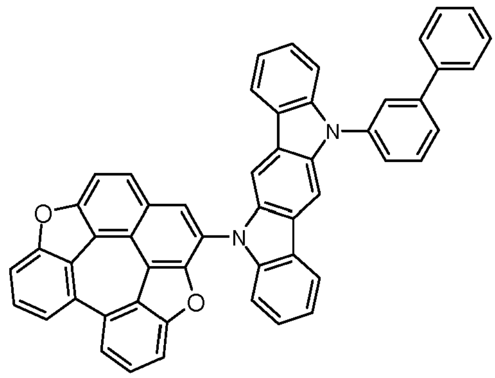 |
| 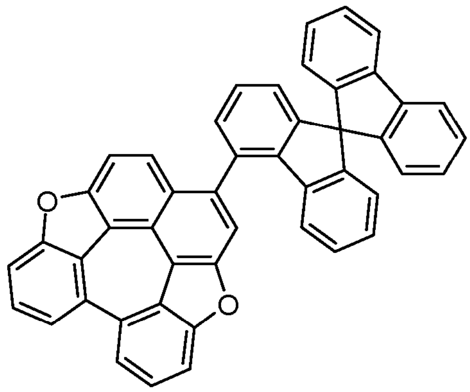 | 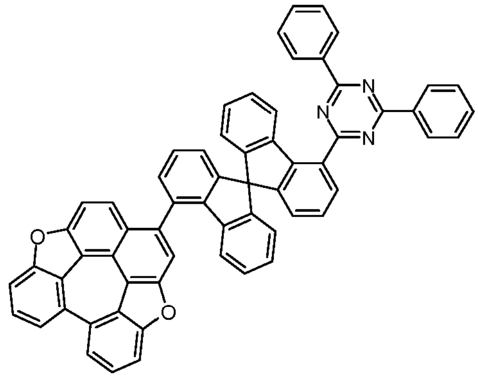 |
| 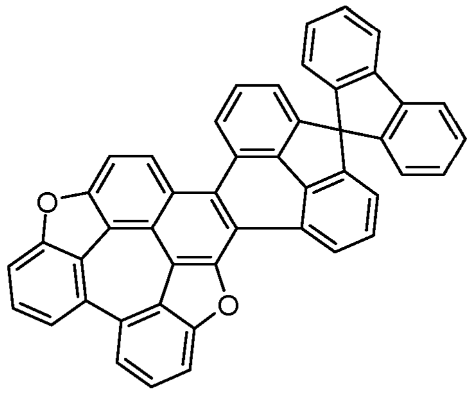 | 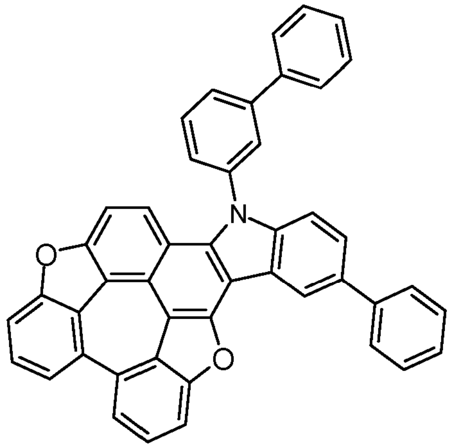 |

| | |
|---|---|
| 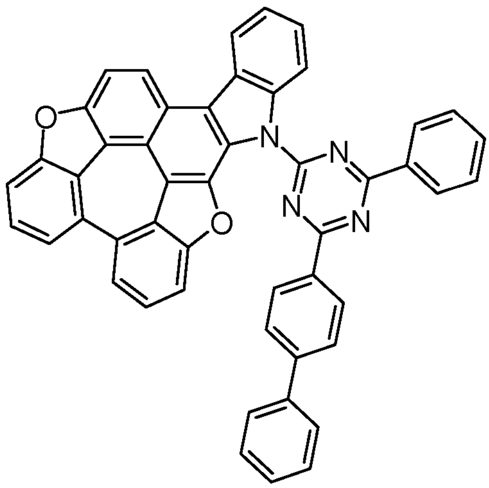 | 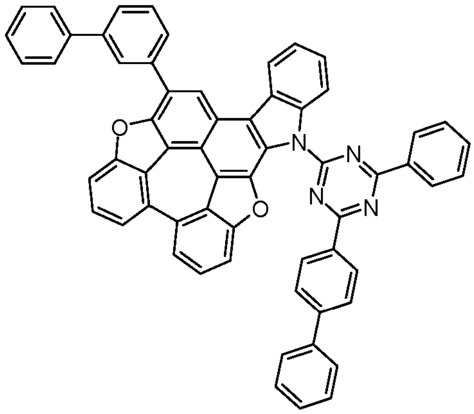 |
| 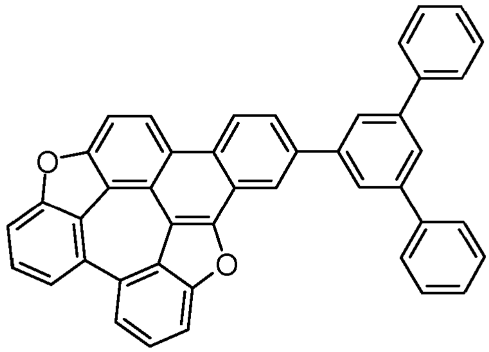 | 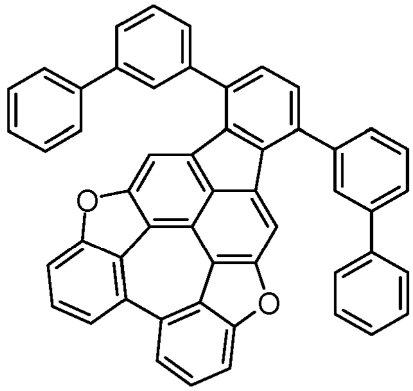 |
| 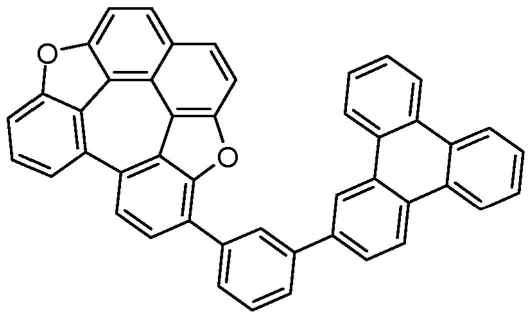 | 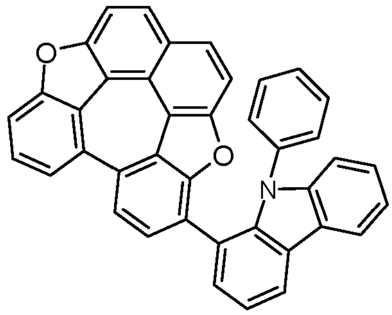 |
| 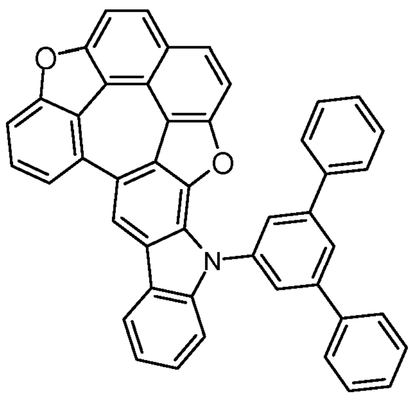 | 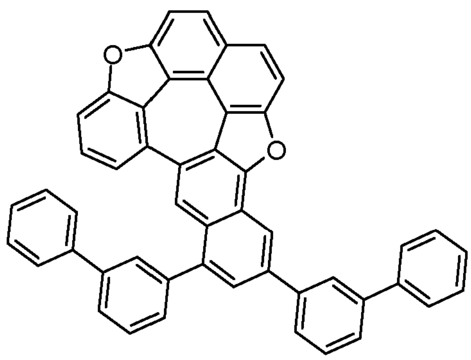 |

| | |
|---|---|
| 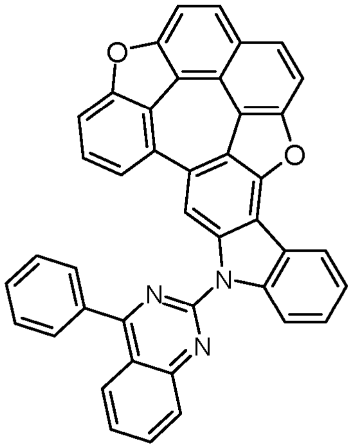 | 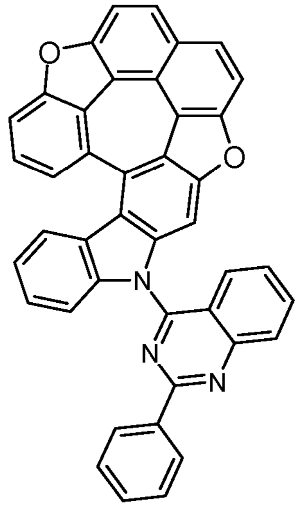 |
| 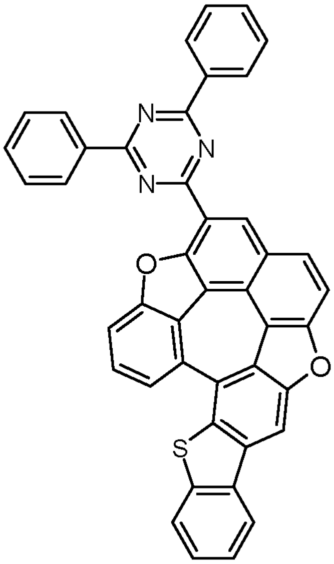 | 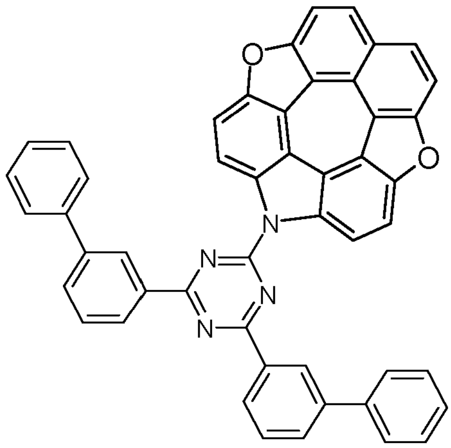 |
| 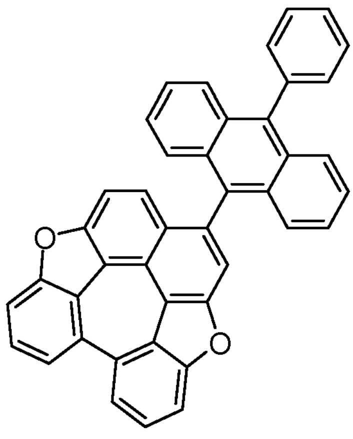 | 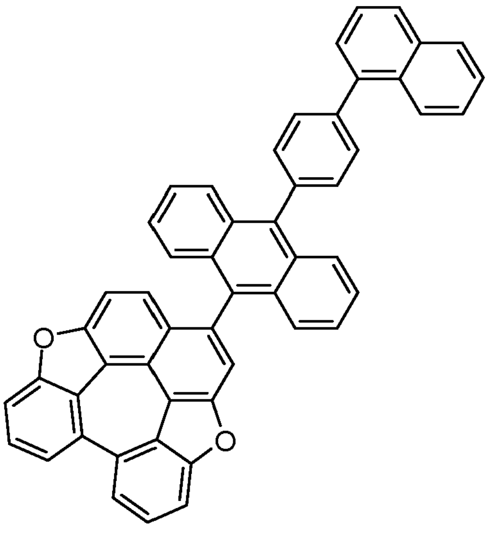 |

| | |
|---|---|
| 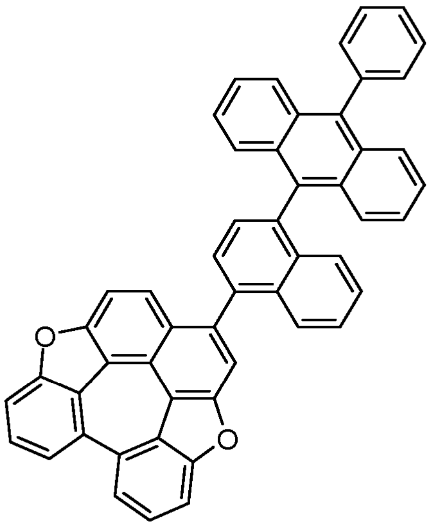 | 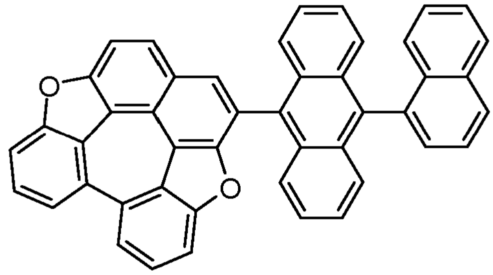 |
| 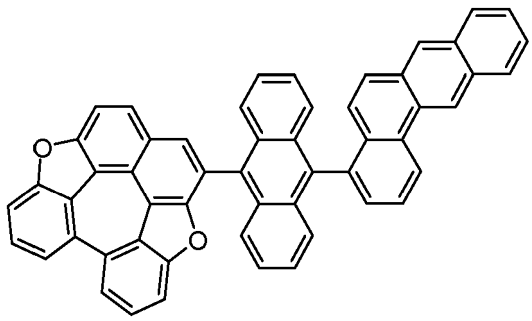 | 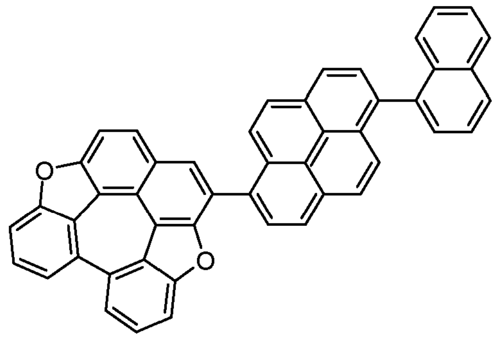 |
| 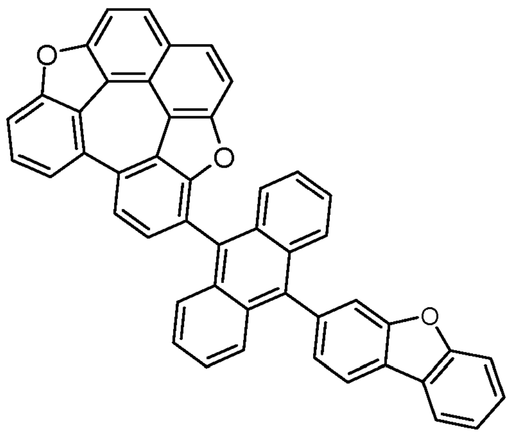 | 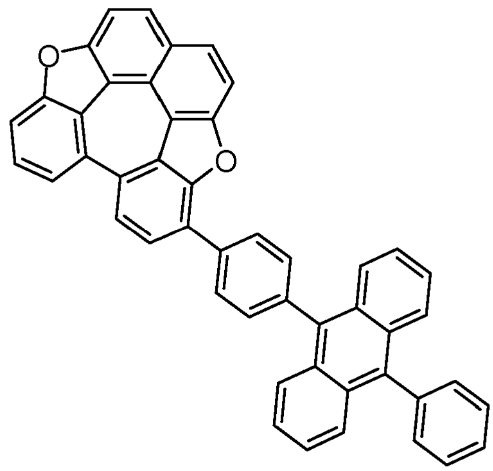 |

| | |
|---|---|
| 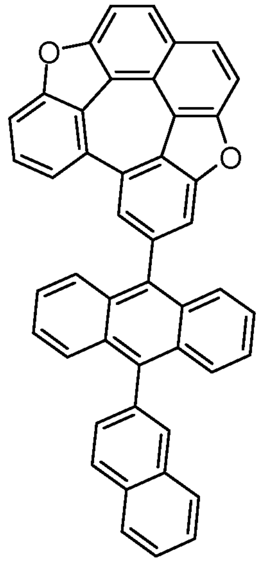 | 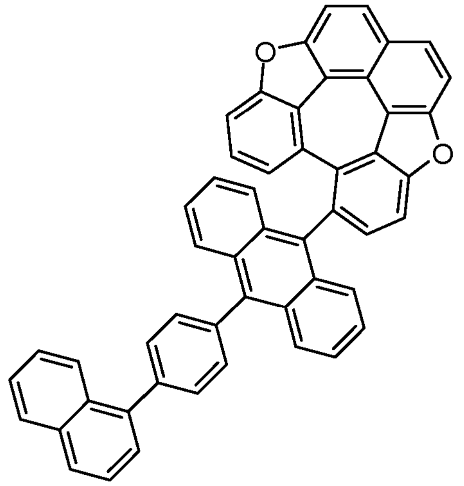 |
| 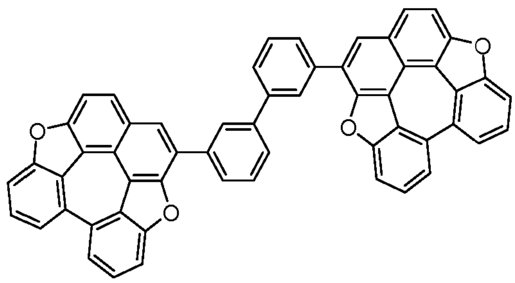 | 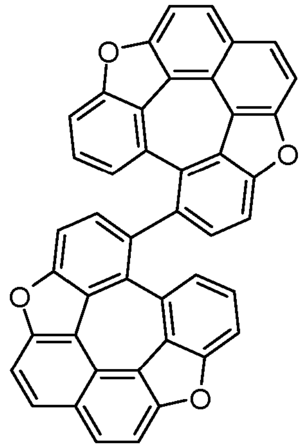 |
| 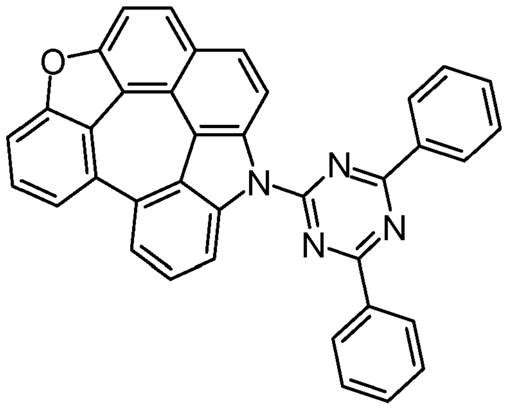 | 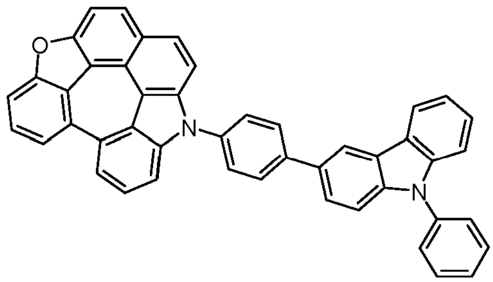 |

| | |
|---|---|
| 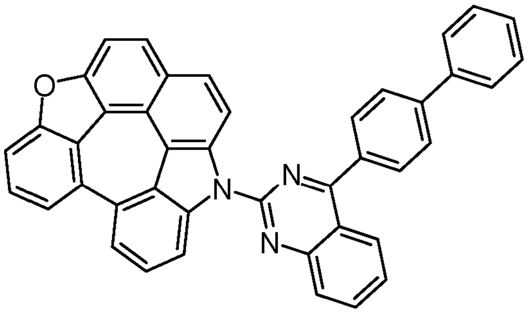 | 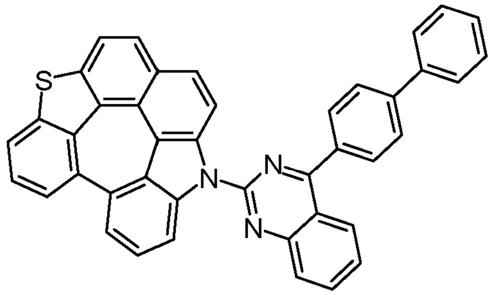 |
| 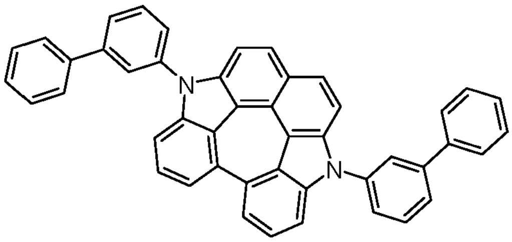 | 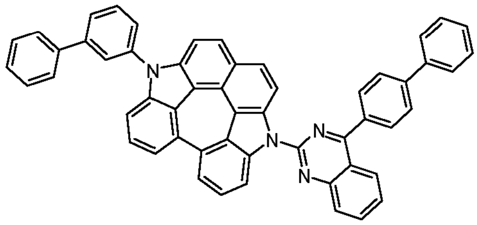 |
| 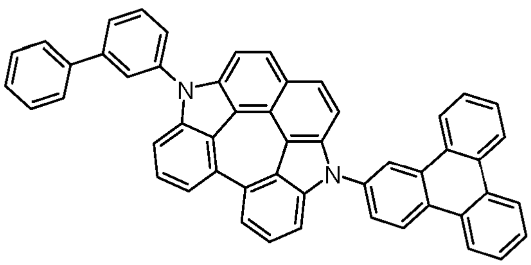 | 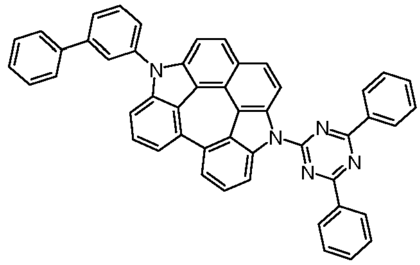 |
| 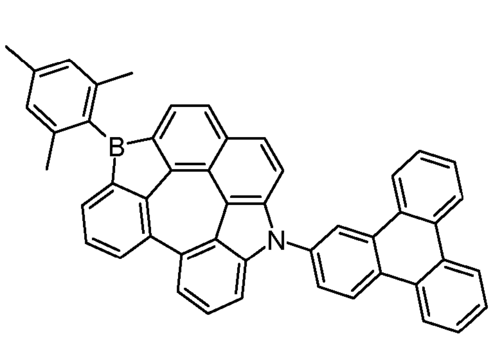 | 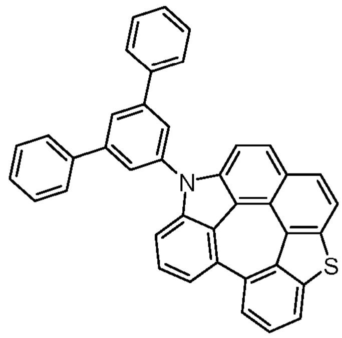 |
| 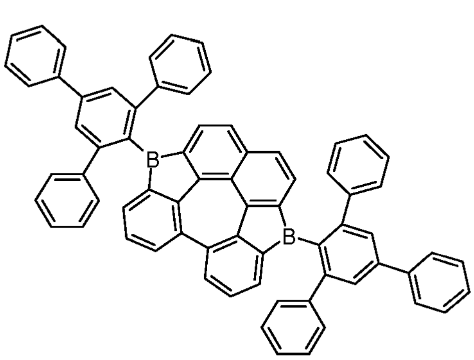 | 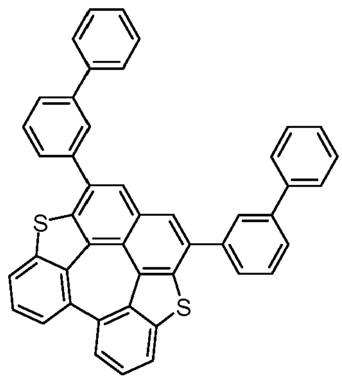 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung gemäß Anspruch 18, bei dem in einer Kupplungsreaktion eine Verbindung, umfassend eine heterocyclische Struktur, mit einer Verbindung, umfassend mindestens eine aromatische oder heteroaromatische Gruppe, verbunden wird.

Geeignete Verbindungen, umfassend mindestens eine heterocyclische Struktur, können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen, umfassend mindestens eine aromatische oder heteroaromatische Gruppe, umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die Verbindungen, umfassend Strukturen gemäß Formel (Ia) bis (If) in hoher Reinheit, bevorzugt mehr als 99 % (bestimmt mittels $^1$H-NMR und/oder HPLC) erhalten.

Die Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, so dass die Verbindungen beispielsweise in Toluol oder Xylol bei Raumtemperatur in ausreichender Konzentration löslich sind, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die Verbindungen, umfassend mindestens eine Struktur der Formeln (Ia) bis (If), (IIa) bis (IIf), (IIIa) bis (IIIf), (IVa) bis (IVf) und/oder (Va) bis (Vk) bereits eine gesteigerte Löslichkeit in diesen Lösungsmitteln besitzen.

Ferner können die Verbindungen eine oder mehrere vernetzbare Gruppen enthalten. "Vernetzbare Gruppe" bedeutet eine funktionelle Gruppe, die in der Lage ist, irreversibel zu reagieren. Dadurch wird ein vernetztes Material gebildet, das unlöslich ist. Die Vernetzung kann gewöhnlich durch Wärme oder durch UV-, Mikrowellen-, Röntgen- oder Elektronenstrahlung unterstützt werden. Hierbei kommt es bei der Vernetzung zu wenig Nebenprodukt-bildung. Zudem vernetzen die vernetzbaren Gruppen, die in den funktionalen Verbindungen enthalten sein können, sehr leicht, so dass geringere Energiemengen für die Vernetzung erforderlich sind (z.B. < 200°C bei der thermischen Vernetzung).

Beispiele für vernetzbare Gruppen sind Einheiten, die einen heterocyclischen, additionspolymerisierbaren Rest enthalten. Vernetzbare Gruppen umfassen unter anderem Di(hydrocarbyl)amino, Cyclobutylphenyl und Tri($C_{1-4}$)-alkylsilyl. Besonders bevorzugt ist Cyclobutylphenyl.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere gemäß Anspruch 14 enthaltend eine oder mehrere der Strukturen der Formeln (Ia) bis (If), (IIa) bis (IIf), (IIIa) bis (IIIf), (IVa) bis (IVf) und/oder (Va) bis (Vk), wobei ein oder mehrere Bindungen der erfindungsgemäßen Verbindungen oder der Strukturen der Formeln (Ia) bis (If), (IIa) bis (IIf), (IIIa) bis (IIIf), (IVa) bis (IVf) und/oder (Va) bis (Vk) zum Polymer, Oligomer oder Dendrimer vorhanden sind. Je nach Verknüpfung der Strukturen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten

der erfindungsgemäßen Verbindungen in Oligomeren, Dendrimeren und Polymeren gelten dieselben Bevorzugungen, wie oben beschrieben.

Zur Herstellung der Oligomere oder Polymere werden die Monomere homopolymerisiert oder mit weiteren Monomeren copolymerisiert. Bevorzugt sind Copolymere, wobei die Einheiten gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung gemäß Anspruch 16 bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Die weitere Verbindung kann auch polymer sein.

Nochmals ein weiterer Gegenstand der vorliegenden Erfindung ist daher eine Zusammensetzung enthaltend eine erfindungsgemäße Verbindung gemäß Anspruch 15. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochtransportmaterialien, Lochinjektionsmaterialien, Elektronenblockiermaterialien, Lochblockiermaterialien, Wide-Band-Gap-Materialien und n-Dotanden.

Anspruch 15 umfasst auch eine Zusammensetzung, enthaltend wenigstens eine erfindungsgemäße Verbindung, bevorzugt eine Verbindung gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0

eV oder mehr, ganz bevorzugt von 3,3 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Anspruch 15 umfasst auch eine Zusammensetzung umfassend wenigstens eine erfindungsgemäße Verbindung gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/001990, WO 2018/019687, WO 2018/019688, WO 2018/041769, WO 2018/054798, WO 2018/069196, WO 2018/069197, WO 2018/069273, WO 2018/178001, WO 2018/177981, WO 2019/020538, WO 2019/115423, WO

2019/158453 und WO 2019/179909 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind in der folgenden Tabelle aufgeführt.

| | | |
|---|---|---|
| 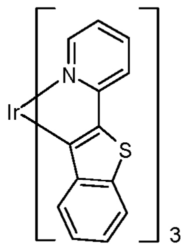 | 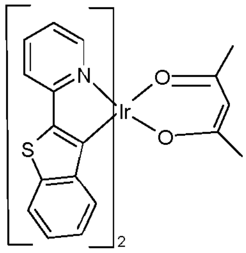 | 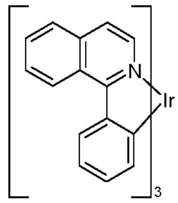 |
| 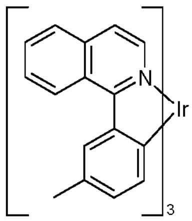 | 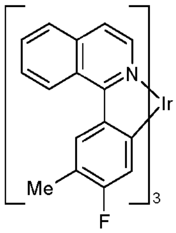 | 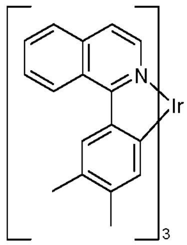 |

| | | |
|---|---|---|
| 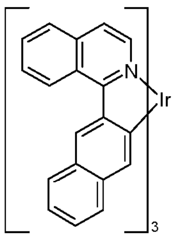 | 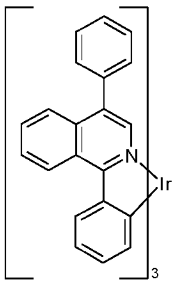 | 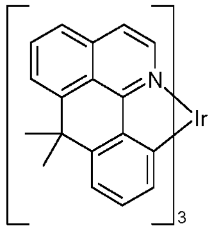 |
| 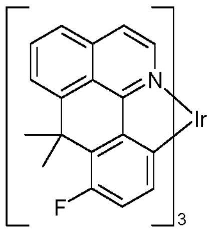 | 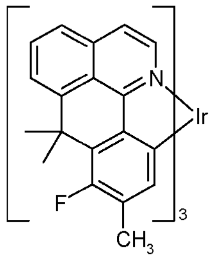 | 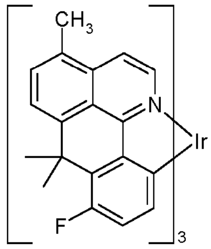 |
| 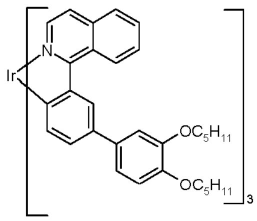 | 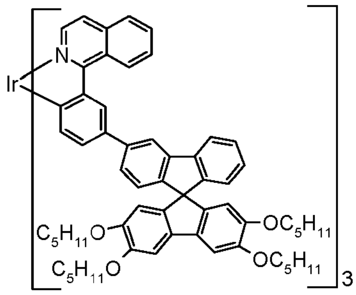 | 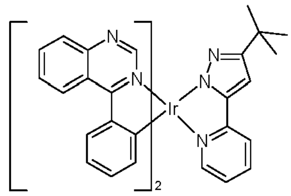 |
| 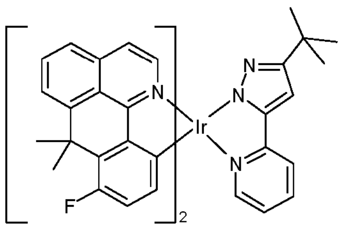 | 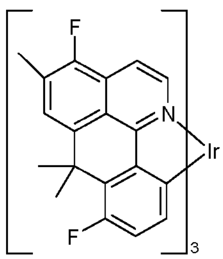 | 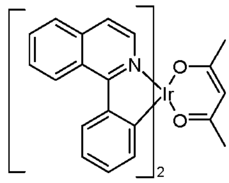 |
| 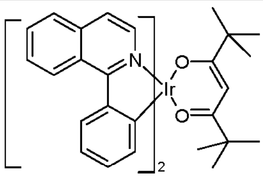 | 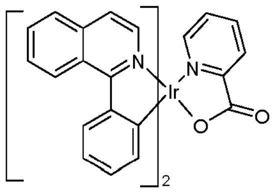 | 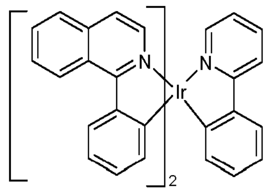 |
| 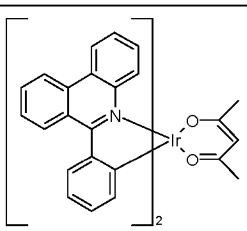 | 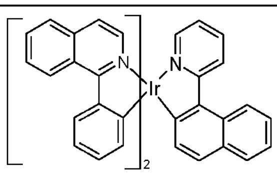 | 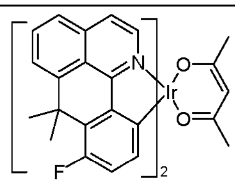 |

| | | |
|---|---|---|
| 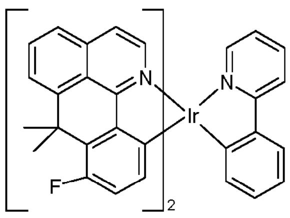 | 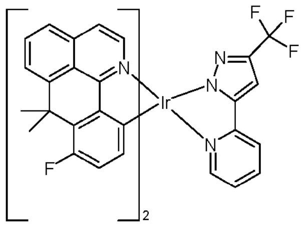 | 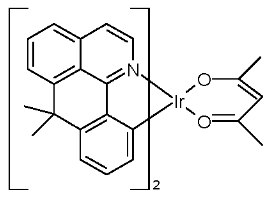 |
| 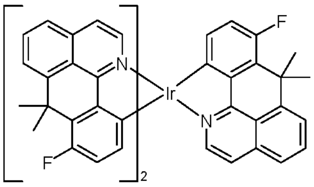 | 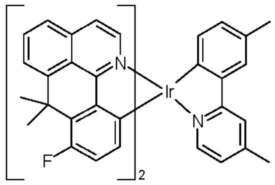 | 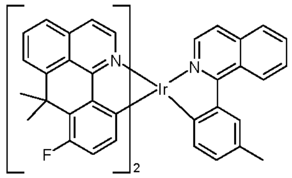 |
| 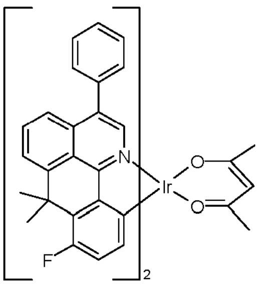 | 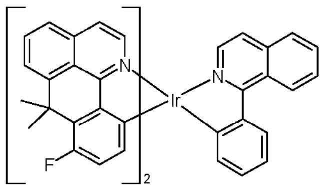 | 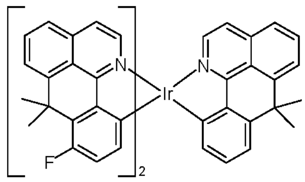 |
| 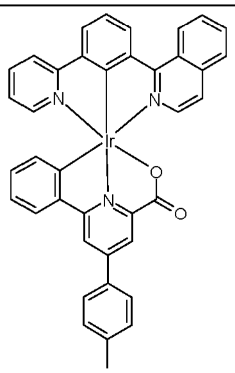 | 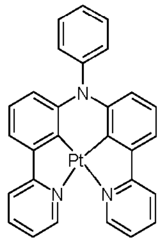 | 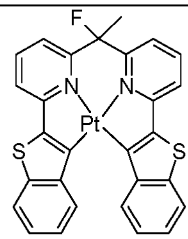 |
| 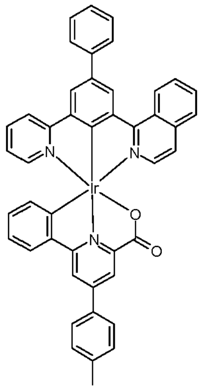 | 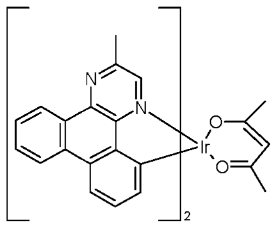 | 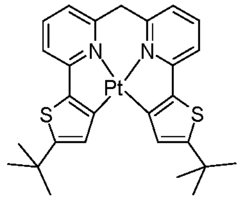 |

| | | |
|---|---|---|
| 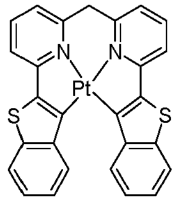 | 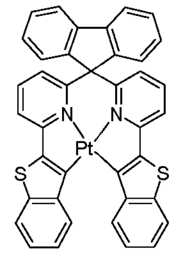 | 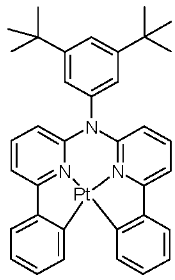 |
| 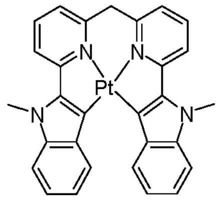 | 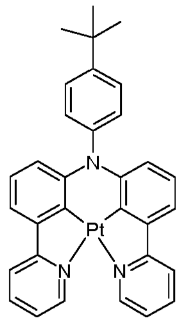 | 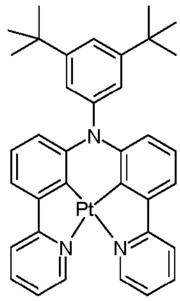 |
| 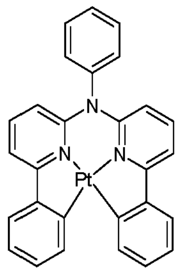 | 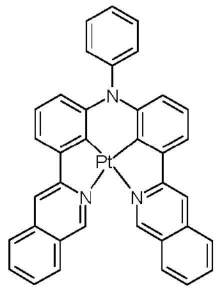 | 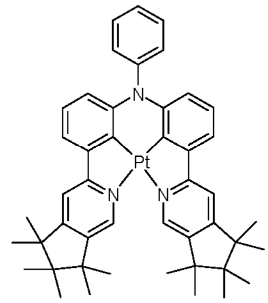 |
| 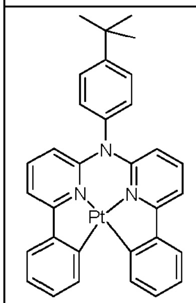 | 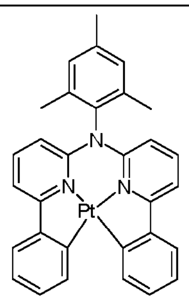 | 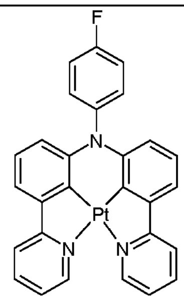 |
| 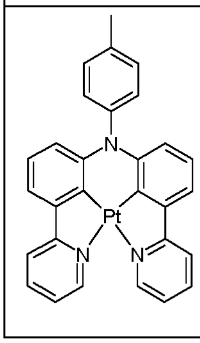 | 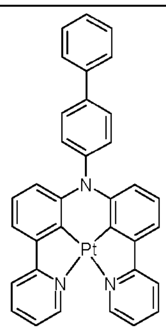 | 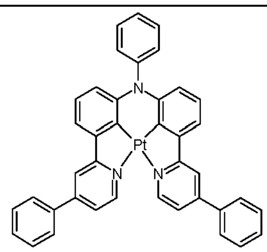 |

| | | |
|---|---|---|
| 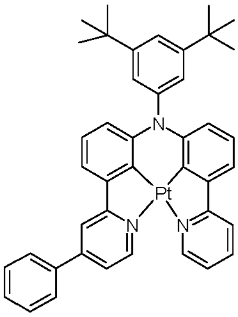 | 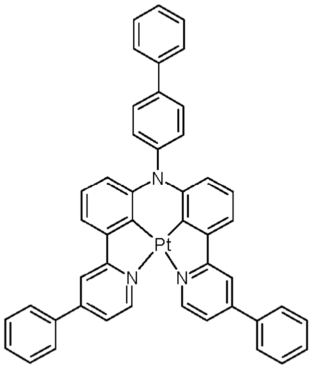 | 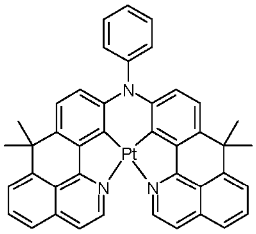 |
| 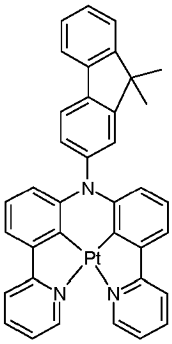 | 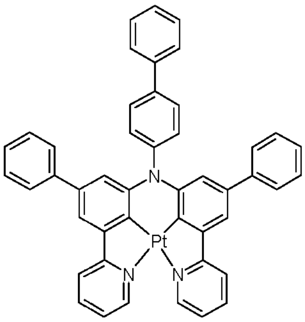 | 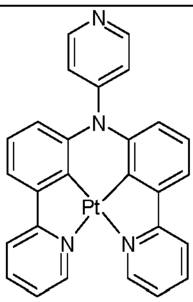 |
| 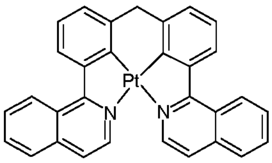 | 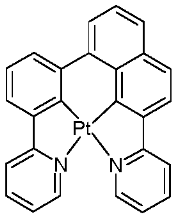 | 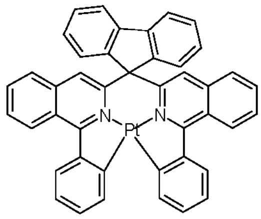 |
| 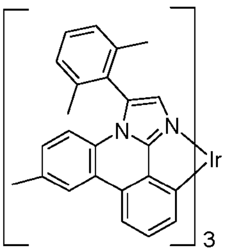 | 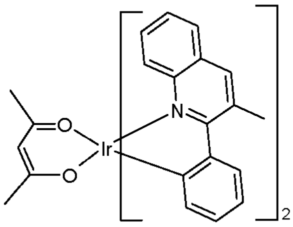 | 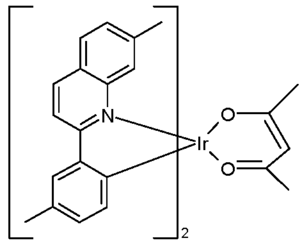 |
| 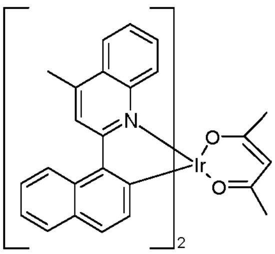 | 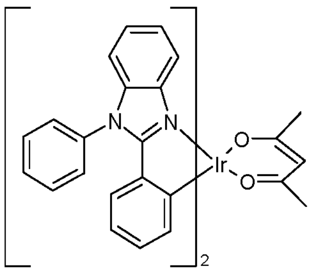 | 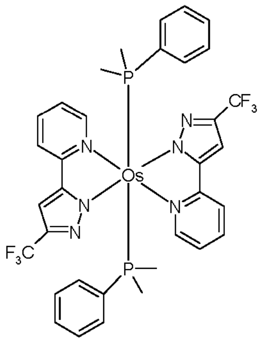 |

| | | |
|---|---|---|
| 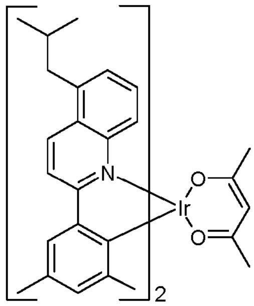 | 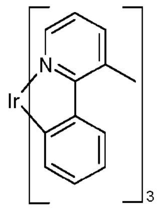 | 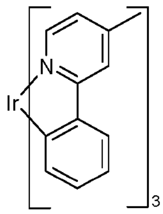 |
| 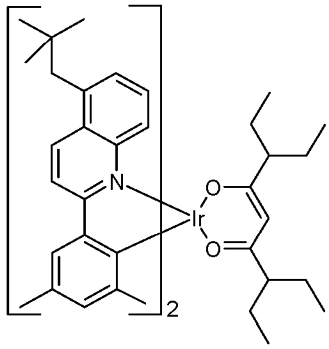 | 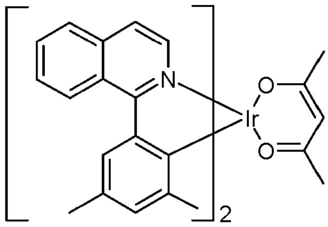 | 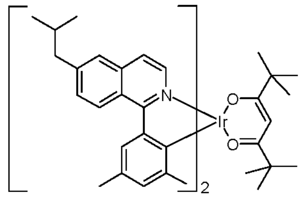 |
| 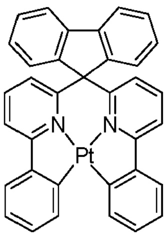 | 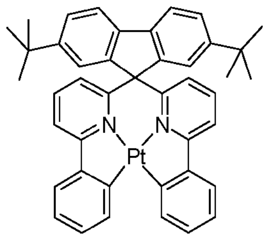 | 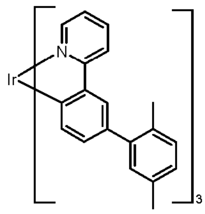 |
| 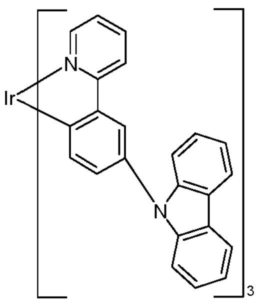 | 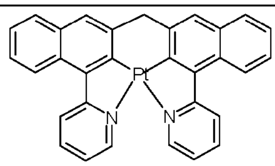 | 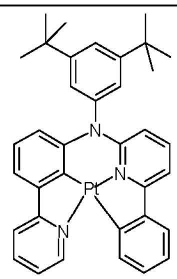 |
| 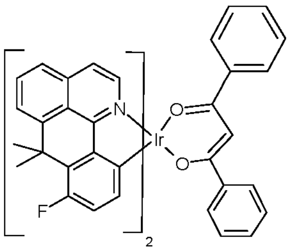 | 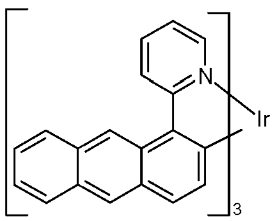 | 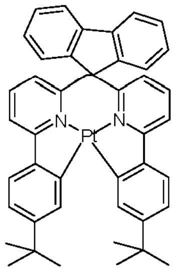 |

| | | |
|---|---|---|
| 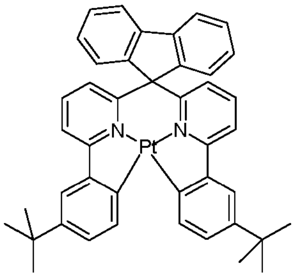 | 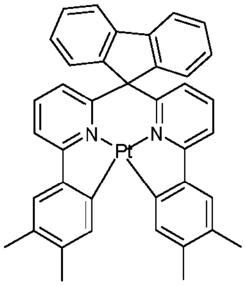 | 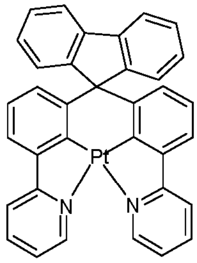 |
| 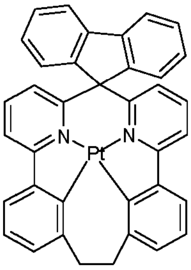 | 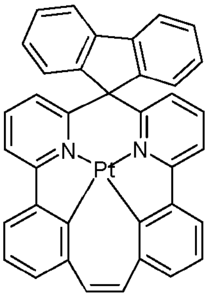 | 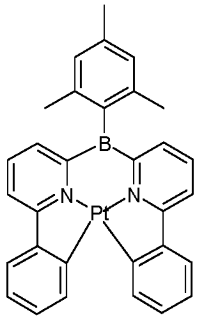 |
| 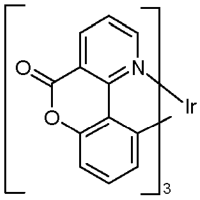 | 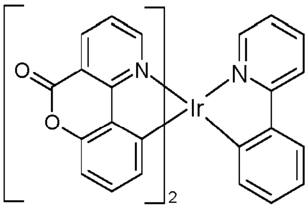 | 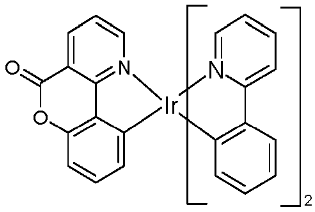 |
| 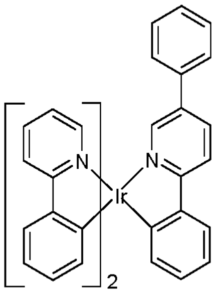 | 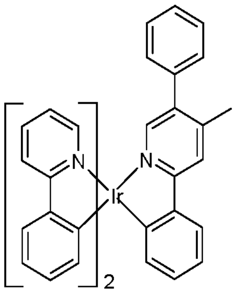 | 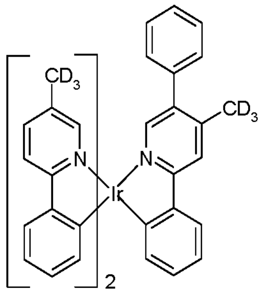 |
| 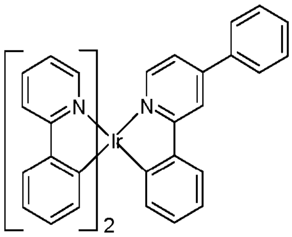 | 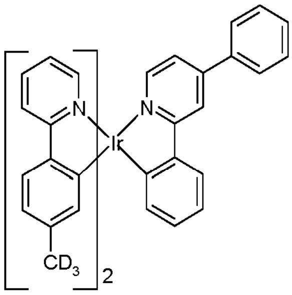 | 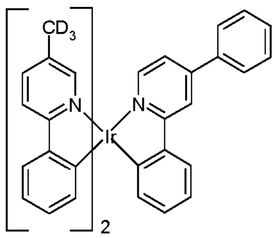 |
| 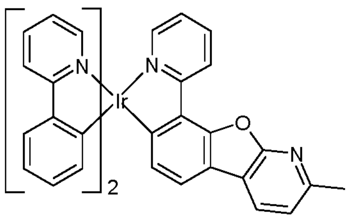 | 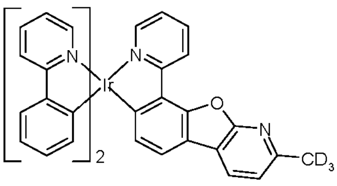 | 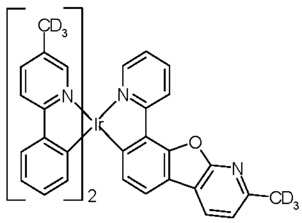 |

| | | |
|---|---|---|
| 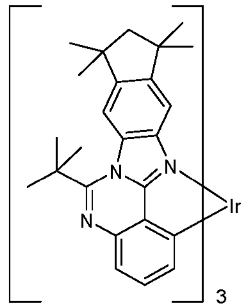 | 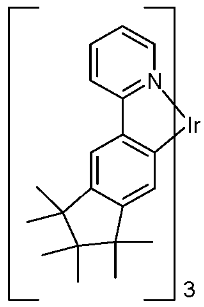 | 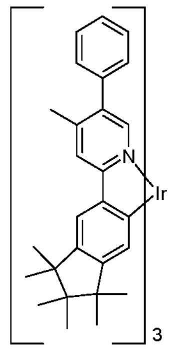 |
| 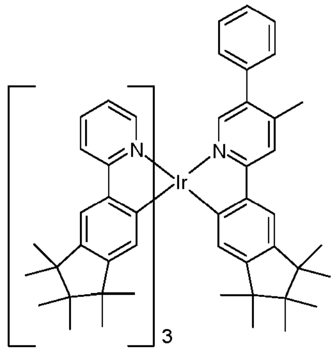 | 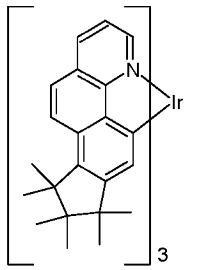 | 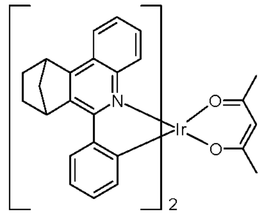 |
| 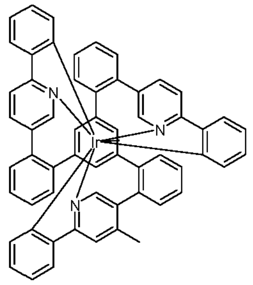 | 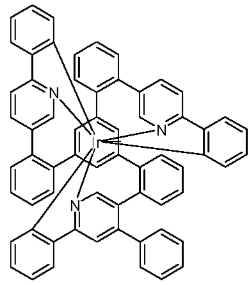 | 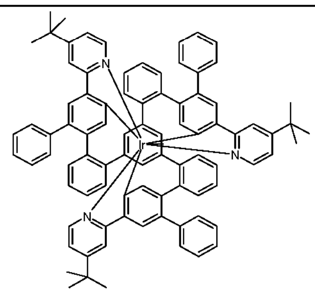 |
| 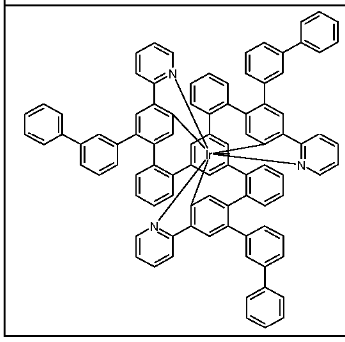 | 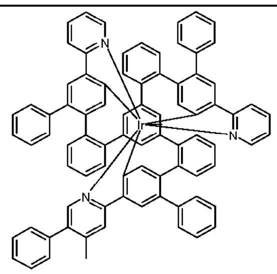 | 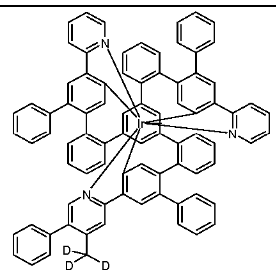 |

| | | |
|---|---|---|
| 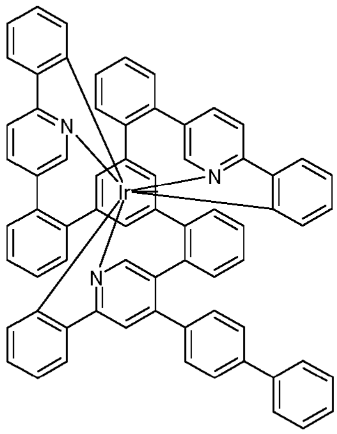 | 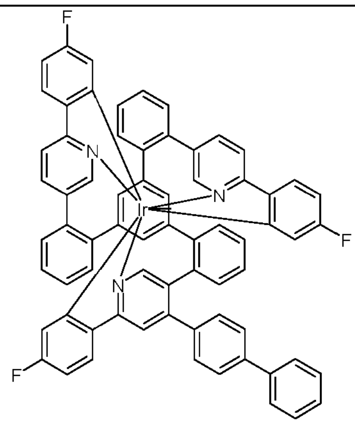 | 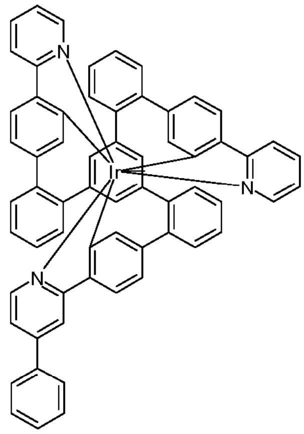 |
| 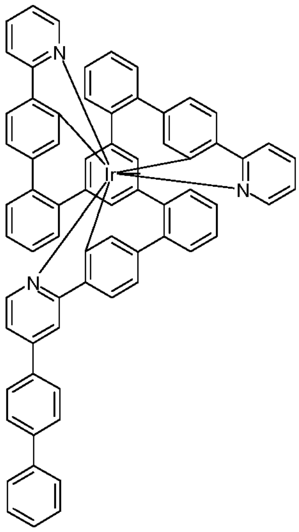 | 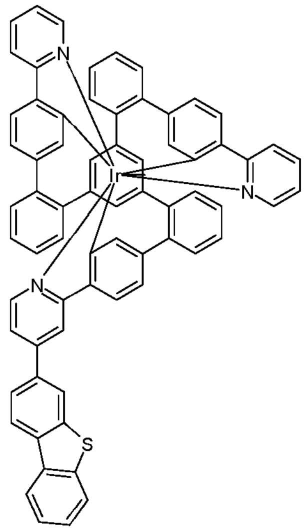 | 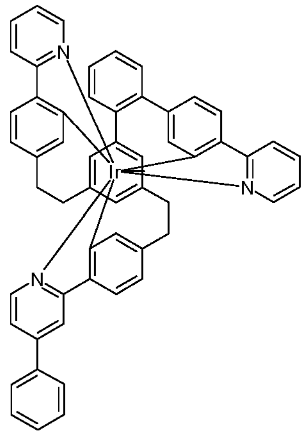 |
| 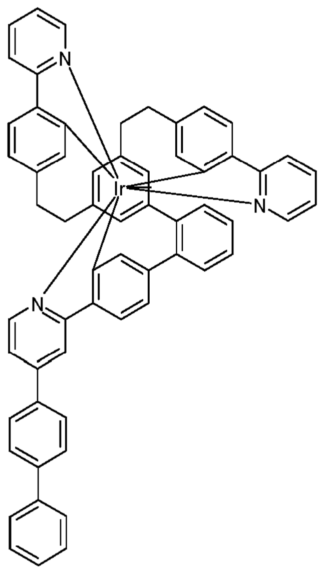 | 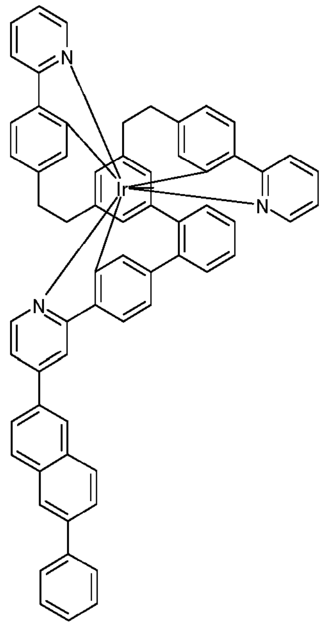 | 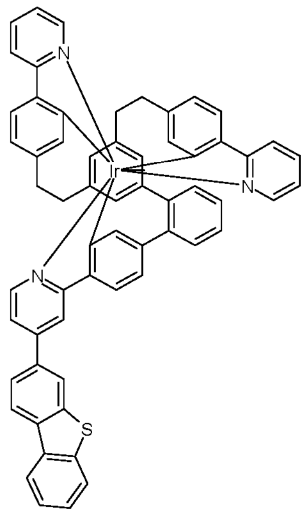 |

| | | |
|---|---|---|
| 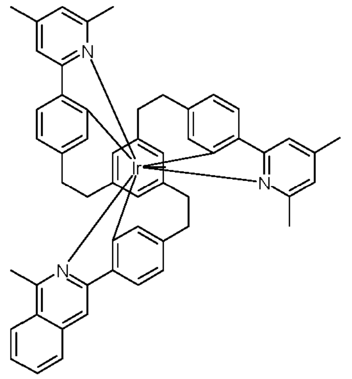 | 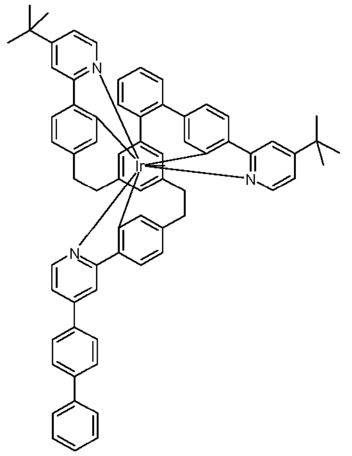 | 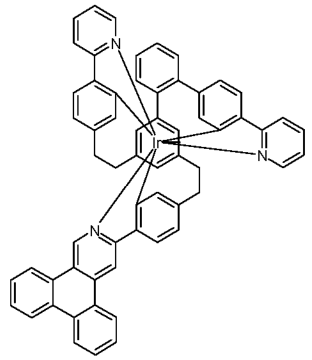 |

Wenn die Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung dabei als einziges Matrixmaterial ("single host") für den phosphoreszierenden Emitter eingesetzt.

In einer bevorzugten Ausführungsform der Erfindung enthält die erfindungsgemäße organische Elektrolumineszenzvorrichtung die Verbindungumfassend Strukturen gemäß Formeln (Ia) bis (If), (IIa) bis (IIf), (IIIa) bis (IIIf), (IVa) bis (IVf) und/oder (Va) bis (Vk) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

In einer weiterhin besonders bevorzugten Ausführungsform der vorliegenden Erfindung umfasst eine erfindungsgemäße organische Elektrolumineszenzvorrichtung die Verbindung umfassend Strukturen gemäß Formeln (Ia) bis (If), (IIa) bis (IIf), (IIIa) bis (IIIf), (IVa) bis (IVf) und/oder (Va) bis (Vk) bzw. die oben aufgeführten bevorzugten Ausführungsformen in einer Lochtransportschicht oder einer Elektronentransportschicht.

Die vorliegende Erfindung betrifft daher auch eine Zusammensetzung gemäß Anspruch 15 enthaltend wenigstens eine erfindungsgemäße Verbindung, bevorzugt eine Verbindung gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen sowie wenigstens ein weiteres Matrixmaterial.

Geeignete Matrixmaterialien, welche in Kombination mit den Verbindungen gemäß Formeln (Ia) bis (If), (IIa) bis (IIf), (IIIa) bis (IIIf), (IVa) bis (IVf) und/oder (Va) bis (Vk) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706,

WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte CarbazolDerivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2, Lactame, z. B. gemäß WO 2011/116865, WO 2011/137951 oder WO 2013/064206, 4-Spirocarbazol-Derivate, z. B. gemäß WO 2014/094963 oder WO 2015/192939. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triazine, Chinazoline, Chinoxaline, Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

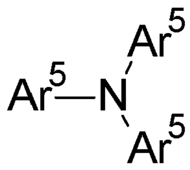

Formel (TA-1) ,

wobei $Ar^5$ gleich oder verschieden bei jedem Auftreten ein aromatisches oder heteroaromatisches Ringsystem mit 6 bis 40 C-Atomen, das jeweils mit einem oder mehreren Resten $R^1$ substituiert sein kann, darstellt, wobei optional zwei oder mehr benachbarte Substituenten $R^1$ ein mono- oder polycyclisches, aliphatisches Ringsystem bilden können, das mit einem oder mehreren Resten $R^2$ substituiert sein kann, wobei die Symbole $R^1$ und $R^2$ die zuvor, insbesondere für Formel (Ia) bis (If) genannte Bedeutung aufweisen. Vorzugsweise stellt $Ar^5$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^1$ substituiert sein kann, vorzugsweise jedoch unsubstituiert ist.

Beispiele für geeignete Gruppen $Ar^5$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, Indenocarbazolyl, 1-, 2-, 3- oder 4-Dibenzothienyl und 1-, 2-, 3- oder 4-Carbazolyl, die jeweils durch einen oder mehrere Reste $R^1$ substituiert sein können, bevorzugt aber unsubstituiert sind.

Bevorzugt sind die Gruppen $Ar^5$ gleich oder verschieden bei jedem Auftreten ausgewählt aus den oben genannten Gruppen $R^1$-1 bis $R^1$-43, besonders bevorzugt $R^1$-1 bis $R^1$-28.

In einer bevorzugten Ausführungsform der Verbindungen der Formel (TA-1) ist mindestens eine Gruppe $Ar^5$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^5$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^5$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^5$ ausgewählt aus einer Fluoren- oder Spirobifluorengruppe, insbesondere einer 4-Fluoren- bzw. 4-Spirobifluorengruppe, und eine Gruppe $Ar^5$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^5$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

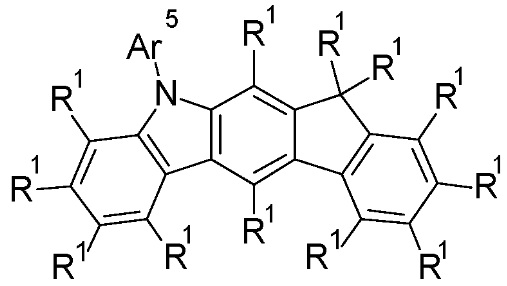

Formel (TA-2)

,

wobei $Ar^5$ und $R^1$ die oben insbesondere für Formeln (I) und/oder (TA-1) aufgeführten Bedeutungen aufweisen. Dabei sind bevorzugte Ausführungsformen der Gruppe $Ar^5$ die oben aufgeführten Strukturen $R^1$-1 bis $R^1$-43, besonders bevorzugt $R^1$-1 bis $R^1$-28.

Eine bevorzugte Ausführungsform der Verbindungen der Formel (TA-2) sind die Verbindungen der folgenden Formel (TA-2a),

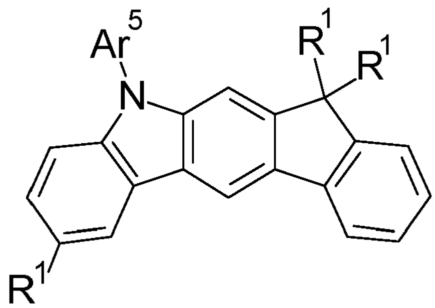

Formel (TA-2a)

,

wobei $Ar^5$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

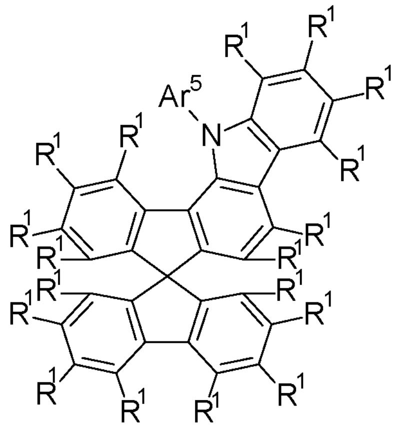

Formel (TA-3)

,

wobei $Ar^5$ und $R^1$ die oben, insbesondere für Formel (TA-1), aufgeführten Bedeutungen aufweisen. Dabei sind bevorzugte Ausführungsformen der Gruppe $Ar^5$ die oben aufgeführten Strukturen $R^1$-1 bis $R^1$-43, besonders bevorzugt $R^1$-1 bis $R^1$-28.

Eine bevorzugte Ausführungsform der Verbindungen der Formel (TA-3) sind die Verbindungen der folgenden Formel (TA-3a),

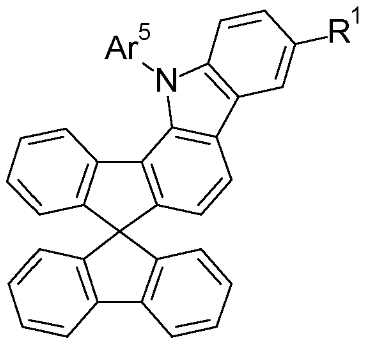

Formel (TA-3a) ,

wobei $Ar^5$ und $R^1$ die oben, insbesondere für Formel (TA-1), aufgeführten Bedeutungen aufweisen. Dabei sind bevorzugte Ausführungsformen der Gruppe $Ar^5$ die oben aufgeführten Strukturen $R^1$-1 bis $R^1$-43, besonders bevorzugt $R^1$-1 bis $R^1$-28.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

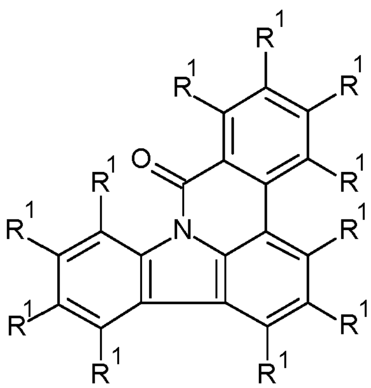

Formel (LAC-1) ,

wobei $R^1$ die oben aufgeführte Bedeutung aufweist.

Eine bevorzugte Ausführungsform der Verbindungen der Formel (LAC-1) sind die Verbindungen der folgenden Formel (LAC-1a),

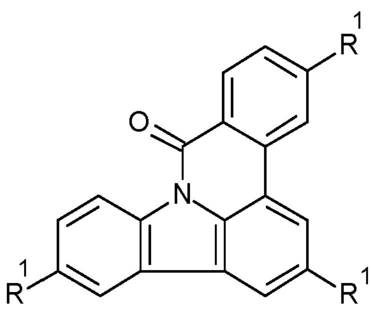

Formel (LAC-1a) ,

wobei $R^1$ die oben, insbesondere für Formel (Ia) bis (If) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung gemäß Formeln (Ia) bis (If), (IIa) bis (IIf), (IIIa) bis (IIIf), (IVa) bis (IVf) und/oder (Va) bis (Vk) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend eine Verbindung gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen umfassend Strukuren gemäß Formeln (Ia) bis (If), (IIa) bis (IIf), (IIIa) bis (IIIf), (IVa) bis (IVf) und/oder (Va) bis (Vk) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen

Verbindung gemäß Anspruch 12 oder 13 und/oder eines erfindungsgemäßen Oligomers, Polymers oder Dendrimers gemäß Anspruch 14 in einer elektronischen Vorrichtung als fluoreszierenden Emitter, Emitter, der TADF (thermally activated delayed fluorescence) zeigt, Hostmaterial, Elektronentransportmaterial, Elektroneninjektionsmaterial, Lochtransportmaterial, Lochinjektionsmaterial, Elektronenblockiermaterial, Lochblockiermaterial und/oder Wide-Band-Gap-Material, vorzugsweise als fluoreszierenden Emitter (Singulet-Emitter), Hostmaterial, Lochtransportmaterial und/oder Elektronentransportmaterial.

Ein nochmals weiterer Gegenstand der vorliegenden Erfindung ist eine elektronische Vorrichtung enthaltend mindestens eine erfindungsgemäße Verbindung gemäß Anspruch 19. Eine elektronische Vorrichtung im Sinne der vorliegenden Erfindung ist eine Vorrichtung, welche Anode, Kathode und mindestens eine dazwischen liegende Schicht aufweist, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-Elektrolumineszenzvorrichtung handeln, insbesondere für weiß emittierende OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung gemäß Formel (Ia) bis (If) bzw. die oben ausgeführten bevorzugten Ausführungsformen in einer emittierenden Schicht als Matrixmaterial für phosphoreszierende Emitter, für Emitter, die TADF (thermally activated delayed fluorescence) zeigen, insbesondere für fluoreszierende Emitter oder phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial für rot, orange oder gelb phosphoreszierende Emitter, insbesondere für rot phosphoreszierende Emitter, in einer emittierenden Schicht oder als Elektronentransport- bzw. Lochblockiermaterial in einer Elektronentransport- bzw. Lochblockierschicht eingesetzt.

Ferner ist eine elektronische Vorrichtung, vorzugsweise eine organische Elektrolumineszenzvorrichtung bevorzugt, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende

Verbindung.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend erfindungsgemäße Verbindungen, Oligomere, Polymere oder Dendrimere, bzw. die zuvor und nachfolgend ausgeführten bevorzugten Ausführungsformen, insbesondere als elektronenleitende Materialien und/oder Lochtransportmaterialien oder als Matrixmaterialien, weisen eine sehr gute Lebensdauer auf.

2. Elektronische Vorrichtungen, insbesondere organische Elektrolumineszenzvorrichtungen enthaltend erfindungsgemäße Verbindungen, Oligomere, Polymere oder Dendrimere, bzw. die zuvor und nachfolgend ausgeführten bevorzugten Ausführungsformen insbesondere als Elektronentransport-Materialien, Lochtransportmaterialien und/oder als Hostmaterialien weisen eine hervorragende Effizienz auf. Insbesondere ist die Effizienz deutlich höher gegenüber analogen Verbindungen, die keine erfindungsgemäße Struktur enthalten. Hierbei bewirken die erfindungsgemäßen Verbindungen, Oligomere, Polymere oder Dendrimere bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.

3. Elektronische Vorrichtungen, insbesondere organische Elektrolumineszenzvorrichtungen enthaltend Verbindungen, Oligomere, Polymere oder Dendrimere bzw. die zuvor und nachfolgend ausgeführten bevorzugten Ausführungsformen als Elektronentransport-Materialien, Lochtransportmaterialien und/oder als Hostmaterialien weisen eine hervorragende Farbreinheit auf.

4. Die erfindungsgemäßen Verbindungen, Oligomere, Polymere oder Dendrimere bzw. die zuvor und nachfolgend ausgeführten bevorzugten Ausführungsformen zeigen eine sehr hohe thermische und photochemische Stabilität und führen zu Verbindungen mit einer sehr hohen Lebensdauer.

5. Mit Verbindungen, Oligomeren, Polymeren oder Dendrimere bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanälen vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

6. Verbindungen, Oligomere, Polymere oder Dendrimere bzw. die zuvor und nachfolgend ausgeführten bevorzugten Ausführungsformen weisen eine ausgezeichnete Glasfilmbildung auf.

7. Verbindungen, Oligomere, Polymere oder Dendrimere bzw. die zuvor und nachfolgend ausgeführten bevorzugten Ausführungsformen bilden aus Lösungen sehr gute Filme.

Diese oben genannten Vorteile gehen im Allgemeinen nicht mit einer Verschlechterung der weiteren elektronischen Eigenschaften einher.

In den weiteren Schichten der erfindungsgemäßen organischen Elektrolumineszenzvorrichtung können alle Materialien verwendet werden, wie sie üblicherweise gemäß dem Stand der Technik eingesetzt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die Erfindung wird durch die nachfolgenden Beispiele näher erläutert, ohne sie dadurch einschränken zu wollen.

**Beispiele**

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbinden die mehrere enantiomere, diastereomere oder tautomere Formen aufweisen können wird eine Form stellvertretend gezeigt.

Das nachfolgende Schema dient zum besseren Verständnis der nachfolgend detaillierter beschriebenen Syntheseroute

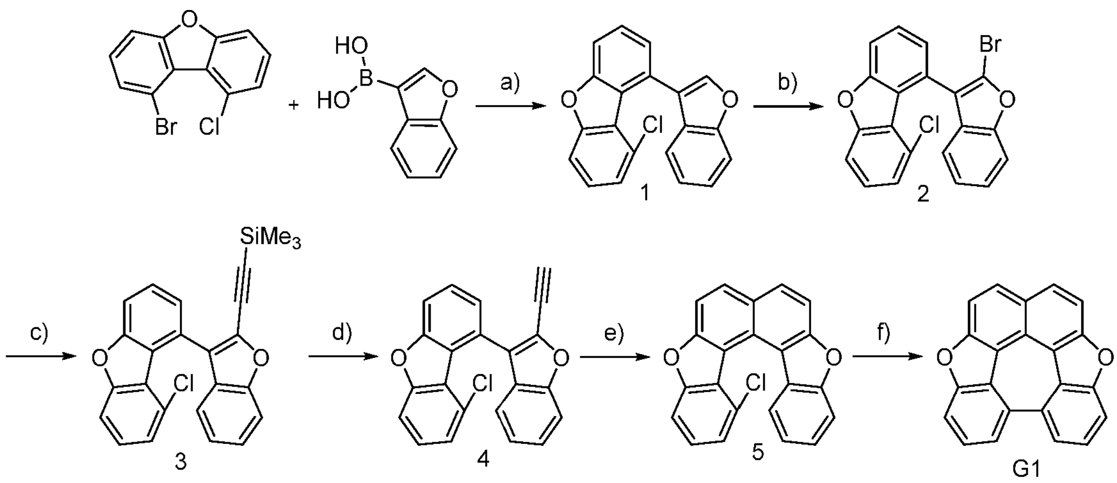

1 2 3 4 5 G1

Reaktionsbedingungen: a) XPhos Pd G3, $K_3PO_4$, RF, THF, $H_2O$, RF, 24 h, 62 %; b) NBS, DCM, RT, 2 h, 95 %; c) $Pd(PPh_3)_2Cl_2$, Trimethylsilylacetylen, TEA, RF, 24 h, 66 %; d) $K_2CO_3$, MeOH, RT, 0.5 h, 93 %; e) Au(SPhos)($NTf_2$), meta-Xylol, RF, 20 h, 48 %; f) $Pd(PCy_3)_2Cl_2$, $K_2CO_3$, DMF, RF, 50 h, 70 %.

**Synthese von Synthonen S:**

**Beispiel S1:**

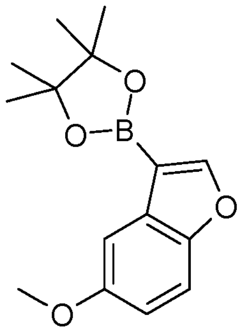

Ein gut gerührtes Gemisch aus 22.7 g (100 mmol) 3-Brom-5-methoxy-benzofuran [333385-25-4], 27.9 g (110 mmol) Bis(pinakolato)diboran [73183-34-3], 29.5 g (300 mmol) Kaliumacetat, wasserfrei, 50 g Glaskugeln (3 mm Durchmesser) und 500 ml THF wird mit 841 mg (3 mmol) Tricyclohexylphosphin und dann mit 224 mg (1 mmol) Palladium(II)acetat versetzt und 16 h unter Rückfluss erhitzt. Man saugt noch warm über ein mit THF vorgeschlämmtes Celite-Bett ab, entfernt das Lösungsmittel im Vakuum, nimmt den Rückstand in 100 ml heißem Methanol auf. Man lässt unter Rühren auf Raumtemperatur erkalten, saugt vom ausgefallenen Produkt ab, wäscht dieses einmal mit 30 ml Methanol und trocknet im Vakuum. Ausbeute: 19.3 g (70 mmol) 70 %; Reinheit: ca. 95 % ig n.$^1$H-NMR.

Analog können folgende Verbindungen dargestellt werden:

| Bsp. | Edukt | Produkt | Ausbeute |
|---|---|---|---|
| S2 | 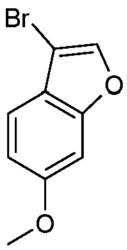 1822631-86-6 | 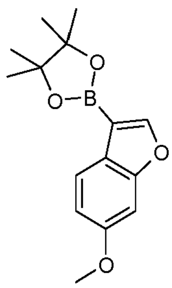 | 74 % |
| S3 | 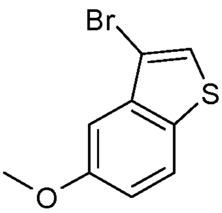 487021-62-5 | 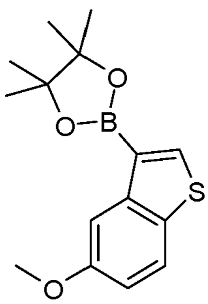 | 66 % |

(fortgesetzt)

| Bsp. | Edukt | Produkt | Ausbeute |
|---|---|---|---|
| G40 | 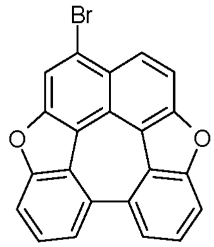 G30 | 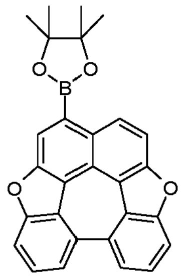 | 72 % |
| G41 | 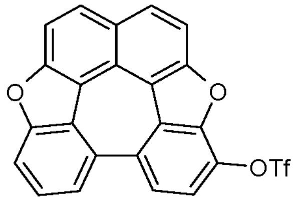 G22 | 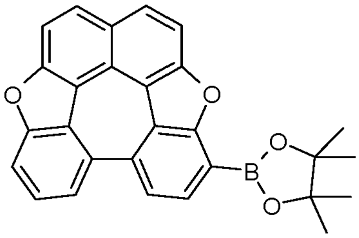 | 69 % |

**Beispiel G1: Synthese von Grundkörper G1, s. Reaktionsschema**

**Stufe a): Verbindung 1**

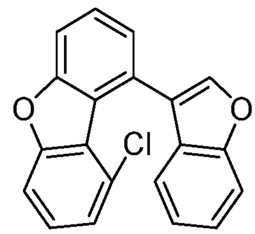

Ein Gemisch aus 28.1 g (100 mmol) 1-Brom-9-chlordibenzofuran [2179279-83-3], 16.3 g (100 mmol) 3-Benzofuranyl-boronsäure [317830-83-4], 53.1 g (250 mmol) Trikaliumphosphat, 3.4 g (4 mmol) **XPhos Pd G3 [1445058-55-1],** 800 ml THF und 200 ml Wasser wird 24 h unter Rückfluss erhitzt. Dann wird das Reaktionsgemisch im Vakuum eingeengt und der Rückstand wird in 800 ml Ethylacetat aufgenommen. Die organische Phase wird dreimal mit je 200 ml Wasser, einmal mit 300 ml ges. Kochsalzlösung gewaschen und dann im Vakuum zur Trockene eingeengt.

Der schwarze ölige Rückstand wird in 500 ml n-Heptan aufgenommen und über Kieselgel fraktioniert filtriert. Das Produkt wird nach Entfernen des n-Heptans im Vakuum als farbloses Öl erhalten. Ausbeute: 19.8 g (62 mmol) 62 %; Reinheit: ca. 95 % ig n. $^{1}$H-NMR.

**Stufe b): Verbindung 2**

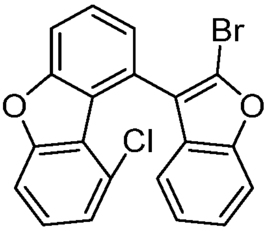

Eine gut gerührte Lösung von 33.9 g (100 mmol) Verbindung 1 in 300 ml Dichlormethan (DCM) wird bei Raumtemperatur mit 18.0 g (100 mmol) NBS versetzt und 2 h gerührt. Anschließend wird das Lösungsmittel im Vakuum entfernt, der ölige Rückstand wird in 500 ml n-Heptan aufgenommen und über Kieselgel filtriert. Das Produkt wird nach Entfernen des n-Heptans im Vakuum als farbloses Öl erhalten: Ausbeute: 37.8 g (95 mmol) 95 %; Reinheit: ca. 95 % ig n. $^1$H-NMR.

**Stufe c): Verbindung 3**

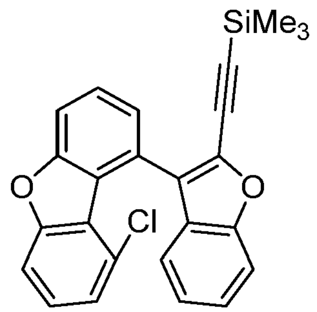

Ein gut entgastes Gemisch aus 39.7 g (100 mmol) Verbindung 2, 42.4 ml (300 mmol) Trimethylsilylacetylen, 1000 ml DMF und 500 ml Triethylamin wird mit 1.2 g (6 mmol) Kupferiodid und dann mit 4.0 g (4 mmol) Tetrakistriphenylphosphino-palladium(0) versetzt und 24 h bei 80°C gerührt. Nach Erkalten wird die Reaktionsmischung über ein mit DMF aufgeschlämmtes Celite-Bett filtriert. Das Filtrat wird im Vakuum bei 30 °C vom Lösungsmittel bereit, der Rückstand wird mit 500 ml DCM auf ISOLUTE® aufgezogen und mit Hilfe eines Säulenautomaten (Torrent der Fa. A. Semrau) mit n-Heptan/Ethylacetat chromatographiert. Ausbeute: 25.3 g, (66 mmol) 66 %, Reinheit: ca. 95 % ig n. $^1$H-NMR.

**Stufe d): Verbindung 4**

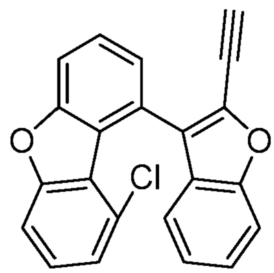

Eine gut gerührte Lösung von 41.7 g (100 mmol) Verbindung 3 in 500 ml MeOH wird bei Raumtemperatur mit Kaliumcarbonat 27.6 g (200 mmol) versetzt und 30 min. gerührt. Man filtriert vom Kaliumcarbonat ab, entfernt das MeOH bei 30 °C im Vakuum, nimmt den Rückstand in 300 ml DCM auf, wäscht dieses dreimal mit je 100 ml Wasser, einmal mit 100 ml ges. Kochsalzlösung, trocknet über Natriumsulfat, filtriert vom Trockenmittel ab und engt das Filtrat im Vakuum zur Trockene ein. Ausbeute: 32.1 g (93 mmol), 93 %; Reinheit: ca. 95 % ig n. $^1$H-NMR.

**Stufe e): Verbindung 5**

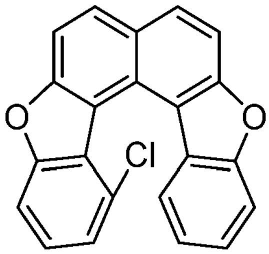

Eine gut gerührte Lösung von 17.2 g (50 mmol) Verbindung 4 in 1000 ml m-Xylol wird unter Rückfluss erhitzt, dann tropfenweise während 8 h mit einer Lösung von 4.4 g (5 mmol) $SPhosAuNTf_2$ [1121960-90-4] in 200 ml m-Xylol versetzt und abschließend weitere 12 h unter Rückfluss erhitzt. Nach Erkalten wird das Lösungsmittel im Vakuum entfernt. Der ölige Rückstand wird in 300 ml DCM aufgenommen und auf ISOLUTE® aufgezogen und mit Hilfe eines Säulenautomaten (Torrent der Fa, A, Semrau) mit n-Heptan chromatographiert. Ausbeute: 8.3 g (48 mmol), 48 %; Reinheit: ca. 95 % ig n. $^1$H-NMR.

**Stufe f): Grundkörper G1**

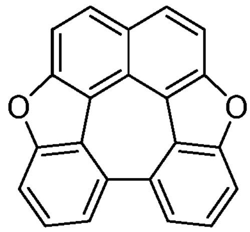

Ein gut gerührtes Gemisch aus 17.2 g (50 mmol) Verbindung 5, 27.6 g (200 mmol) Kaliumcarbonat, 1.9 g (2.5 mmol) $Pd(PCy_3)_2Cl_2$[29934-17-6], 50 g Glaskugeln und 1000 ml DMF wird 50 h unter schwachem Rückfluss erhitzt. Nach Erkalten entfernt man das DMF im Vakuum, nimmt den Rückstand in 1000 ml heißem Chlorbenzol auf, filtriert über ein vorgeschlämmtes Alox-basisch-Bett ab, engt das Filtrat im Vakuum zur Trockene ein und rührt den beigen Rückstand zweimal mit 300 ml heißem n-Heptan aus. Ausbeute: 10.9 g (35 mmol), 70 %; Reinheit: ca. 95 % ig n. $^1$H-NMR.

Analog können folgende Verbindungen dargestellt werden:

| Bsp. | Edukte | Produkt | Ausbeute über 6 Stufen |
|---|---|---|---|
| G2 | 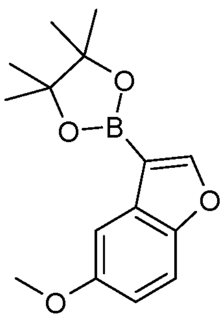 S1 | 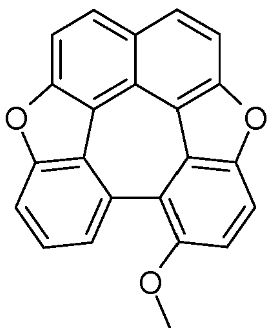 | 10 % |

(fortgesetzt)

| Bsp. | Edukte | Produkt | Ausbeute über 6 Stufen |
|---|---|---|---|
| G3 | 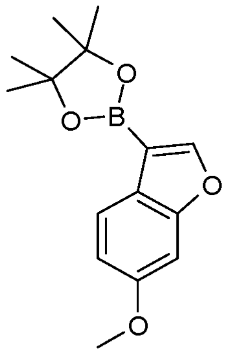 S2 | 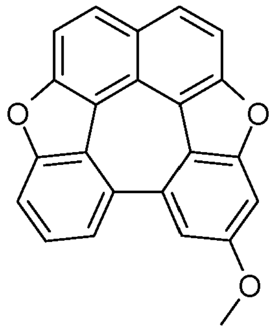 | 15 % |
| G4 | 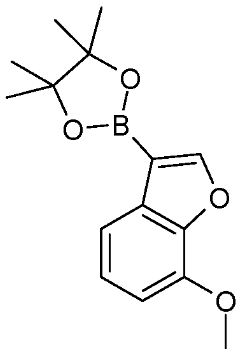 675876-98-9 | 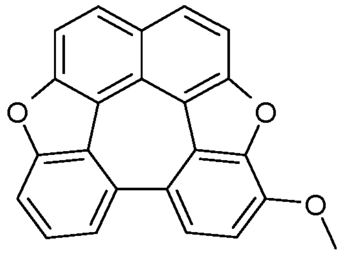 | 21 % |
| G5 | 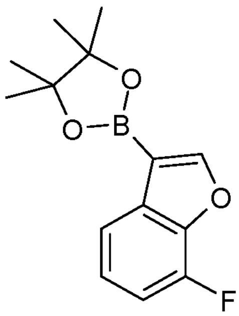 1569089-52-6 | 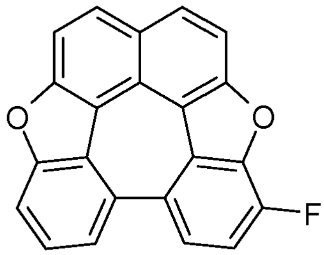 | 17 % |
| G6 | 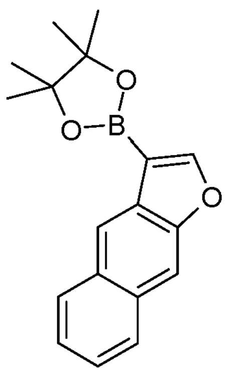 1869141-62-7 | 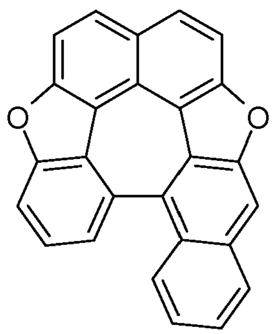 | 15 % |

(fortgesetzt)

| Bsp. | Edukte | Produkt | Ausbeute über 6 Stufen |
|---|---|---|---|
| G7 | 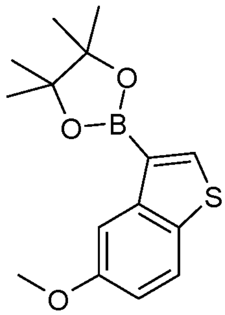 S3 | 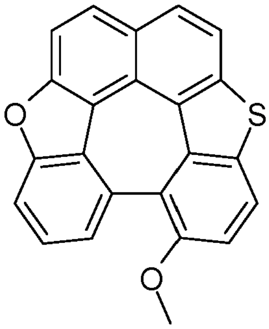 | 13 % |
| TMM30 | 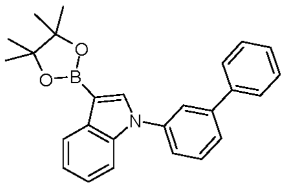 1869140-80-6 | 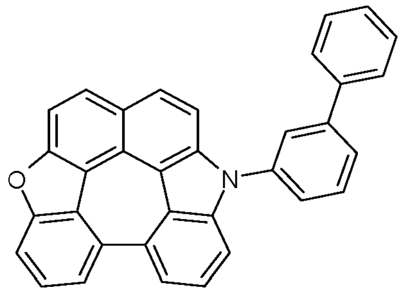 | 15 % |
| TMM31 | 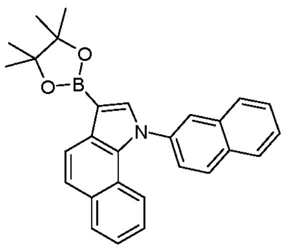 1869140-91-9 | 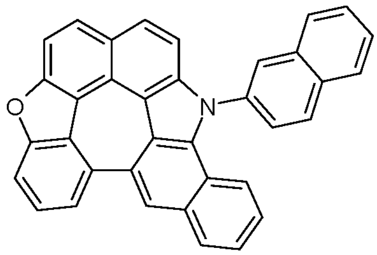 | 14 % |

**Beispiel G10:**

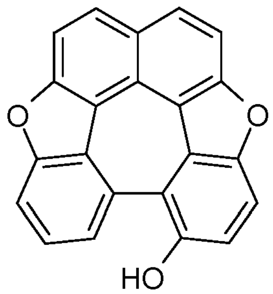

Ein Gemisch aus 16.8 g (50 mmol) G2 und 115.6 g (1 mol) Pyridiniumhydrochlorid [628-13-7] wird aufgeschmolzen und unter gutem Rühren am Wasserabscheider für 3 h auf 230 °C (Heizschalentemperatur) erhitzt, wobei das gebildete Pyridin von Zeit zu Zeit abgelassen wird. Man lässt die Reaktionsmischung auf ca. 100 °C abkühlen und tropft vorsichtig 500 ml Wasser zu. Nach Erkalten saugt man vom ausgefallenen Feststoff ab, wäscht diesen dreimal mit 100 ml warmem Wasser und einmal mit 50 ml kaltem Methanol und trocknet im Vakuum. Ausbeute: 16.1 g (48 mmol), 96 %; Reinheit: ca. 95 % ig n. $^1$H-NMR.

Analog können folgende Verbindungen dargestellt werden:

| Bsp. | Edukt | Produkt | Ausbeute |
|---|---|---|---|
| G11 | 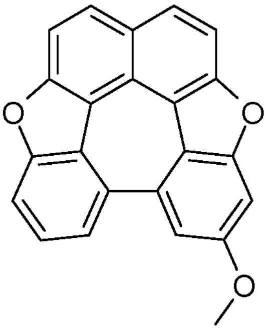 G3 | 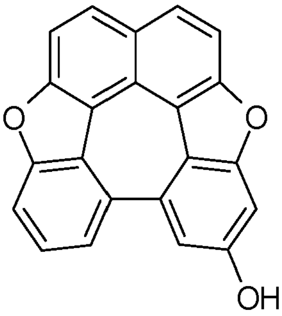 | 95 % |
| G12 | 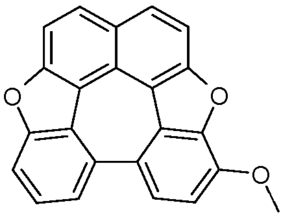 G4 | 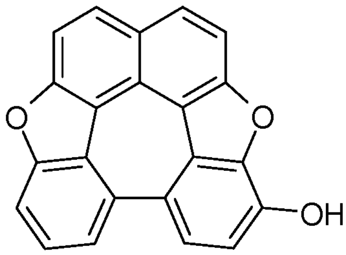 | 93 % |
| G13 | 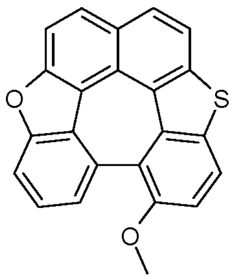 G7 | 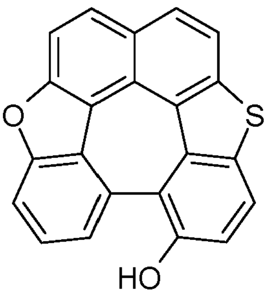 | 91 % |

**Beispiel G20:**

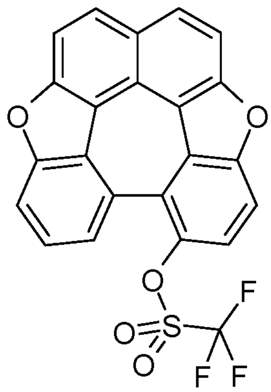

Eine gut gerührte auf 0 °C gekühlte Suspension von 16.1 g (50 mmol) G10 in 300 ml DCM wird 12 ml Pyridin und dann tropfenweise mit 16.8 ml (100 mmol) Trifluormethansulfonsäureanhydrid [358-23-6] versetzt und 2 h bei 0 °C gerührt. Man lässt die Reaktionsmischung auf Raumtemperatur erwärmen, rührt 2 h nach, gießt dann auf 500 g Eis und rührt 30 min. nach. Man trennt die org. Phase ab, wäscht diese zweimal mit 100 ml ges. Kochsalzlösung und entfernt das Lösungsmittel im Vakuum. Ausbeute: 21.7 g (47.5 mmol), 95 %; Reinheit: ca. 95 % ig n. $^{1}$H-NMR.

Analog können folgende Verbindungen dargestellt werden:

| Bsp. | Edukt | Produkt | Ausbeute |
|---|---|---|---|
| G21 | 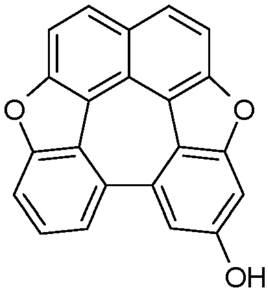 G11 | 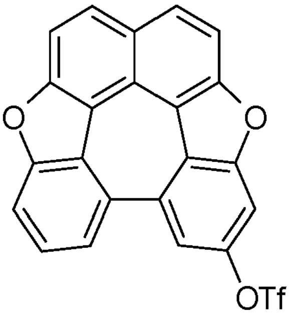 | 93 % |
| G22 | 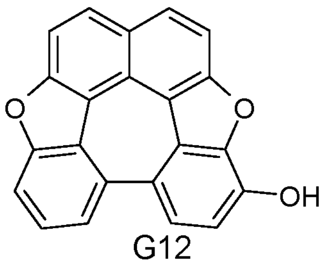 G12 | 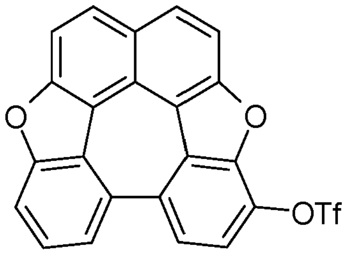 | 95 % |
| G23 | 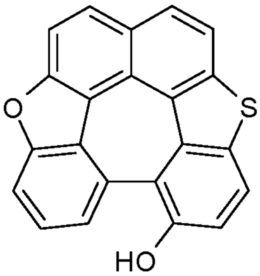 G13 | 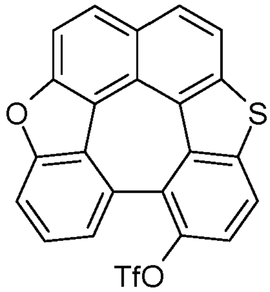 | 90 % |

**Beispiel G30:**

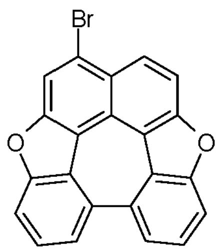

Eine Suspension von 3.06 g (10.0 mmol) G1 in 100 ml Chloroform wird unter Lichtausschluß mit 3.53 g (11 mmol) Pyridiniumperbromid versetzt und dann 24 h bei 60 °C gerührt. Nach Erkalten versetzt man mit 30 ml 10 Gew.-%iger Natriumsulftitlösung, rührt 30 min. nach, trennt die org. Phase ab, engt im Vakuum zur Trockene ein, rührt den Rückstand zweimal mit 25 ml heißem Methanol aus und kristallisiert aus wenig Chlorbenzol um. Ausbeute: 3.05 g (7.8 mmol), 78 %; Reinheit: ca. 95 % ig n. $^{1}$H-NMR.

**Beispiel TMM1:**

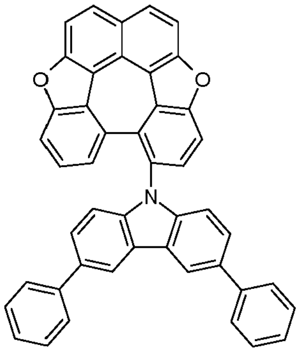

Ein Gemisch aus 4.54 g (10 mmol) G20, 3.83 g (12 mmol) 3,6-Diphenylcarbazol [56525-79-2], 5.31 g (25 mmol) Trikaliumphosphat, 20 g Glaskugeln (3 mm Durchmesser) und 100 ml o-Xylol wird unter Rühren bei Raumtemperatur mit 289 mg (0.5 mmol) XantPhos und mit 112 mg (0.5 mmol) Palladium(II)acetat versetzt und dann 36 h unter Rückfluss gerührt. Man entfernt das Lösungsmittel weitgehend im Vakuum, rührt der Feststoff zweimal mit je 200 ml heißem Wasser aus, saugt vom ausgefallenen Produkt ab, wäscht dieses dreimal mit je 100 ml Wasser, dreimal mit 30 ml Methanol und trocknet im Vakuum. Das Rohprodukt wird in 300 ml DCM gelöst und über ein mit DCM vorgeschlämmtes Kielegelbett filtriert. Das Filtrat wird mit 100 ml Methanol versetzt, das DCM wird im Vakuum entfernt, der ausgefallene Feststoff wird abgesaugt und im Vakuum getrocknet. Die Reinigung erfolgt durch wiederholte Heißextraktion mit Toluol, Vorlagemenge 150 ml, Cellulose-Extraktionshülsen der Fa. Whatman. Alternativ können andere Lösemittel verwendet werden. Abschließend wird das Produkt im Hochvakuum ($p \sim 10^{-5}$ mbar) fraktioniert sublimiert. Ausbeute: 3.93 g (6.3 mmol), 63 %; Reinheit: > 99.9 % ig n. HPLC.

Analog können folgende Verbindungen dargestellt werden:

| **Bsp.** | **Edukte** | **Produkt** | **Ausbeute** |
|---|---|---|---|
| TMM2 | G21 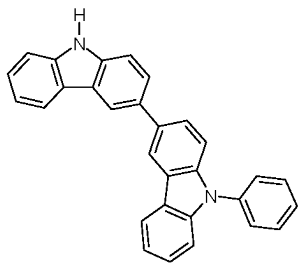 1060735-14-9 | 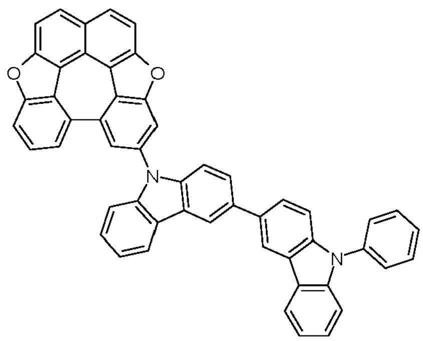 | 72 % |
| TMM3 | G22 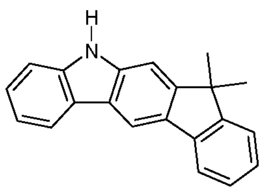 1257220-47-5 | 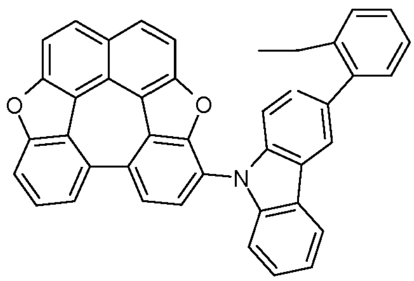 | 68 % |

(fortgesetzt)

| Bsp. | Edukte | Produkt | Ausbeute |
|---|---|---|---|
| TMM4 | G23 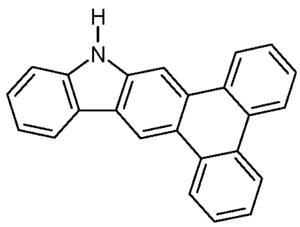 1374446-05-5 | 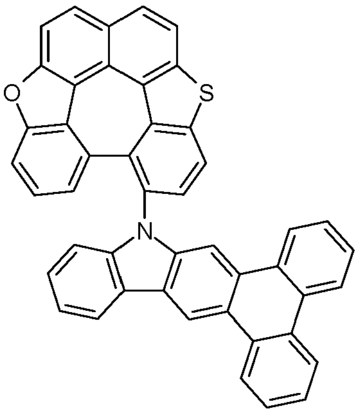 | 44 % |
| TMM5 | G30 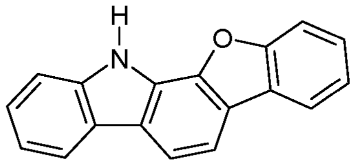 1338919-70-2 | 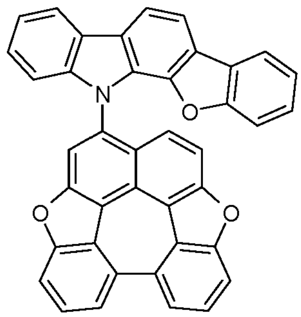 | 61 % |

**Beispiel TMM10:**

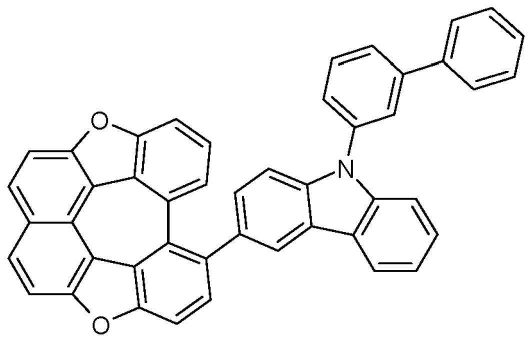

Ein Gemisch aus 4.54 g (10 mmol) G20, 5.34 g (12 mmol) 9-[1,1'-Biphenyl]-3-yl-3-(4,4,5,5-tetramethyl-1,3,2-dioxaborolan-2-yl)-9H-carbazol [1533406-38-0], 6.9 g (30 mmol) Trikaliumphosphat-Monohydrat [27176-10-9], 20 g Glaskugeln (3 mm Durchmesser), 347 mg (0.3 mmol) Tetrakis-triphenylphosphino-palladium(0) und 100 ml DMSO wird 24 h bei 100 °C gerührt. Man gießt die warme Reaktionsmischung in 300 ml heißes Wasser ein, rührt 20 min. nach, saugt vom ausgefallenen Produkt ab, wäscht dieses dreimal mit je 50 ml Wasser, dreimal mit je 30 ml Methanol und trocknet im Vakuum. Das Rohprodukt wird in 300 ml DCM gelöst und über ein mit DCM vorgeschlämmtes Kielegelbett filtriert. Das Filtrat wird mit 100 ml Methanol versetzt, das DCM wird im Vakuum entfernt, der ausgefallene Feststoff wird abgesaugt und im Vakuum getrocknet. Die Reinigung erfolgt durch wiederholte Heißextraktion mit Toluol, Vorlagemenge 150 ml, Cellulose-Extraktionshülsen der Fa. Whatman. Alternativ können andere Lösemittel verwendet werden. Abschließend wird das Produkt im Hochvakuum (p ~ $10^{-5}$ mbar) fraktioniert sublimiert. Ausbeute: 2.8 g (4.5 mmol), 45 %; Reinheit: > 99.9 % ig n. HPLC.

Analog können folgende Verbindungen dargestellt werden:

| Bsp. | Edukte | Produkt | Ausbeute |
|---|---|---|---|
| TMM 11 | G21<br>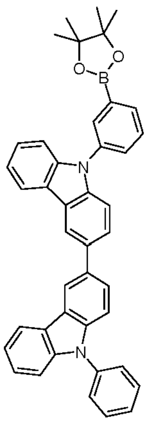<br>2088364-11-6 | 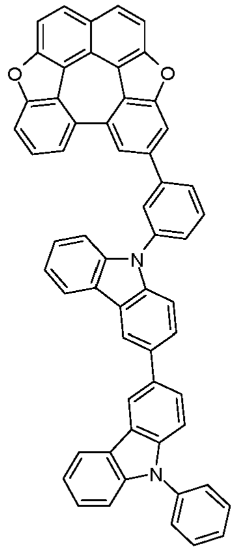 | 57 % |
| TMM12<br>ETM1 | G22<br>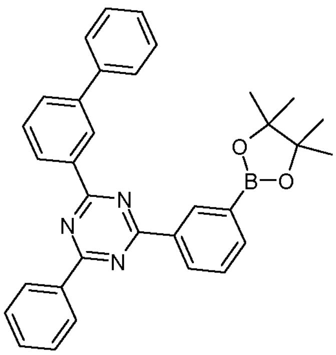<br>2032365-30-1 | 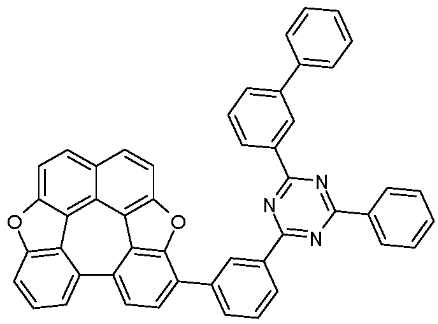 | 63 % |
| TMM13 | G23<br>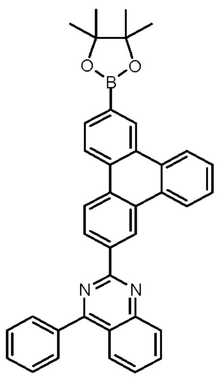<br>1852465-33-8 | 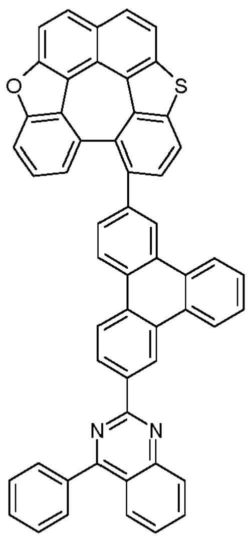 | 38 % |

(fortgesetzt)

| Bsp. | Edukte | Produkt | Ausbeute |
|---|---|---|---|
| TMM14 | G30 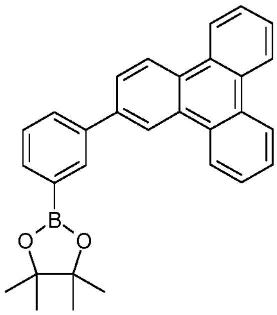 1115639-92-3 | 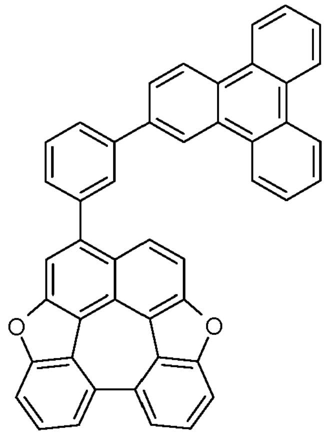 | 71 % |

**TMM20:**

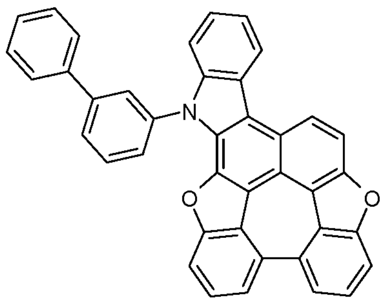

**Stufe a)**

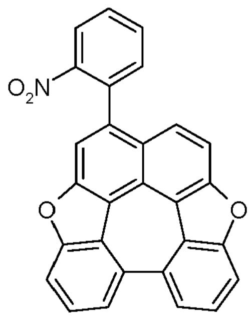

Ein gut gerührtes Gemisch aus 4.32 g (10 mmol) G30, 2.22 g (11 mmol) 2-Brom-nitrobenzol [577-19-5], 4.15 g (30 mmol) Kaliumcarbonat, 116 mg (0.1 mmol) Tetrakis-triphenylphosphino-palladium(0), 150 ml THF und 30 ml Wasser wird 16 h unter Rückfluss erhitzt. Man versetzt die noch warme Reaktionsmischung mit 200 ml Wasser, engt die Reaktionsmischung im Vakuum auf ca. 100 ml ein, saugt vom ausgefallenen Feststoff ab, wäscht diesen dreimal mit je 50 ml Wasser, zweimal mit je 30 ml Methanol und Trocknet im Vakuum. Ausbeute: 3.99 g (9.3 mmol), 93 %; Reinheit: ca. 95 % ig n. $^{1}$H-NMR.

**Stufe b):**

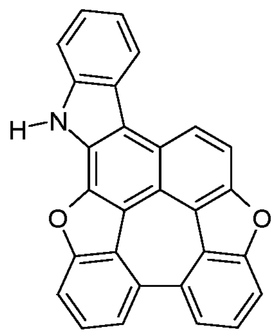

Analog S. H. Smitrovich et al., Org. Lett, 2004, 6, 4, 533. Ein Gemisch aus 4.27 g (10 mmol) TMM20 Stufe a), 72 mg (0.4 mmol) 1,10-Phenanthrolin, 45 mg (0.2 mmol) Palladium(II)acetat und 50 ml DMF wird in einem Autoklaven unter 5 bar CO-Duck 16 h bei 140 °C gerührt. Nach Erkalten entfernt man das DMF im Vakuum, nimmt den Rückstand in 100 ml siedendem Chlorbenzol auf und filtriert noch heiß über ein mit Chlorbenzol vorgeschlämmtes Kieselgelbett ab, engt dann das Filtrat bei 80 °C bis zur beginnenden Kristallisation im Vakuum ein und rührt bei Raumtemperatur zur Vervollständigung der Kristallisation nach. Man saugt das Produkt ab, wäscht dieses dreimal mit 30 ml Methanol und trocknet im Vakuum. Ausbeute: 3.44 g (8.7 mmol), 87 %; Reinheit: ca. 95 % ig n. $^{1}$H-NMR.

**Stufe c): TMM20**

Ein Gemisch aus 3.95 g (10 mmol) TMM20 Stufe b), 3.27 g (14 mmol) 3-Brombiphenyl [2113-57-7], 1.44 g (15 mmol) Natrium-tert-butanolat, 41.0 mg (0.1 mmol) S-Phos, 22.5 mg (0.1 mmol) Palladium(II)acetat und 100 ml o-Xylol wird 24 h unter Rückfluss gerührt. Nach Erkalten entfernt man das o-Xylol im Vakuum und rührt den Rückstand mit 100 ml eines Gemischs aus Wasser/Methanol 1:2 heiß aus, saugt vom Feststoff ab, wäscht dreimal mit je 30 ml Methanol nach und trocknet diesen im Vakuum. Man nimmt den Rückstand in 100 ml siedendem Chlorbenzol auf und filtriert noch heiß über ein mit Chlorbenzol vorgeschlämmtes Kieselgelbett ab, engt dann das Filtrat bei 80 °C bis zur beginnenden Kristallisation im Vakuum ein und rührt bei Raumtemperatur zur Vervollständigung der Kristallisation nach. Man saugt das Produkt ab, wäscht dieses dreimal mit 30 ml Methanol und trocknet im Vakuum. Die Reinigung erfolgt durch wiederholte Heißextraktion mit Toluol, Vorlagemenge 150 ml, Cellulose-Extraktionshülsen der Fa. Whatman. Alternativ können andere Lösemittel verwendet werden. Abschließend wird das Produkt im Hochvakuum (p ~ $10^{-5}$ mbar) fraktioniert sublimiert. Ausbeute: 3.84 g (7.0 mmol), 70 %; Reinheit: > 99.9 % ig n. HPLC.

Analog können folgende Verbindungen dargestellt werden:

| Bsp. | Edukte | Produkt | Ausbeute |
|---|---|---|---|
| TMM21 | 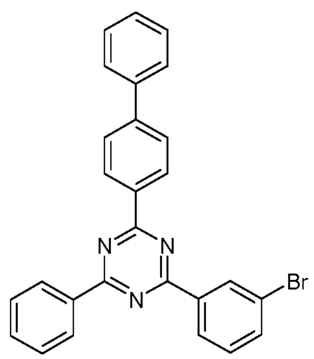 1955546-91-4 | 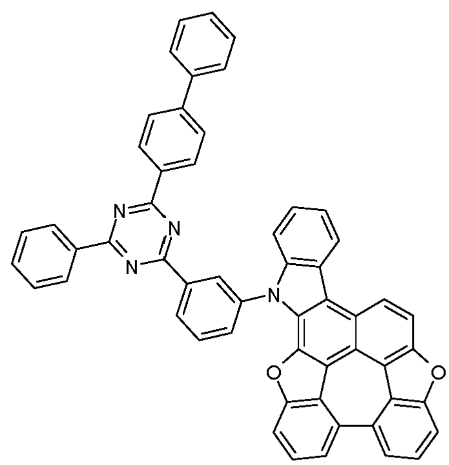 | 48 % |

(fortgesetzt)

| Bsp. | Edukte | Produkt | Ausbeute |
|---|---|---|---|
| TMM22 | 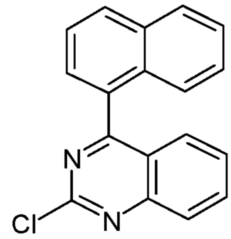<br>1413367-86-9 | 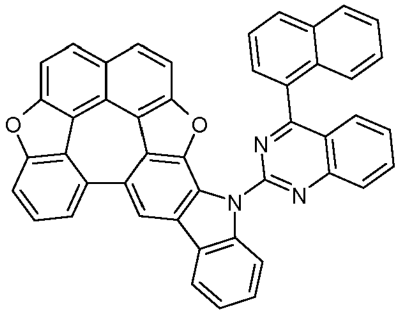 | 45 % |

**Beispiel SMM1:**

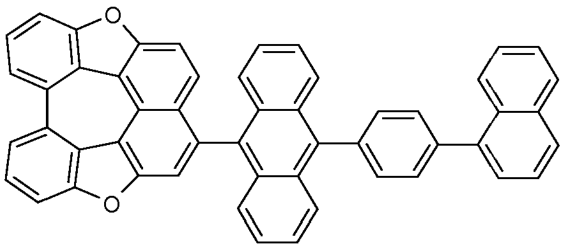

Ein gut gerührtes Gemisch aus 4.32 g (10 mmol) G30, 5.05 g (11 mmol) 10-(4-(1-Naphthenyl)phenyl)-9-bromoanthracen [1092390-01-6], 6.37 g (30 mmol) Trikaliumphosphat, 183 mg (0.6 mmol) Tri-o-tolylphosphin, 22.5 mg (0.1 mmol) Palladium(II)acetat, 100 ml Toluol, 50 ml Dioxan und 100 ml Wasser wird 16 h unter Rückfluss erhitzt. Nach Erkalten saugt man vom ausgefallenen Feststoff ab, wäscht diesen dreimal mit je 50 ml Wasser, dreimal mit 30 ml Methanol und trocknet im Vakuum. Man nimmt den Feststoff in 200 ml DCM auf, filtriert über ein mit DCM vorgeschlämmtes Kieselgelbett, versetzt das Filtrat mit 100 ml Methanol und engt im Vakuum bis zur beginnenden Kristallisation ein. Man saugt vom Kristallisat ab, wäscht dieses zweimal mit 30 ml Methanol und trocknet im Vakuum. Die Reinigung erfolgt durch wiederholte Heißextraktion mit Toluol, Vorlagemenge 150 ml, Cellulose-Extraktionshülsen der Fa. Whatman. Alternativ können andere Lösemittel verwendet werden. Abschließend wird das Produkt im Hochvakuum (p ~ $10^{-5}$ mbar) fraktioniert sublimiert. Ausbeute: 4.60 g (6.7 mmol), 67 %; Reinheit: > 99.9 % ig n. HPLC.

Analog kannen folgende Verbindung dargestellt werden:

| Bsp. | Edukte | Produkt | Ausbeute |
|---|---|---|---|
| SMM2 | G41<br>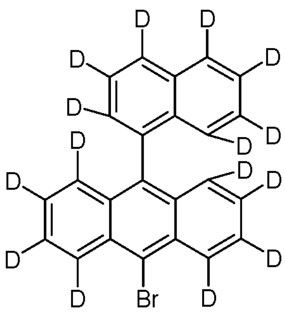 | 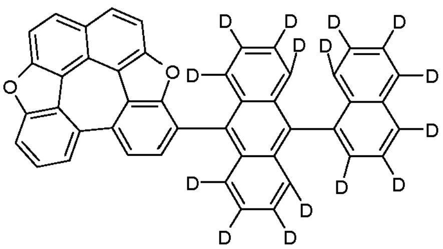 | 60 % |

**Vakuum-prozessierte Devices:**

Die Herstellung von erfindungsgemäßen OLEDs sowie OLEDs nach dem Stand der Technik erfolgt nach einem

allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert) und anschließend bei 180°C 10 min. lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochinjektionsschicht 1 (HIL1) bestehend aus Ref-HTM1 dotiert mit 5 % NDP-9 (kommerziell erhältlich von der Fa. Novaled), 20 nm / Lochstransportschicht 1 (HTL1) aus 200 nm Ref-HTM1 / Lochtransportschicht 2 (HTL2) aus 10 nm Ref-HTM2 / Emissionsschicht (EML) 30 nm / Lochblockierschicht (HBL) 10 nm / Elektronentransportschicht (ETL) 30 nm / optionale Elektroneninjektionsschicht (EIL) und abschließend eine Kathode. Die Kathode wird durch eine 100 nm dicke Aluminiumschicht gebildet.

Zunächst werden vakuum-prozessierte OLEDs beschrieben. Hierfür werden alle Materialien in einer Vakuumkammer thermisch aufgedampft. Dabei besteht die Emissionsschicht immer aus mindestens einem Matrix-material (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie Ref-TMM1:Ref-TMM2:Ir1 (55%:35%:10%) bedeutet hierbei, dass das Material Ref-TMM1 in einem Volumenanteil von 55%, Ref-TMM2 in einem Anteil von 35% und IrL1 in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 3 gezeigt.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m$^2$ bestimmt und daraus die CIE 1931 x und y Farbkoordinaten berechnet. Als Lebensdauer LD90 wird die Zeit definiert, nach der die Leuchtdichte bei Betrieb mit einer Starthelligkeit von 10000 cd/m$^2$ auf 90% der Startleuchtdichte abgesunken ist.

Die OLEDs können initial auch bei anderen Startleuchtdichten betrieben werden. Die Werte für die Lebensdauer können dann mit Hilfe dem Fachmann bekannten Umrechnungsformeln auf eine Angabe für andere Startleuchtdichten umgerechnet werden.

**Verwendung von erfindungsgemäßen Verbindungen als Materialien in phosphoreszierenden OLEDs**

Die erfindungsgemäßen Verbindungen lassen sich unter anderem als TMM (Triplet Matrix Material), ETM (Electron Transport Material) sowie als Hostmaterialien SMM (Singulet Matrix Material) in der Emissionsschicht in OLEDs einsetzen. Als Vergleich gemäß dem Stand der Technik werden Verbindungen gemäß Tabelle 3 verwendet. Die Ergebnisse der OLEDs sind in Tabelle 2 zusammengefasst.

**Tabelle 1: Aufbau der OLEDs**

| Bsp. | EML | HBL | ETL |
|---|---|---|---|
| Ref.D1 | Ref-TMM1:Ref-TMM2:Ir1 (45%:40%:15%) | Ref-ETM1 | Ref-ETM1:Ref-ETM2 (50%:50%) |
| Ref.D2 | Ref-TMM1:Ref-TMM2:Ir2 (45%:40%:15%) | Ref-ETM1 | Ref-ETM1:Ref-ETM2 (50%:50%) |
| Ref.D3 | Ref-TMM1:Ref-TMM2:Ir3 (45%:50%:5%) | Ref-ETM1 | Ref-ETM1:Ref-ETM2 (50%:50%) |
| D1 | Ref-TMM1:TMM1:Ir1 (45%:40%:15%) | Ref-ETM1 | Ref-ETM 1: Ref-ETM2 (50%:50%) |
| D2 | TMM12:TMM2:Ir1 (45%:40%:15%) | Ref-ETM1 | Ref-ETM1:Ref-ETM2 (50%:50%) |
| D3 | TMM12:TMM2:Ir2 (40%:50%:10%) | Ref-ETM1 | Ref-ETM1:Ref-ETM2 (50%:50%) |
| D4 | TMM12:TMM3:Ir2 (40%:50%:10%) | Ref-ETM1 | Ref-ETM1:Ref-ETM2 (50%:50%) |
| D5 | Ref-TMM1:TMM4:Ir2 (30%:60%:10%) | Ref-ETM1 | Ref-ETM1:Ref-ETM2 (50%:50%) |
| D6 | Ref-TMM1:TMM5:Ir2 (30%:55%:15%) | Ref-ETM1 | Ref-ETM1:Ref-ETM2 (50%:50%) |
| D7 | Ref-TMM1:TMM10:Ir1 (30%:55%:15%) | Ref-ETM1 | Ref-ETM1:Ref-ETM2 (50%:50%) |
| D8 | TMM12:TMM11:Ir1 (30%:55%:15%) | Ref-ETM1 | Ref-ETM1:Ref-ETM2 (50%:50%) |

(fortgesetzt)

| Bsp. | EML | HBL | ETL |
|---|---|---|---|
| D9 | TMM13:Ir3 (95%:5%) | --- | Ref-ETM1:Ref-ETM2 (50%:50%) |
| D10 | TMM12:TMM13: Ir1 (40%:40%:20%) | Ref-ETM1 | Ref-ETM1:Ref-ETM2 (50%:50%) |
| D11 | TMM21:TMM20:Ir1 (50%:40%:10%) | Ref-ETM1 | Ref-ETM1:Ref-ETM2 (50%:50%) |
| D12 | TMM22:Ir3 (95%:5%) | --- | Ref-ETM1:Ref-ETM2 (50%:50%) |
| D13 | TMM22:Ir3 (95%:5%) | ETM1 | Ref-ETM 1: Ref-ETM2 (50%:50%) |
| D14 | TMM22: Ir3 (95%:5%) | ETM1 | ETM1:Ref-ETM2 (50%:50%) |
| D15 | TMM21:TMM30: Ir3 (50%:45%:5%) | --- | Ref-ETM1:Ref-ETM2 (50%:50%) |
| D16 | TMM21:TMM31:Ir3 (50%:45%:5%) | --- | Ref-ETM1:Ref-ETM2 (50%:50%) |
| D17 | SMM1:GD1 (94%:6%) | --- | Ref-ETM1:Ref-ETM2 (50%:50%) |
| D18 | SMM2:GD1 (94%:6%) | --- | Ref-ETM1:Ref-ETM2 (50%:50%) |

**Tabelle 2: Ergebnisse der Vakuum-prozessierten OLEDs**

| Bsp. | EQE (%) 1000 cd/m$^2$ | Spannung (V) 1000 cd/m$^2$ | CIE x/y 1000 cd/m$^2$ | LD90 (h) 10000 cd/m$^2$ |
|---|---|---|---|---|
| **Gelbe und Rote OLEDs** | | | | |
| Ref.D1 | 27.4 | 3.2 | 0.49/0.51 | 1700 |
| Ref.D2 | 27.0 | 3.1 | 0.51/0.48 | 1300 |
| Ref.D3 | 25.1 | 3.4 | 0.67/0.33 | 300 |
| D1 | 27.3 | 2.9 | 0.49/0.51 | 1800 |
| D2 | 27.6 | 2.9 | 0.49/0.51 | 1700 |
| D3 | 27.4 | 2.8 | 0.51/0.48 | 1100 |
| D4 | 27.8 | 2.8 | 0.51/0.48 | --- |
| D5 | 27.6 | 2.8 | 0.51/0.48 | --- |
| D6 | 27.6 | 2.9 | 0.51/0.49 | --- |
| D7 | 27.9 | 3.0 | 0.49/0.51 | --- |
| D8 | 27.1 | 2.9 | 0.49/0.51 | --- |
| D9 | 25.5 | 3.2 | 0.67/0.33 | 700 |
| D10 | 27.4 | 3.0 | 0.49/0.51 | --- |
| D11 | 28.1 | 3.0 | 0.49/0.51 | --- |
| D12 | 25.9 | 3.1 | 0.67/0.33 | --- |
| D13 | 26.2 | 3.0 | 0.67/0.33 | --- |
| D14 | 26.3 | 3.0 | 0.67/0.33 | --- |
| D15 | 26.1 | 3.0 | 0.67/0.33 | --- |
| D16 | 25.9 | 2.9 | 0.68/0.32 | 600 |
| **Grüne OLEDs** | | | | |
| **Bsp.** | **EQE (%) 1000 cd/m$^2$** | **Spannung (V) 1000 cd/m$^2$** | **CIE x/y 1000 cd/m$^2$** | |
| D17 | 8.6 | 3.7 | 0.29/0.62 | |
| D18 | 8.8 | 3.5 | 0.29/0.62 | |

**Tabelle 3:**

| **Strukturformeln der verwendeten Materialien** | |
|---|---|
| 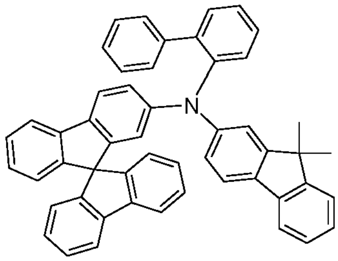 HTM1 [136463-07-5] | 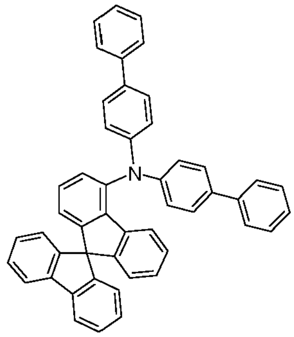 HTM2 [1450933-43-3] |
| 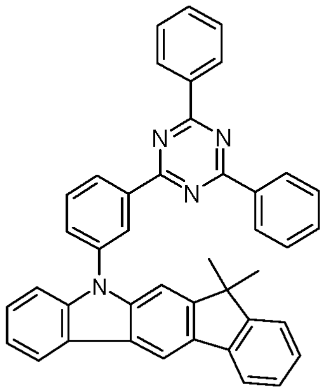 Ref-TMM1 [1257248-13-7] | 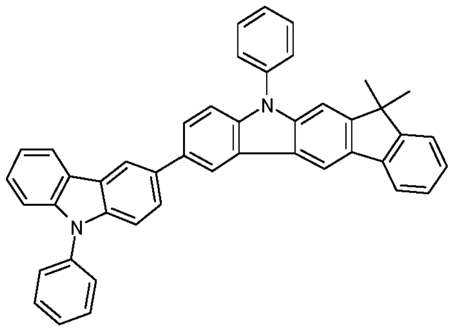 Ref-TMM2 [1357150-54-9] |
| 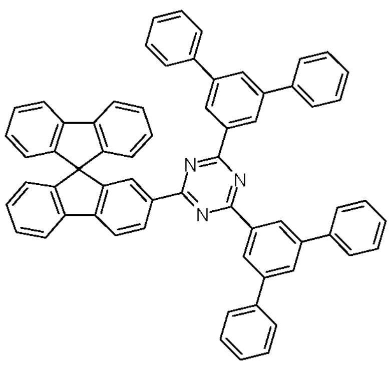 Ref-ETM1 [1233200-52-6] | 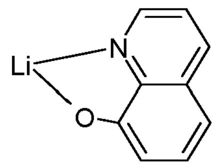 Ref-ETM2 [25387-93-3] |
| 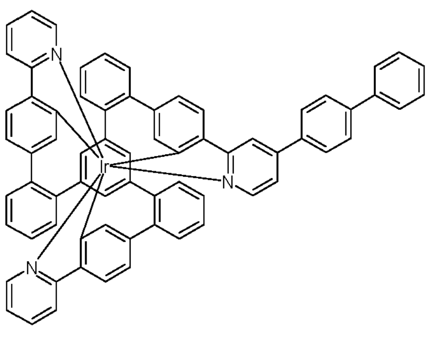 Ir1 [2245945-28-0] | 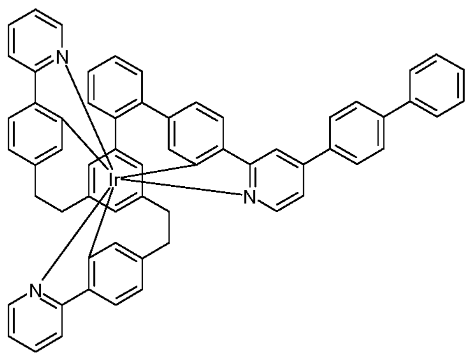 Ir2 [2245945-28-0] |

(fortgesetzt)

| **Strukturformeln der verwendeten Materialien** | |
|---|---|
| 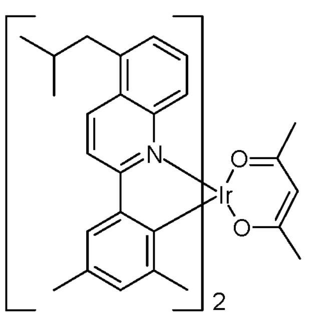 Ir3 [1388666-65-6] | 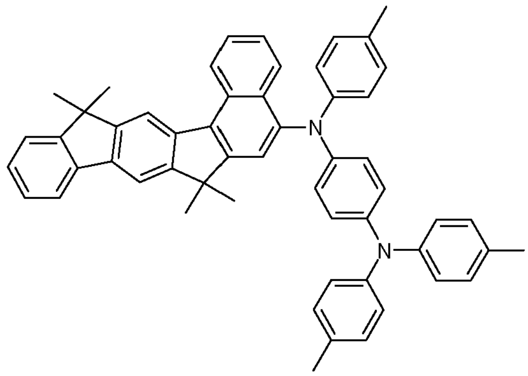 GD1 [1182175-24-1] |
| 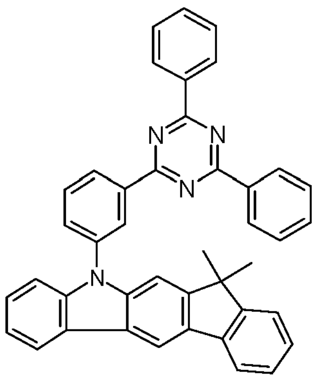 Ref-TMM1 [1257248-13-7] | 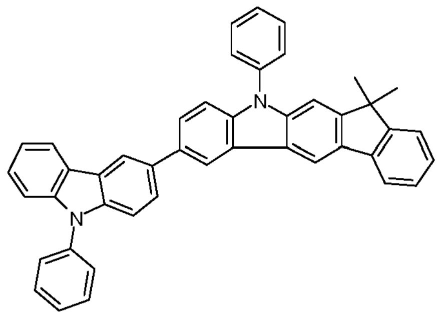 Ref-TMM2 [1357150-54-9] |

Die zuvor dargelegten Beispiele und Vergleichsbeispiele zeigen, dass durch die erfindungsgemäßen Verbindungen unerwartete Verbesserungen hinsichtlich der Lebensdauer, der Quanteneffizienz (EQE) und der notwendigen Betriebsspannung erzielt werden.

Beispielsweise zeigt ein Vergleich des Referenzbeispiels Ref.D1 mit den Beispielen D1, D2, D7, D8, D10 und D11 , die jeweils die Verbindung Ir1 als Emitter aufweisen, dass die erfindungsgemäßen Beispiele deutliche Verbesserungen in oben genannten Kriterien erzielen, ohne dass nennenswerte Nachteile in Kauf genommen werden müssten. Überraschende Vorteile hinsichtlich der Quanteneffizienz zeigt insbesondere Beispiel D11. Die Vorteilhaftigkeit von Strukturen gemäß Formeln (Vf) bis (Vk), insbesondere (Vh) wird durch den Vergleich der Beispiele D12 mit den Beispielen D15 und D16 bestätigt.

Ein ähnliches Resultat liefert ein Vergleich des Referenzbeispiels Ref.D2 mit den Beispielen D3, D4, D5 und D6, die jeweils die Verbindung Ir2 als Emitter aufweisen. Gleiches gilt für ein Vergleich des Referenzbeispiels Ref.D3 mit den Beispielen D9, D12, D13, D14, D15 und D16, die jeweils die Verbindung Ir3 als Emitter aufweisen. Hierbei zeigt Beispiel D9 gegenüber Ref-D3, dass die Lebensdauer mehr als verdoppelt wird, bei gleichzeitiger Verbesserung der Quanteneffizienz (EQE) und der notwendigen Betriebsspannung.

Weiterhin zeigt ein Vergleich der Beispiele D12, D13 und D14, dass durch die Verwendung von erfindungsgemäßen Materialien in einer Loch¬blockierschicht (HBL) unerwartet starke Verbesserungen erzielt werden können. Ferner führt auch die Verwendung von erfindungsgemäßen Materialien in einer Elektronentransportschicht (ETM) zu Verbesserungen.

## Patentansprüche

1. Elektronische Vorrichtung, enthaltend mindestens eine Verbindung, umfassend mindestens eine Struktur der Formel (Ia), vorzugsweise (Ib), (Ic), (Id), (Ie) und/oder (If),

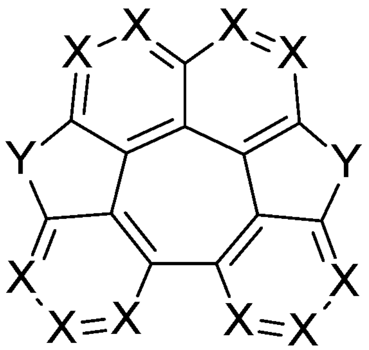

Formel (Ia)

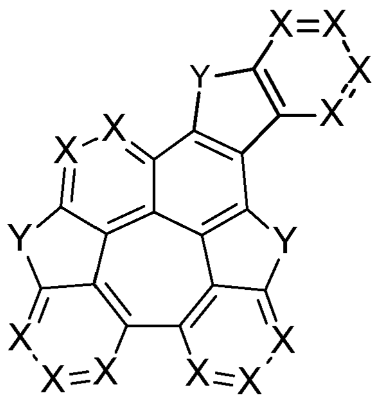

Formel (Ib)

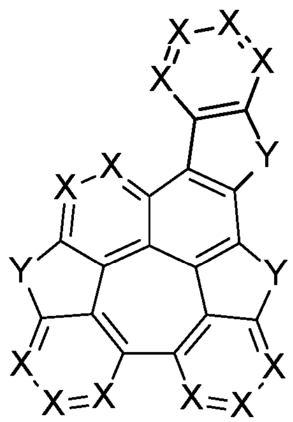

Formel (Ic)

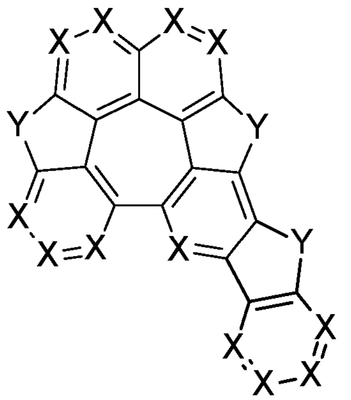

Formel (Id)

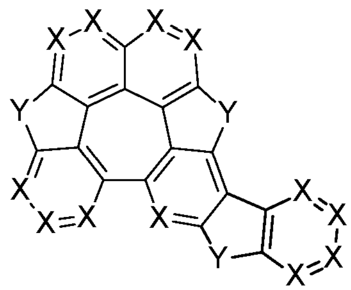

Formel (Ie)

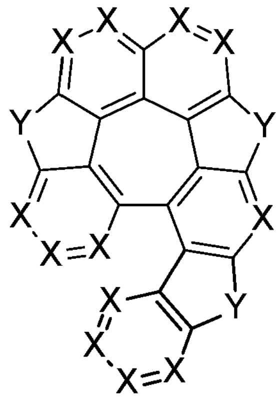

Formel (If),

wobei in den Formeln (Ia) bis (If) gilt:

X ist bei jedem Auftreten gleich oder verschieden CR; wobei höchstens 2 der Gruppen CR, für die X steht, ungleich der Gruppe CH ist;

Y ist bei jedem Auftreten gleich oder verschieden eine Brücke, ausgewählt aus O, S, B(R), C=O, N(R) und N(Ar), besonders bevorzugt O, S, N(Ar);

R ist bei jedem Auftreten gleich oder verschieden H, D, F, CN, $N(Ar)_2$, $N(R^1)_2$, $Si(Ar)_3$, $Si(R^1)_3$, $B(Ar)_2$, $B(R^1)_2$, eine geradkettige Alkyl-, oder Alkoxygruppe mit 1 bis 40 C-Atomen oder eine verzweigte oder cyclische Alkyl-, oder Alkoxygruppe mit 3 bis 20 C-Atomen, wobei die Alkyl-, oder Alkoxygruppe jeweils mit einem oder mehreren

Resten $R^1$ substituiert sein kann, oder ein aromatisches oder heteroaromatisches Ringsystem mit 5 bis 60 aromatischen Ringatomen, das jeweils durch einen oder mehrere Reste $R^1$ substituiert sein kann; dabei können zwei Reste R auch miteinander ein Ringsystem bilden;

Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann, dabei können zwei Reste Ar, welche an dasselbe Si-Atom, N-Atom, oder B-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, $N(R^1)$, $P(R^1)$ und $P(=O)R^1$, miteinander verbrückt sein;

$R^1$ ist bei jedem Auftreten gleich oder verschieden H, D, F, CN, $N(Ar^1)_2$, $N(R^2)_2$, $B(Ar^1)_2$, $B(R^2)_2$, $Si(Ar^1)_3$, $Si(R^2)_3$, eine geradkettige Alkyl-, oder Alkoxygruppe mit 1 bis 40 C-Atomen oder eine verzweigte oder cyclische Alkyl-, oder Alkoxygruppe mit 3 bis 40 C-Atomen, die jeweils mit einem oder mehreren Resten $R^2$ substituiert sein kann, wobei ein oder mehrere H-Atome durch D ersetzt sein können, oder ein aromatisches oder heteroaromatisches Ringsystem mit 5 bis 60 aromatischen Ringatomen, das jeweils durch einen oder mehrere Reste $R^2$ substituiert sein kann; dabei können zwei oder mehrere, vorzugsweise benachbarte Reste $R^1$ miteinander ein Ringsystem bilden; dabei können einer oder mehrere Reste $R^1$ mit einem weiteren Teil der Verbindung ein Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann, dabei können zwei Reste $Ar^1$, welche an dasselbe Si-Atom, N-Atom, oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, $N(R^2)$, $P(R^2)$ und $P(=O)R^2$, miteinander verbrückt sein;

$R^2$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten $R^2$ miteinander ein Ringsystem bilden.

2. Elektronische Vorrichtung gemäß Anspruch 1, **dadurch gekennzeichnet, dass** die Verbindung mindestens eine Struktur der Formeln (IIa), (IIb), (IIc), (IId), (IIe) und/oder (IIf) umfasst,

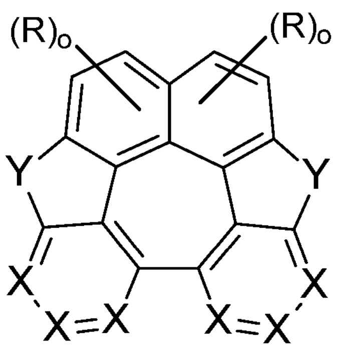

Formel (IIa)

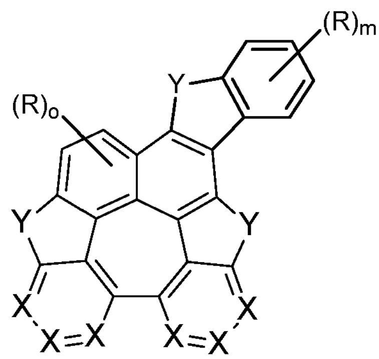

Formel (IIb)

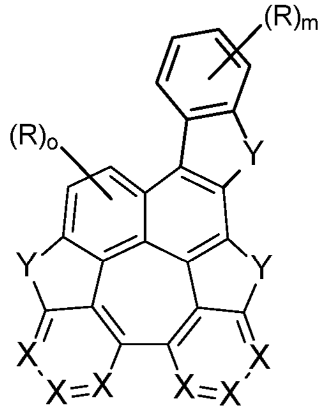

Formel (IIc)

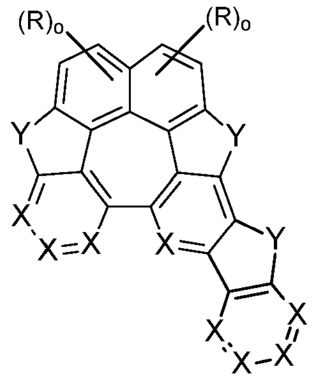

Formel (IId)

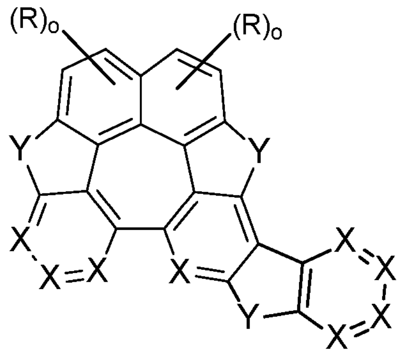

Formel (IIe)

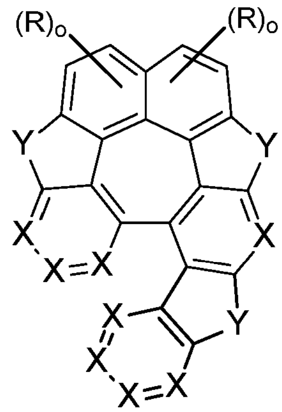

Formel (IIf),

wobei die Reste X, Y und R die in Anspruch 1 genannte Bedeutung haben, der Index m gleich oder verschieden 0, 1, 2, 3 oder 4, bevorzugt 0, 1, 2 oder 3, vorzugsweise 0, 1 oder 2, besonders bevorzugt 0 oder 1 ist und der Index o gleich oder verschieden 0, 1 oder 2, vorzugsweise 0 oder 1 ist, wobei die Summe der Indices o beziehungsweise o und m vorzugsweise 1 oder 2, besonders bevorzugt 1 ist.

3. Elektronische Vorrichtung gemäß Anspruch 1 oder 2, **dadurch gekennzeichnet, dass** die Verbindung mindestens eine Struktur der Formeln (IIIa), (IIIb), (IIIc), (IIId), (IIIe) und/oder (IIIf) umfasst,

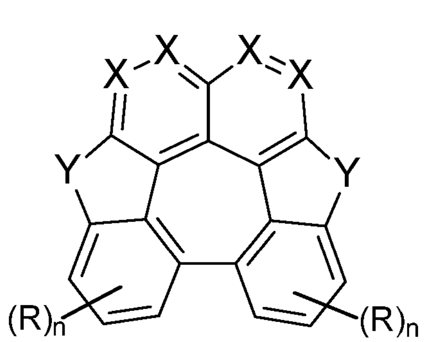

Formel (IIIa)

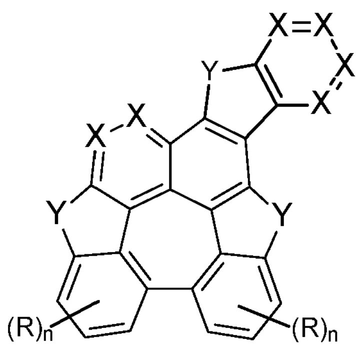

Formel (IIIb)

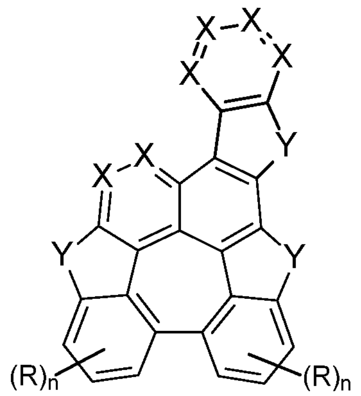

Formel (IIIc)

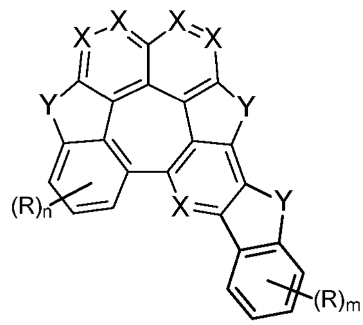

Formel (IIId)

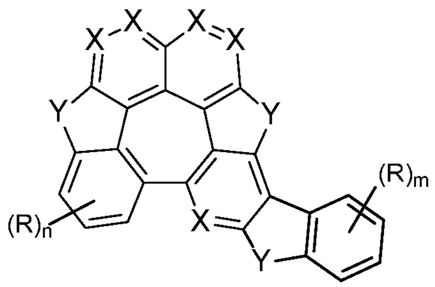

Formel (IIIe)

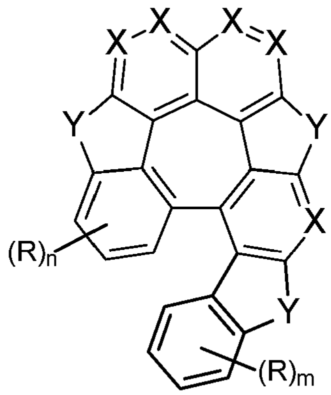

Formel (IIIf),

wobei die Reste X, Y und R die in Anspruch 1 genannte Bedeutung haben, der Index m gleich oder verschieden 0, 1, 2, 3 oder 4, bevorzugt 0, 1, 2 oder 3, vorzugsweise 0, 1 oder 2, besonders bevorzugt 0 oder 1 ist und der Index n gleich oder verschieden 0, 1, 2 oder 3, vorzugsweise 0, 1 oder 2, besonders bevorzugt 0 oder 1 ist, wobei die Summe der Indices n beziehungsweise n und m vorzugsweise 1 oder 2, besonders bevorzugt 1 ist.

**4.** Elektronische Vorrichtung gemäß einem oder mehreren der vorhergehenden Ansprüche, **dadurch gekennzeichnet, dass** die Verbindung mindestens eine Struktur der Formeln (IVa), (IVb), (IVc), (IVd), (IVe) und/oder (IVf) umfasst,

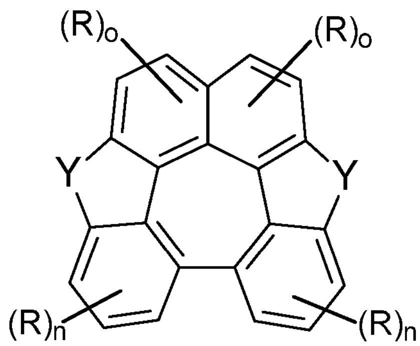

Formel (IVa)

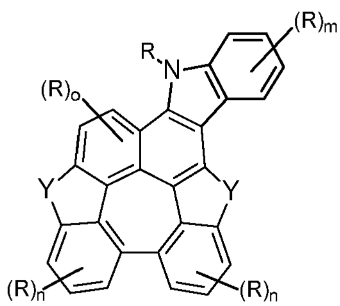

Formel (IVb)

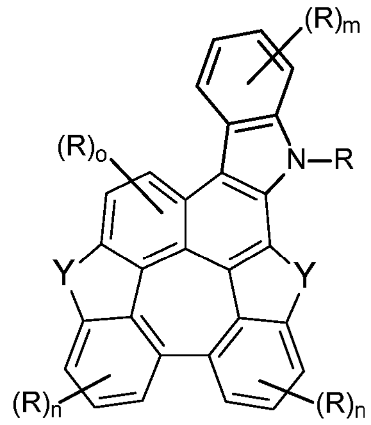

Formel (IVc)

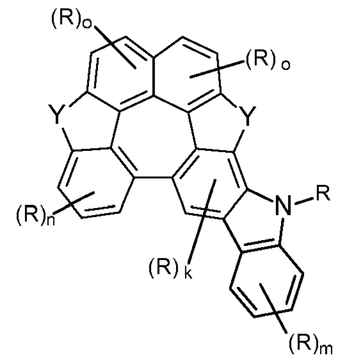

Formel (IVd)

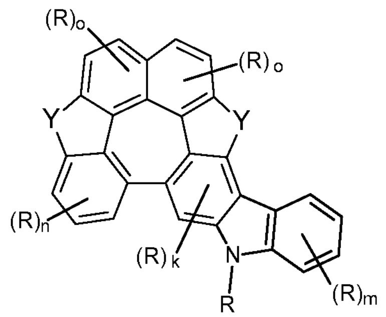

Formel (IVe)

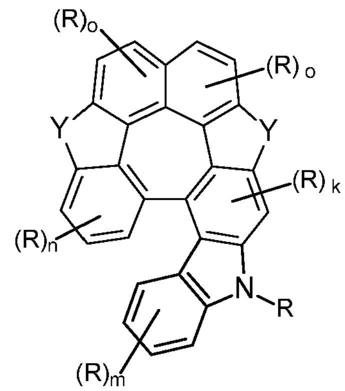

Formel (IVf),

wobei die Reste Y und R die in Anspruch 1 genannte Bedeutung haben, der Index k gleich oder verschieden 0 oder 1, vorzugsweise 0 ist, der Index o gleich oder verschieden 0, 1 oder 2, vorzugsweise 0 oder 1 ist, der Index n gleich oder verschieden 0, 1, 2 oder 3, vorzugsweise 0, 1 oder 2, besonders bevorzugt 0 oder 1 ist und der Index m gleich oder verschieden 0, 1, 2, 3 oder 4, bevorzugt 0, 1, 2 oder 3, vorzugsweise 0, 1 oder 2, besonders bevorzugt 0 oder 1 ist, wobei die Summe der Indices k, m, n und o vorzugsweise 1 oder 2, besonders bevorzugt 1 ist.

**5.** Elektronische Vorrichtung gemäß einem oder mehreren der vorhergehenden Ansprüche 2 bis 4, **dadurch gekennzeichnet, dass** die Summe der Indices k, m, n und o höchstens 6, vorzugsweise höchstens 4, besonders bevorzugt höchstens 2 und vorzugsweise mindestens 1 und speziell bevorzugt genau 1 beträgt.

**6.** Elektronische Vorrichtung gemäß einem oder mehreren der vorhergehenden Ansprüche, **dadurch gekennzeichnet, dass** die Verbindung mindestens eine Struktur der Formeln (Va), (Vb), (Vc), (Vd), (Ve), (Vf), (Vg), (Vh), (Vi), (Vj) und/oder (Vk) umfasst,

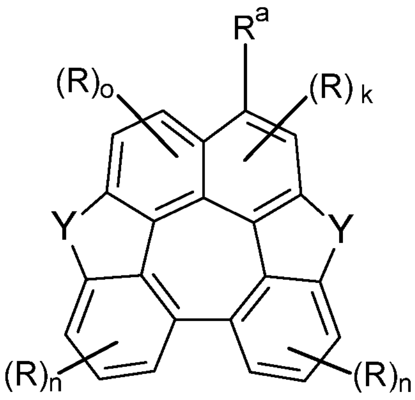

Formel (Va)

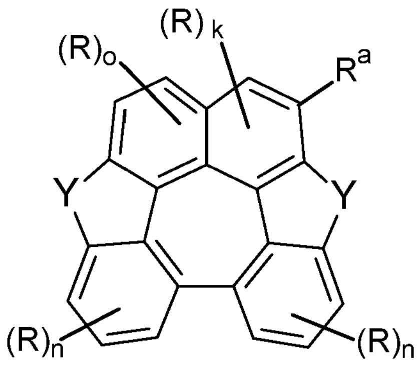

Formel (Vb)

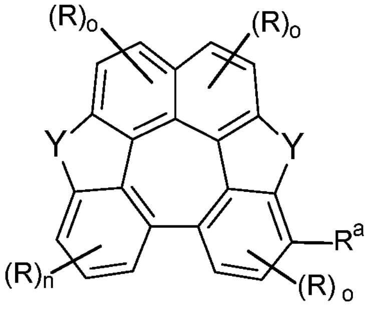

Formel (Vc)

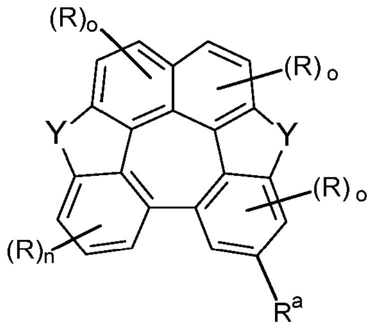

Formel (Vd)

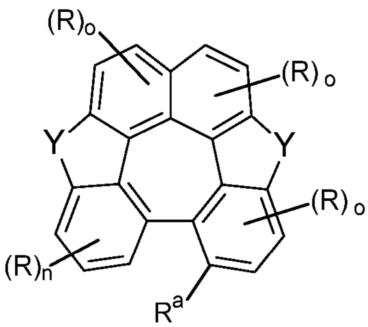

Formel (Ve)

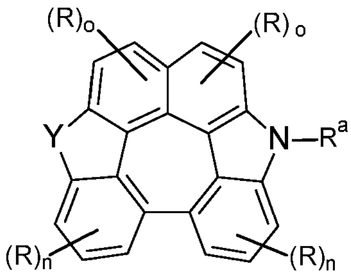

Formel (Vf)

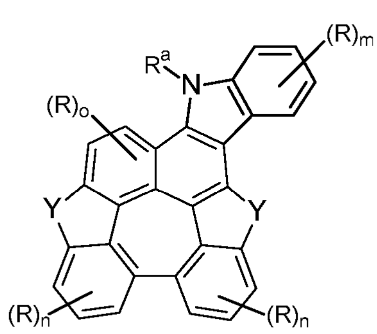

Formel (Vg)

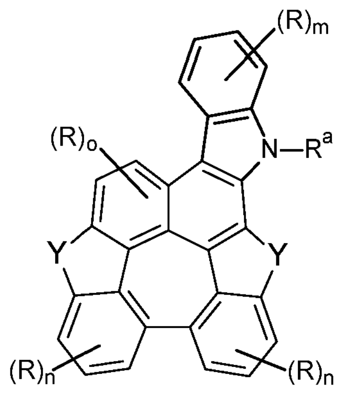

Formel (Vh)

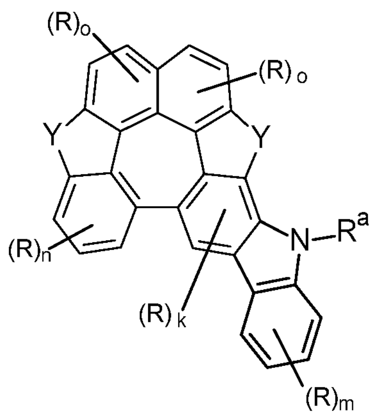

Formel (Vi)

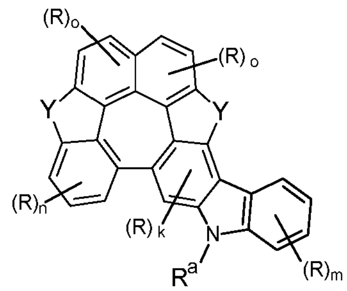

Formel (Vj)

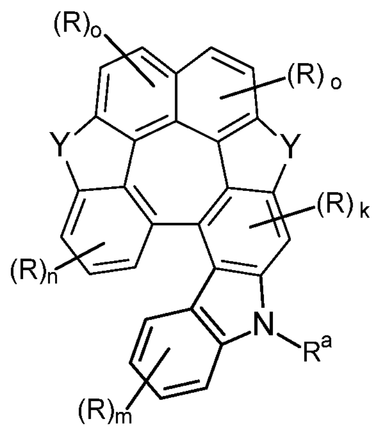

Formel (Vk),

wobei die Reste Y und R die in Anspruch 1 genannte Bedeutung haben und weiterhin gilt:

$R^a$ ist bei jedem Auftreten gleich oder verschieden F, CN, $N(Ar)_2$, $N(R^1)_2$, $Si(Ar)_3$, $Si(R^1)_3$, $B(Ar)_2$, $B(R^1)_2$, eine geradkettige Alkyl-, oder Alkoxygruppe mit 1 bis 20 C-Atomen oder eine verzweigte oder cyclische Alkyl-, oder Alkoxygruppe mit 3 bis 40 C-Atomen, wobei die Alkyl-, oder Alkoxygruppe jeweils mit einem oder mehreren Resten $R^1$ substituiert sein kann, oder ein aromatisches oder heteroaromatisches Ringsystem mit 5 bis 60 aromatischen Ringatomen, das jeweils durch einen oder mehrere Reste $R^1$ substituiert sein kann; dabei können Reste $R^a$ auch mit einem Rest R ein Ringsystem bilden, wobei die Reste Ar und $R^1$ die in Anspruch 1 genannte

Bedeutung haben;
k ist gleich oder verschieden 0 oder 1, vorzugsweise 0,
o ist gleich oder verschieden 0, 1 oder 2, vorzugsweise 0 oder 1;
n ist gleich oder verschieden 0, 1, 2 oder 3, vorzugsweise 0, 1 oder 2, besonders bevorzugt 0 oder 1; und
m ist gleich oder verschieden 0, 1, 2, 3 oder 4, vorzugsweise 0, 1 oder 2, besonders bevorzugt 0 oder 1.

**7.** Elektronische Vorrichtung gemäß Anspruch 6, **dadurch gekennzeichnet, dass** die Summe der Indices k, m, n und o höchstens 6, vorzugsweise höchstens 4, besonders bevorzugt höchstens 2, speziell bevorzugt höchstens 1 beträgt und ganz besonders bevorzugt 0 ist.

**8.** Elektronische Vorrichtung gemäß mindestens einem der vorhergehenden Ansprüche, **dadurch gekennzeichnet, dass** mindestens einer der Reste R und/oder $R^a$ ausgewählt ist aus der Gruppe der Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene, Anthracene, Benzanthracene, Fluoradene.

**9.** Elektronische Vorrichtung gemäß mindestens einem der vorhergehenden Ansprüche, **dadurch gekennzeichnet, dass** die Verbindung eine Lochtransportgruppe umfasst, wobei vorzugsweise eine der Gruppen R und/oder $R^a$ eine Lochtransportgruppe umfasst.

**10.** Elektronische Vorrichtung gemäß mindestens einem der vorhergehenden Ansprüche, **dadurch gekennzeichnet, dass** eine der Gruppen R und/oder $R^a$ eine Lochtransportgruppe darstellt.

**11.** Elektronische Vorrichtung gemäß mindestens einem der vorhergehenden Ansprüche, **dadurch gekennzeichnet, dass** eine der Gruppen R und/oder $R^a$ eine Elektronentransportgruppe darstellt.

**12.** Verbindung gemäß der Formel (Ib), (Ic), (Id), (Ie) oder (If),

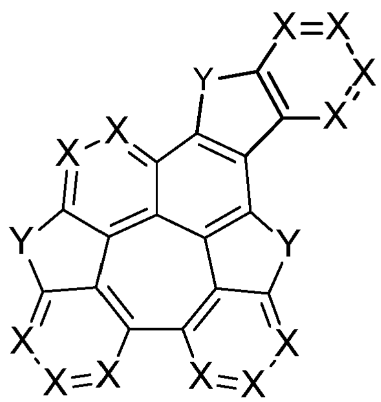

Formel (Ib)

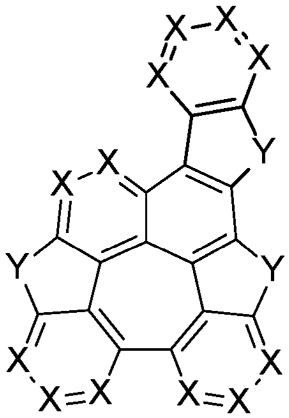

Formel (Ic)

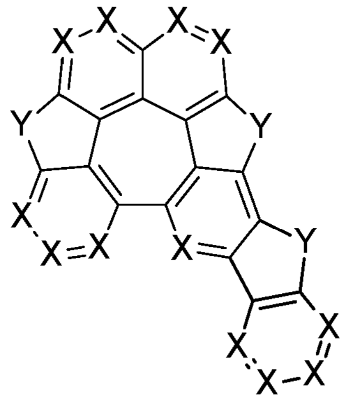

Formel (Id)

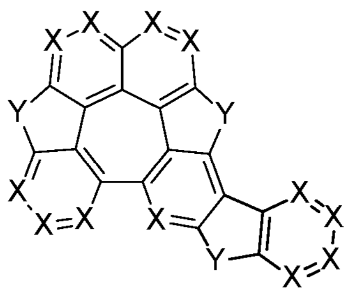

Formel (Ie)

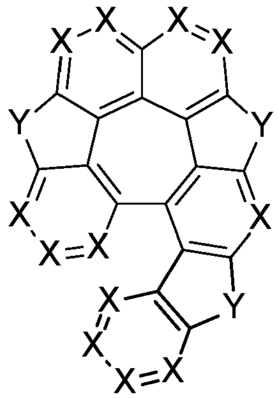

Formel (If),

wobei die Reste X und Y die in Anspruch 1 genannte Bedeutung haben.

**13.** Verbindung gemäß der Formel (Ia),

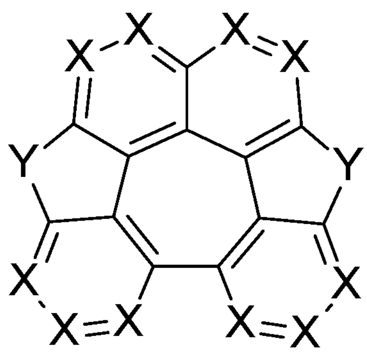

Formel (Ia),

wobei gilt

X ist bei jedem Auftreten gleich oder verschieden CR, wobei höchstens 2 der Gruppen CR, für die X steht, ungleich der Gruppe CH ist;

Y ist bei jedem Auftreten gleich oder verschieden eine Brücke, ausgewählt aus N(Ar), O, S, B(R) und C=O, besonders bevorzugt N(Ar),O und S;

R ist bei jedem Auftreten gleich oder verschieden H, D, F, CN, $N(Ar)_2$, $N(R^1)_2$, $Si(Ar)_3$, $Si(R^1)_3$, $B(Ar)_2$, $B(R^1)_2$, eine geradkettige Alkyl-, oder Alkoxygruppe mit 1 bis 40 C-Atomen oder eine verzweigte oder cyclische Alkyl-, oder Alkoxygruppe mit 3 bis 20 C-Atomen, wobei die Alkyl-, oder Alkoxygruppe jeweils mit einem oder mehreren Resten $R^1$ substituiert sein kann, oder ein aromatisches oder heteroaromatisches Ringsystem mit 5 bis 60 aromatischen Ringatomen, das jeweils durch einen oder mehrere Reste $R^1$ substituiert sein kann;
Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann, dabei können zwei Reste Ar, welche an dasselbe Si-Atom, N-Atom oder B-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, $N(R^1)$, $P(R^1)$ und $P(=O)R^1$, miteinander verbrückt sein;
$R^1$ ist bei jedem Auftreten gleich oder verschieden H, D, F, CN, $N(Ar^1)_2$, $N(R^2)_2$, $B(Ar^1)_2$, $B(R^2)_2$, $Si(Ar^1)_3$, $Si(R^2)_3$, eine geradkettige Alkyl-, oder Alkoxygruppe mit 1 bis 40 C-Atomen oder eine verzweigte oder cyclische Alkyl-, oder Alkoxygruppe mit 3 bis 40 C-Atomen, die jeweils mit einem oder mehreren Resten $R^2$ substituiert sein kann, wobei ein oder mehrere H-Atome durch D ersetzt sein können, oder ein aromatisches oder heteroaromatisches Ringsystem mit 5 bis 60 aromatischen Ringatomen, das jeweils durch einen oder mehrere Reste $R^2$ substituiert sein kann;
$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann, dabei können zwei Reste $Ar^1$, welche an dasselbe Si-Atom, N-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, $N(R^2)$, $P(R^2)$ und $P(=O)R^2$, miteinander verbrückt sein;
$R^2$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D ersetzt sein können und das durch ein oder mehrere Alkylgruppen mit jeweils 1 bis 4 Kohlenstoffatomen substituiert sein kann.

**14.** Oligomer, Polymer oder Dendrimer enthaltend eine oder mehrere Verbindungen nach Anspruch 12 oder 13, wobei statt eines Wasserstoffatoms oder eines Substituenten ein oder mehrere Bindungen der Verbindungen zum Polymer, Oligomer oder Dendrimer vorhanden sind.

**15.** Zusammensetzung enthaltend wenigstens eine Verbindung nach Anspruch 12 oder 13 oder ein Oligomer, Polymer oder Dendrimer nach Anspruch 14 und wenigstens eine weitere Verbindung ausgewählt aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Elektroneninjektionsmaterialien, Lochtransportmaterialien, Loch-injektionsmaterialien, Elektronenblockiermaterialien und Loch-blockiermaterialien, Wide-Band-Gap-Materialien und n-Dotanden.

**16.** Formulierung, enthaltend mindestens eine Verbindung nach Anspruch 12 oder 13 oder ein Oligomer, Polymer oder Dendrimer nach Anspruch 14 oder eine Zusammensetzung nach Anspruch 15 und mindestens ein Lösemittel.

**17.** Verwendung einer Verbindung nach Anspruch 12 oder 13, eines Oligomers, Polymers oder Dendrimers nach Anspruch 14 oder einer Zusammensetzung nach Anspruch 15 in einer elektronischen Vorrichtung als Emitter, vorzugsweise fluoreszierenden Emitter, Emitter, der TADF (thermally activated delayed fluorescence) zeigt, Hostmaterial, Elektronentransportmaterial, Elektroneninjektionsmaterial, Lochtransportmaterial, Lochinjektionsmaterial, Elektronenblockiermaterial, Lochblockiermaterial und/oder Wide-Band-Gap-Material, besonders bevorzugt als fluoreszierenden Emitter (Singulet-Emitter), Hostmaterial, Lochtransportmaterial und/oder Elektronentransportmaterial.

**18.** Verfahren zur Herstellung einer Verbindung nach Anspruch 12 oder 13 oder eines Oligomers, Polymers und/oder Dendrimers nach Anspruch 14, **dadurch gekennzeichnet, dass** in einer Kupplungsreaktion eine Verbindung, umfassend eine heterocyclische Struktur, mit einer Verbindung, umfassend mindestens eine aromatische oder heteroaromatische Gruppe, verbunden wird.

**19.** Elektronische Vorrichtung nach einem oder mehreren der Ansprüche 1 bis 11, enthaltend mindestens eine Verbindung nach Anspruch 12 oder 13, ein Oligomer, Polymer oder Dendrimer nach Anspruch 14 oder eine Zusammensetzung gemäß Anspruch 15, wobei die elektronische Vorrichtung ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

**1.** An electronic device comprising at least one compound comprising at least one structure of formula (Ia), preferably (Ib), (Ic), (Id), (Ie) and/or (If),

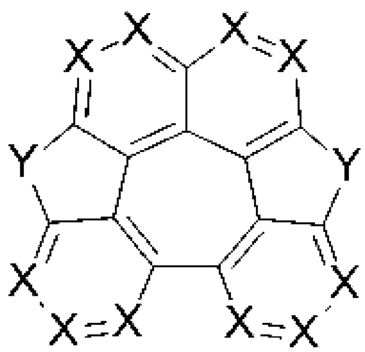

formula (Ia)

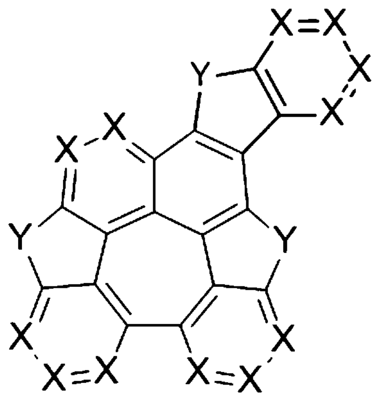

formula (Ib)

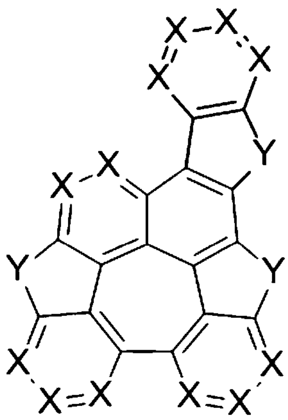

formula (Ic)

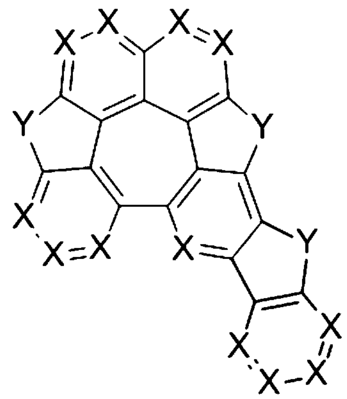

formula (Id)

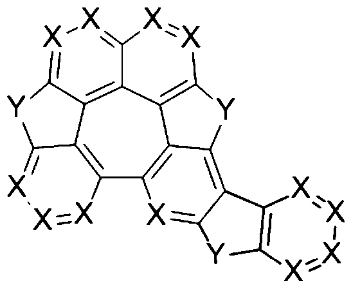

formula (Ie)

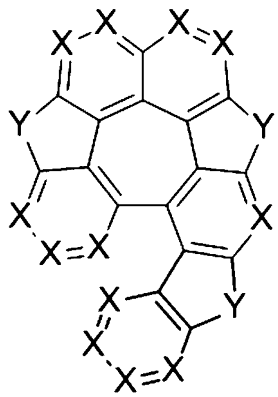

formula (If),

wherein formulae (Ia) to (If) are as follows

X is equal to or different from CR at each occurrence; wherein at most 2 of the groups CR, for which X stands, are not equal to the group CH;
Y is, at each occurrence, identical or different, a bridge selected from O, S, B(R), C=O, N(R) and N(Ar), particularly preferred O, S, N(Ar);
R in each occurrence is the same or different H, D, F, CN, $N(Ar)_2$, $N(R^1)_2$, $Si(Ar)_3$, $Si(R^1)_3$, $B(Ar)_2$, $B(R^1)_2$, a straight-chain alkyl or alkoxy group having 1 to 40 carbon atoms or a branched or cyclic alkyl or alkoxy group having 3 to 20 carbon atoms, wherein the alkyl or alkoxy group may each be substituted by one or more radicals $R^1$, or an aromatic or heteroaromatic ring system with 5 to 60 aromatic ring atoms, which may each be substituted by one or more radicals $R^1$; two radicals R can also form a ring system with each other;
Ar is in each occurrence the same or different an aromatic or heteroaromatic ring system with 5 to 60 aromatic ring atoms, which can be substituted by one or more radicals $R^1$, whereby two radicals Ar, which bind to the same Si atom, N atom or B atom can also be linked by a single bond or a bridge selected from $B(R^1)$, $C(R^1)_2$, $Si(R^1)_2$, C=O, $C=NR^1$, $C=C(R^1)_2$, O, $N(R^1)$, $P(R^1)$ and $P(=O)R^1$;
$R^1$ is the same or different for each occurrence H, D, F, CN, $N(Ar^1)_2$, $N(R^2)_2$, $B(Ar^1)_2$, $B(R^2)_2$, $Si(Ar^1)_3$, $Si(R^2)_3$, a straight-chain alkyl or alkoxy group with 1 to 40 carbon atoms or a branched or cyclic alkyl or alkoxy group with 3 to 40 carbon atoms, which may each be substituted by one or more radicals $R^2$, wherein one or more H atoms may be replaced by D, or an aromatic or heteroaromatic ring system with 5 to 60 aromatic ring atoms, which may each be substituted by one or more radicals $R^2$; two or more preferably neighbouring radicals $R^1$ can form a ring system with one another; one or more radicals $R^1$ can form a ring system with a further part of the communication;
$Ar^1$ is in each occurrence the same or different an aromatic or heteroaromatic ring system with 5 to 30 aromatic ring atoms, which can be substituted with one or more nonaromatic radicals $R^2$, whereby two radicals $Ar^1$, which bind to the same Si atom, N atom or B atom, can also be linked by a single bond or a bridge selected from $B(R^2)$, $C(R^2)_2$, $Si(R^2)_2$, C=O, $C=NR^2$, $C=C(R^2)_2$, O, $N(R^2)$, $P(R^2)$ and $P(=O)R^2$;
$R^2$ in each occurrence is the same or different and is selected from the group consisting of H, D, F, CN, an aliphatic hydrocarbon radical having 1 to 20 carbon atoms or an aromatic or heteroaromatic ring system having 5 to 30 aromatic ring atoms, in which one or more H atoms may be replaced by D and which may be substituted by one or more alkyl groups each having 1 to 4 carbon atoms, two or more preferably neighbouring substituents $R^2$ forming a ring system with one another.

2. Electronic device according to claim 1, **characterised in that** the compound comprises at least one structure of formulae (IIa), (IIb), (IIc), (IId), (IIe) and/or (IIf),

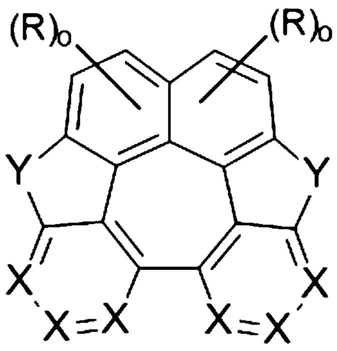

formula (IIa)

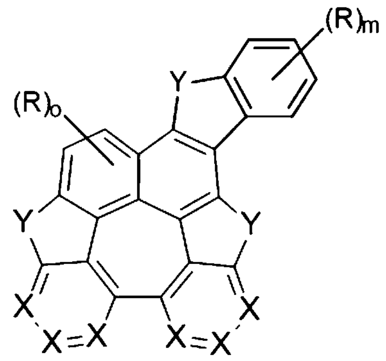

formula (IIb)

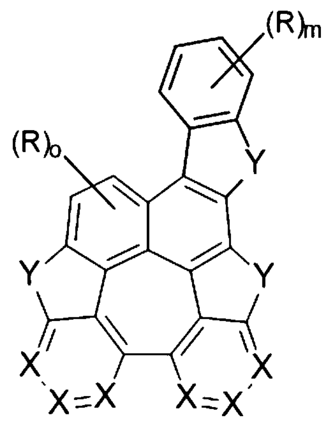

formula (IIc)

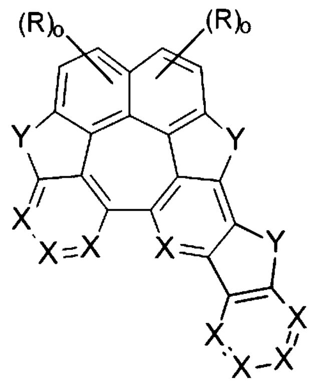

formula (IId)

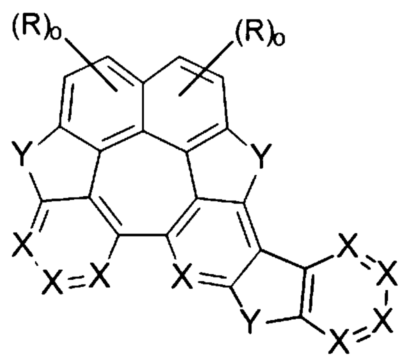

formula (IIe)

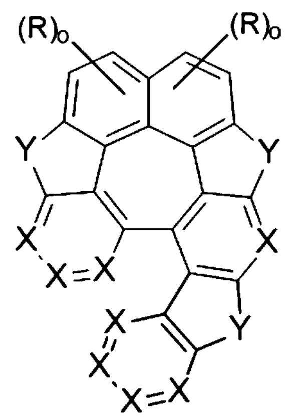

formula (IIf),

wherein the radicals X, Y and R have the meaning given in claim 1, the index m is equal to or different from 0, 1, 2, 3 or 4, preferably 0, 1, 2 or 3, preferably 0, 1 or 2, particularly preferably 0 or 1, and the index o is equal to or different from 0, 1 or 2, preferably 0 or 1, wherein the sum of the indices o or 0 and m is preferably 1 or 2, particularly preferably 1.

3. Electronic device according to claim 1 or 2, **characterised in that** the compound comprises at least one structure of formulae (IIIa), (IIIb), (IIIc), (IIId), (IIIe) and/or (IIIf),

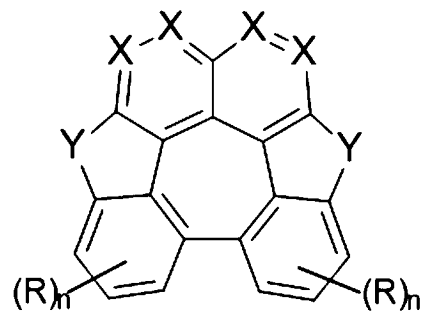

formula (IIIa)

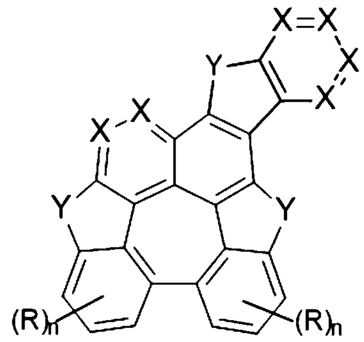

formula (IIIb)

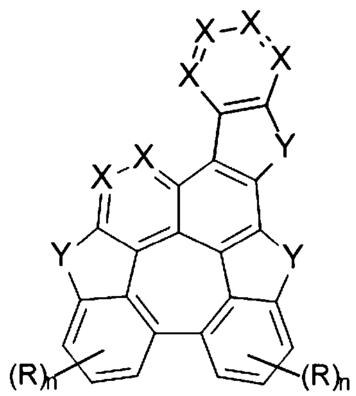

formula (IIIc)

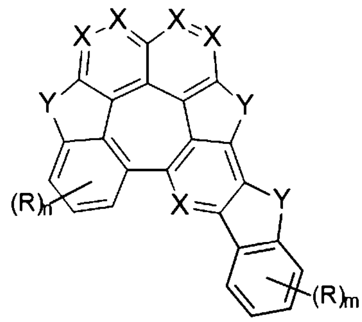

formula (IIId)

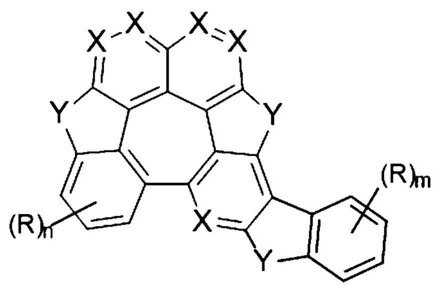

formula (IIIe)

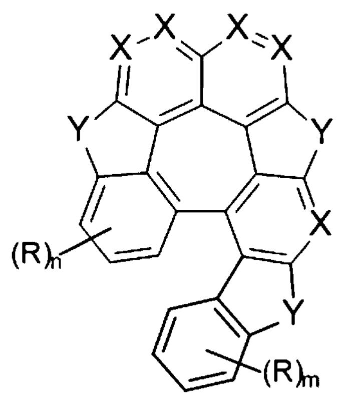

formula (IIIf),

wherein the radicals X, Y and R have the meaning given in claim 1, the index m is equal to or different from 0, 1, 2, 3 or 4, preferably 0, 1, 2 or 3, preferably 0, 1 or 2, particularly preferably 0 or 1, and the index n is equal to or different from 0, 1, 2 or 3, preferably 0, 1 or 2, particularly preferably 0 or 1, wherein the sum of the indices n or n and m is preferably 1 or 2, particularly preferably 1.

4. Electronic device according to one or more of the preceding claims, **characterised in that** the compound comprises at least one structure of formulae (IVa), (IVb), (IVc), (IVd), (IVe) and/or (IVf),

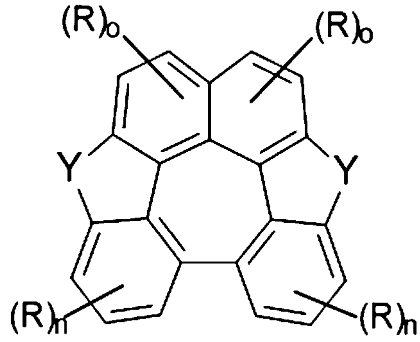

formula (IVa)

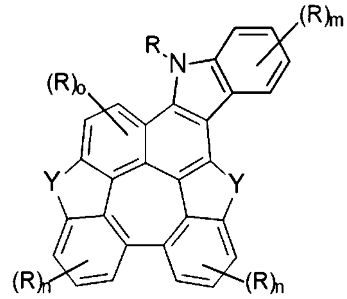

formula (IVb)

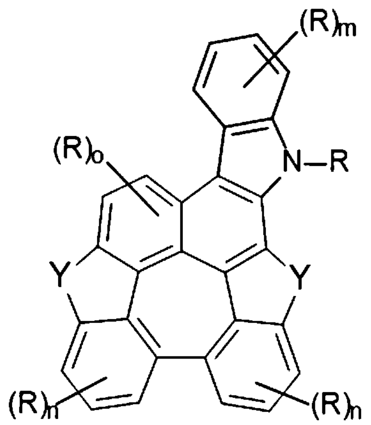

formula (IVc)

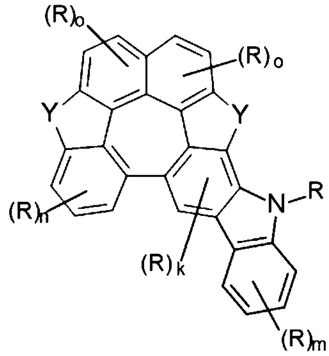

formula (IVd)

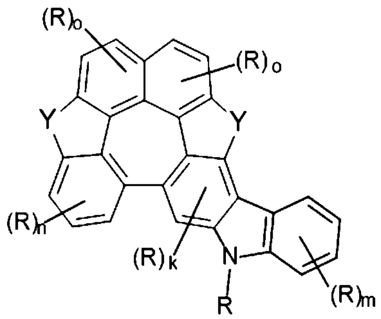

formula (IVe)

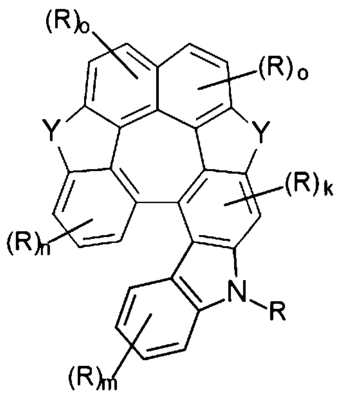

formula (IVf),

wherein the radicals Y and R are as defined in claim 1, the subscript k is equal to or different from 0 or 1, preferably 0, the subscript o is equal to or different from 0, 1 or 2, preferably 0 or 1, the subscript n is equal to or different from 0, 1, 2 or 3, preferably 0, 1 or 2, particularly preferred 0 or 1, and the index m is equal to or different from 0, 1, 2, 3 or 4, preferably 0, 1, 2 or 3, preferably 0, 1 or 2, particularly preferred 0 or 1, wherein the sum of the indices k, m, n and o is preferably 1 or 2, particularly preferred 1.

**5.** Electronic device according to one or more of the preceding claims 2 to 4, **characterised in that** the sum of the indices

k, m, n and o is at most 6, preferably at most 4, particularly preferably at most 2 and preferably at least 1 and particularly preferably exactly 1.

**6.** Electronic device according to one or more of the preceding claims, **characterised in that** the compound comprises at least one structure of formulae (Va), (Vb), (Vc), (Vd), (Ve), (Vf), (Vg), (Vh), (Vi), (Vj) and/or (Vk),

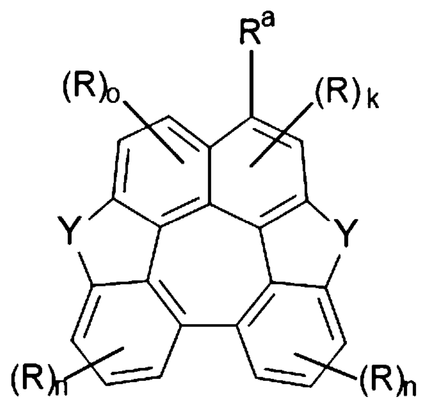

formula (Va)

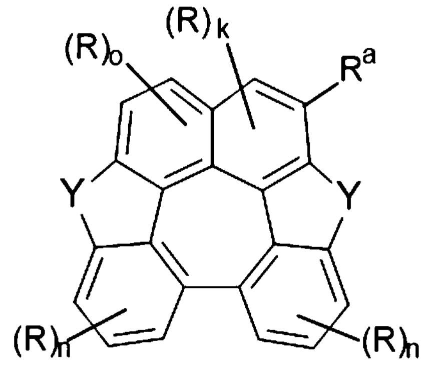

formula (Vb)

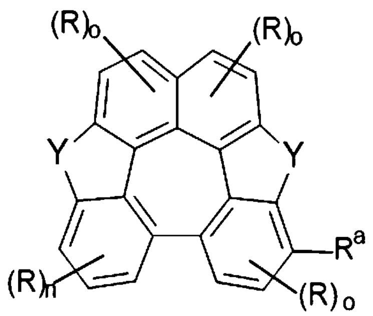

formula (Vc)

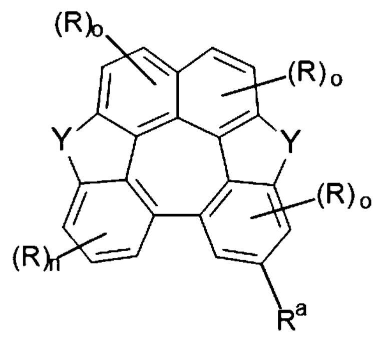

formula (Vd)

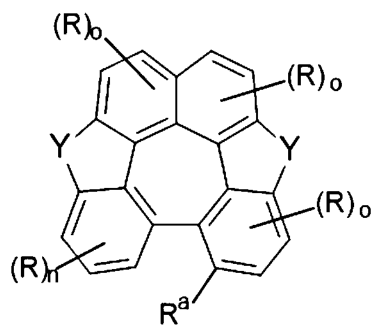

formula (Ve)

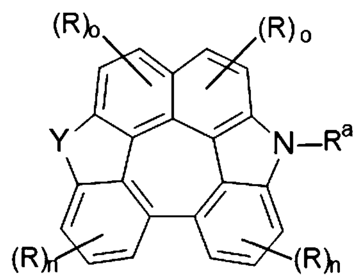

formula (Vf)

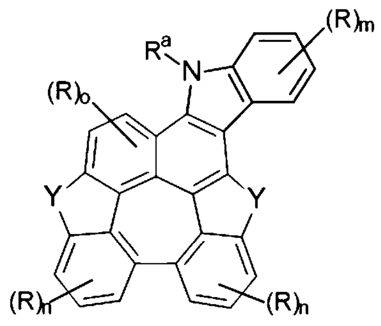

formula (Vg)

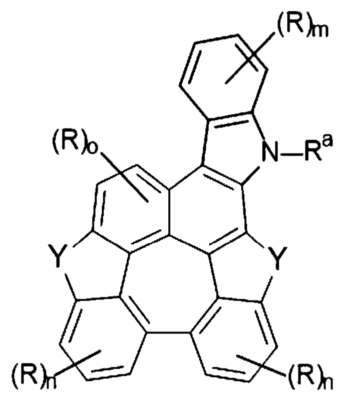

formula (Vh)

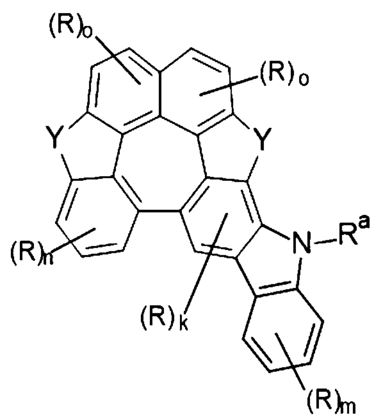

formula (Vi)

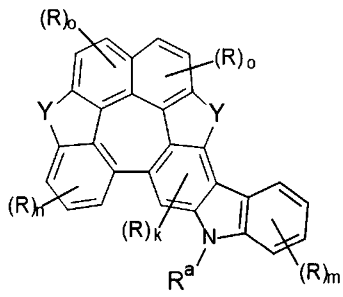

formula (Vj)

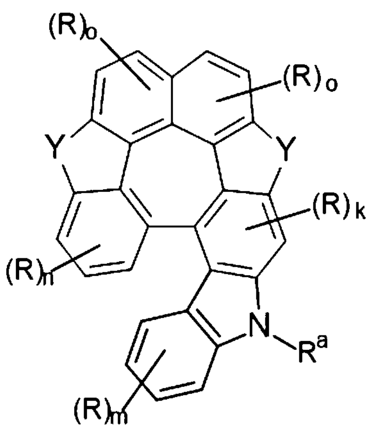

formula (Vk),

wherein the radicals Y and R are as defined in claim 1 and furthermore

$R^a$ in each occurrence is the same or different F, CN, $N(Ar)_2$, $N(R^1)_2$, $Si(Ar)_3$, $Si(R^1)_3$, $B(Ar)_2$, $B(R^1)_2$, a straight-chain alkyl or alkoxy group having 1 to 20 carbon atoms or a branched or cyclic alkyl or alkoxy group having 3 to 40 carbon atoms, wherein the alkyl or alkoxy group may each be substituted by one or more radicals $R^1$, or an aromatic or heteroaromatic ring system with 5 to 60 aromatic ring atoms, which may each be substituted by one or

more radicals $R^1$; radicals $R^a$ can also form a ring system with a radical R, wherein the radicals Ar and $R^1$ have the meaning given in claim 1;
k is equal to or different from 0 or 1, preferably 0,
o is the same or different 0, 1 or 2, preferably 0 or 1;
n is equal to or different from 0, 1, 2 or 3, preferably 0, 1 or 2, particularly preferred 0 or 1; and
m is equal to or different from 0, 1, 2, 3 or 4, preferably 0, 1 or 2, particularly preferred 0 or 1.

**7.** Electronic device according to claim 6, **characterised in that** the sum of the indices k, m, n and o is at most 6, preferably at most 4, particularly preferably at most 2, especially preferably at most 1, and very particularly preferably 0.

**8.** Electronic device according to at least one of the preceding claims, **characterised in that** at least one of the radicals R and/or $R^a$ is selected from the group of fluorenes, indenofluorenes, spirobifluorenes, carbazoles, indenocarbazoles, indolocarbazoles, spirocarbazoles, pyrimidines, triazines, lactams, triarylamines, dibenzofurans, dibenzothienes, imidazoles, benzimidazoles, benzoxazoles, benzthiazoles, 5-aryl-phenanthridin-6-ones, 9,10-dehydrophenanthrenes, fluoranthenes, anthracenes, benzanthracenes, fluoradenes.

**9.** Electronic device according to at least one of the preceding claims, **characterised in that** the connection comprises a hole transport group, wherein preferably one of the groups R and/or $R^a$ comprises a hole transport group.

**10.** Electronic device according to at least one of the preceding claims, **characterised in that** one of the groups R and/or $R^a$ is a hole transport group.

**11.** Electronic device according to at least one of the preceding claims, **characterised in that** one of the groups R and/or $R^a$ represents an electron transport group.

**12.** A compound according to formula (Ib), (Ic), (Id), (Ie) or (If),

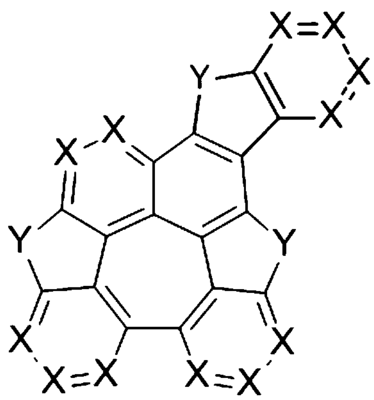

formula (Ib)

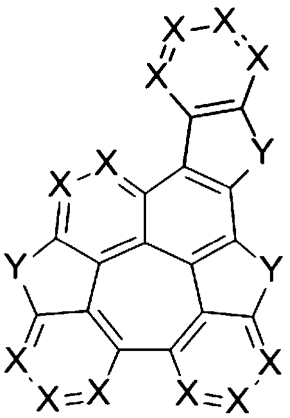

formula (Ic)

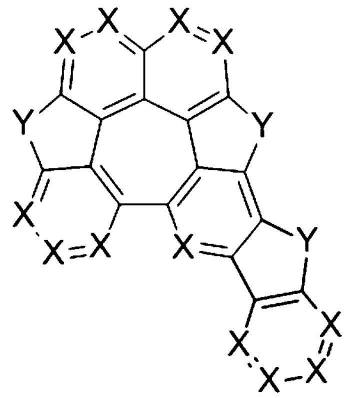

formula (Id)

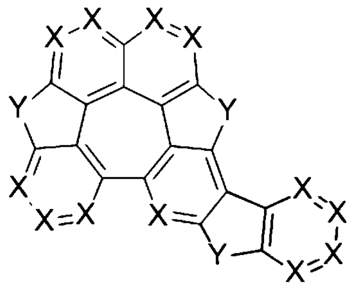

formula (Ie)

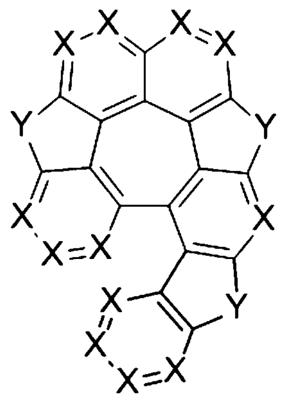

formula (If),

wherein the radicals X and Y are as defined in claim 1.

**13.** A compound according to formula (Ia),

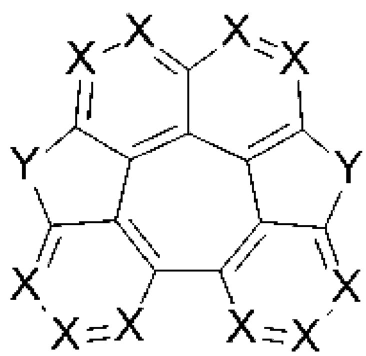

formula (Ia),

wherein

X is identical to or different from CR at each occurrence, wherein at most 2 of the groups CR, for which X stands, are not identical to the group CH;

Y is, at each occurrence, identical or different, a bridge selected from N(Ar), O, S, B(R) and C=O, particularly preferred N(Ar), O and S;
R is the same or different at each occurrence H, D, F, CN, $N(Ar)_2$, $N(R^1)_2$, $Si(Ar)_3$, $Si(R^1)_3$, $B(Ar)_2$, $B(R^1)_2$, a straight-chain alkyl or alkoxy group having 1 to 40 carbon atoms or a branched or cyclic alkyl or alkoxy group having 3 to 20 carbon atoms, wherein the alkyl or alkoxy group may be substituted in each case by one or more radicals $R^1$, or an aromatic or heteroaromatic ring system having 5 to 60 aromatic ring atoms, which may be substituted in each case by one or more radicals $R^1$;
Ar is in each occurrence the same or different an aromatic or heteroaromatic ring system with 5 to 60 aromatic ring atoms, which can be substituted by one or more radicals $R^1$, whereby two radicals Ar, which bind to the same Si atom, N atom or B atom can also be linked by a single bond or a bridge selected from $B(R^1)$, $C(R^1)_2$, $Si(R^1)_2$, C=O, $C=NR^1$, $C=C(R^1)_2$, O, $N(R^1)$, $P(R^1)$ and $P(=O)R^1$;
$R^1$ is the same or different for each occurrence H, D, F, CN, $N(Ar^1)_2$, $N(R^2)_2$, $B(Ar^1)_2$, $B(R^2)_2$, $Si(Ar^1)_3$, $Si(R^2)_3$, a straight-chain alkyl or alkoxy group with 1 to 40 carbon atoms or a branched or cyclic alkyl or alkoxy group with 3 to 40 carbon atoms, which may each be substituted by one or more radicals $R^2$, wherein one or more H atoms may be replaced by D, or an aromatic or heteroaromatic ring system with 5 to 60 aromatic ring atoms, which may each be substituted by one or more radicals $R^2$;
$Ar^1$ is in each occurrence the same or different an aromatic or heteroaromatic ring system with 5 to 30 aromatic ring atoms, which can be substituted with one or more nonaromatic radicals $R^2$, whereby two radicals $Ar^1$, which bind to the same Si atom, N atom or B atom can also be linked by a single bond or a bridge selected from $B(R^2)$, $C(R^2)_2$, $Si(R^2)_2$, C=O, $C=NR^2$, $C=C(R^2)_2$, O, $N(R^2)$, $P(R^2)$ and $P(=O)R^2$;
$R^2$ in each occurrence is the same or different and is selected from the group consisting of H, D, F, CN, an aliphatic hydrocarbon radical having 1 to 20 carbon atoms or an aromatic or heteroaromatic ring system having 5 to 30 aromatic ring atoms, in which one or more H atoms may be replaced by D and which may be substituted by one or more alkyl groups each having 1 to 4 carbon atoms.

**14.** An oligomer, polymer or dendrimer containing one or more compounds according to claim 12 or 13, wherein instead of a hydrogen atom or a substituent one or more bonds of the compounds to the polymer, oligomer or dendrimer are present.

**15.** A composition containing at least one compound according to claim 12 or 13 or an oligomer, polymer or dendrimer according to claim 14 and at least one further compound selected from the group consisting of fluorescent emitters, phosphorescent emitters, emitters exhibiting TADF (thermally activated delayed fluorescence), host materials, electron transport materials, electron injection materials, hole transport materials, hole injection materials, electron blocking materials and hole blocking materials, wide-band-gap materials and n-dotands.

**16.** A formulation containing at least one compound according to claim 12 or 13 or an oligomer, polymer or dendrimer according to claim 14 or a composition according to claim 15 and at least one solvent.

**17.** Use of a compound according to claim 12 or 13, an oligomer, polymer or dendrimer according to claim 14 or a composition according to claim 15 in an electronic device as emitter, preferably fluorescent emitter, emitter showing TADF (thermally activated delayed fluorescence), host material, electron transport material, electron injection material, hole transport material, hole injection material, electron blocking material, hole blocking material and/or wide-band-gap material, particularly preferred as fluorescent emitter (singlet emitter), host material, hole transport material and/or electron transport material.

**18.** A process for preparing a compound according to claim 12 or 13 or an oligomer, polymer and/or dendrimer according to claim 14, **characterised in that** a compound comprising a heterocyclic structure is combined in a coupling reaction with a compound comprising at least one aromatic or heteroaromatic group.

**19.** An electronic device according to one or more of claims 1 to 11, containing at least one compound according to claim 12 or 13, an oligomer, polymer or dendrimer according to claim 14 or a composition according to claim 15, wherein the electronic device is selected from the group consisting of organic electroluminescent devices, organic integrated circuits, organic field effect transistors, organic thin film transistors, organic light emitting transistors, organic solar cells, organic optical detectors, organic photoreceptors, organic field quench devices, light emitting electrochemical cells or organic laser diodes.

## Revendications

1. Dispositif électronique comprenant au moins un composé contenant au moins une structure de formule (Ia), de préférence (Ib), (Ic), (Id), (Ie) et/ou (If),

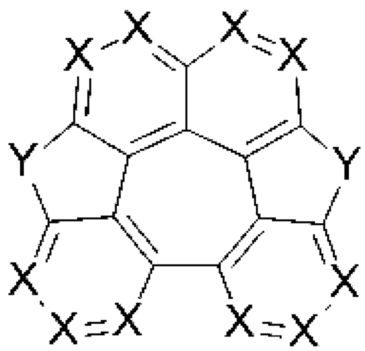

formule (Ia)

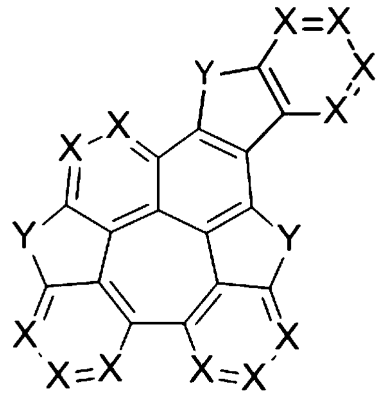

formule (Ib)

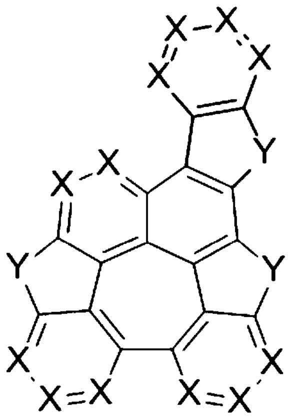

formule (Ic)

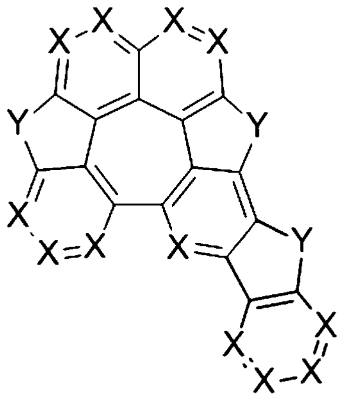

formule (Id)

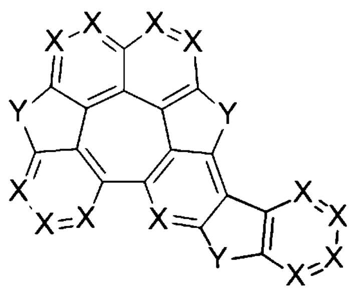

formule (Ie)

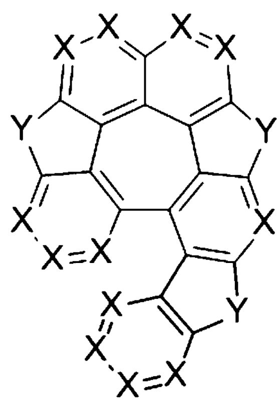

formule (If),

dans lesquelles, dans les formules (Ia) à (If), on a :

X est, à chaque occurrence, identique ou différent de CR ; dans lequel au plus 2 des groupes CR que représente X ne sont pas identiques au groupe CH ;

Y est, à chaque occurrence, identique ou différent, un pont choisi parmi O, S, B(R), C=O, N(R) et N(Ar), de manière particulièrement préférée O, S, N(Ar) ;

R est à chaque occurrence identique ou différente H, D, F, CN, $N(Ar)_2$, $N(R^1)_2$, $Si(Ar)_3$, $Si(R^1)_3$, $B(Ar)_2$, $B(R^1)_2$, un groupe alkyle ou alcoxy à chaîne droite ayant de 1 à 40 atomes de carbone ou un groupe alkyle ou alcoxy ramifié ou cyclique ayant de 3 à 20 atomes de carbone, dans lequel le groupe alkyle ou alcoxy peut être substitué dans chaque cas par un ou plusieurs radicaux $R^1$, ou un système cyclique aromatique ou hétéroaromatique comportant 5 à 60 atomes cycliques aromatiques, qui peut être substitué dans chaque cas par un ou plusieurs radicaux $R^1$ ; où deux radicaux R peuvent également former ensemble un système cyclique;

Ar est à chaque fois identique ou différent un système cyclique aromatique ou hétéroaromatique avec 5 à 60 atomes de cycle aromatiques, qui peut être substitué par un ou plusieurs radicaux $R^1$, où deux radicaux Ar qui sont liés au même atome Si, N, ou B, peuvent également être pontés par une liaison simple ou un pont choisi parmi $B(R^1)$, $C(R^1)_2$, $Si(R^1)_2$, C=O, $C=NR^1$, $C=C(R^1)_2$, O, $N(R^1)$, $P(R^1)$ et $P(=O)R^1$;

$R^1$ est à chaque occurrence identique ou différente H, D, F, CN, $N(Ar^1)_2$, $N(R^2)_2$, $B(Ar^1)_2$, $B(R^2)_2$, $Si(Ar^1)_3$, $Si(R^2)_3$, un groupe alkyle, ou alcoxy à chaîne droite ayant de 1 à 40 atomes de carbone ou un groupe alkyle, ou alcoxy ramifié ou cyclique ayant de 3 à 40 atomes de carbone, qui peut être substitué dans chaque cas par un ou plusieurs radicaux $R^2$, un ou plusieurs atomes de H pouvant être remplacés par D, ou un système cyclique aromatique ou hétéroaromatique comportant 5 à 60 atomes cycliques aromatiques, qui peut être substitué dans chaque cas par un ou plusieurs radicaux $R^2$ ; deux ou plusieurs radicaux $R^1$, de préférence voisins, peuvent former ensemble un système cyclique ; un ou plusieurs radicaux $R^1$ peuvent former un système cyclique avec une autre partie du composé ;

$Ar^1$ est à chaque fois identique ou différent un système cyclique aromatique ou hétéroaromatique avec 5 à 30 atomes cycliques aromatiques, qui peut être substitué par un ou plusieurs radicaux non aromatiques $R^2$, où deux radicaux $Ar^1$, qui sont liés au même atome Si, N ou B, peuvent également être pontes ensemble par une liaison simple ou un pont choisi parmi $B(R^2)$, $C(R^2)_2$, $Si(R^2)_2$, C=O, $C=NR^2$, $C=C(R^2)_2$, O, $N(R^2)$, $P(R^2)$ et $P(=O)R^2$;

$R^2$ est choisi à chaque occurrence, de manière identique ou différente, dans le groupe constitué par H, D, F, CN, un radical hydrocarboné aliphatique comportant 1 à 20 atomes de carbone ou un système cyclique aromatique ou hétéroaromatique comportant 5 à 30 atomes cycliques aromatiques, dans lequel un ou plusieurs atomes de H peuvent être remplacés par D et qui peut être substitué par un ou plusieurs groupes alkyle comportant chacun 1 à 4 atomes de carbone, deux ou plusieurs substituants $R^2$, de préférence voisins, pouvant former ensemble un système cyclique.

2. Dispositif électronique selon la revendication 1, **caractérisé en ce que** le composé comprend au moins une structure de formules (IIa), (IIb), (IIc), (IId), (IIe) et/ou (IIf),

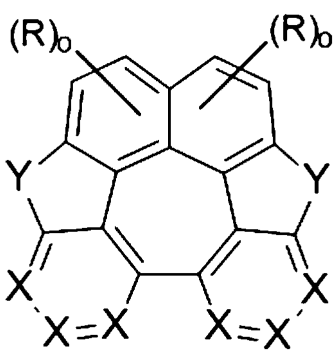

formule (IIa)

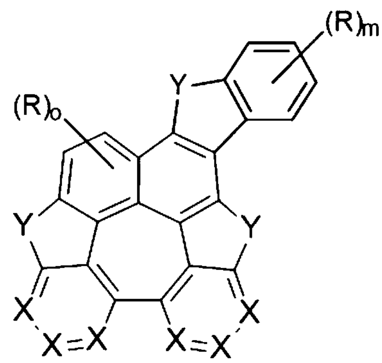

formule (IIb)

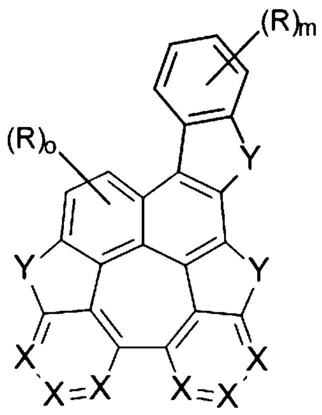

formule (IIc)

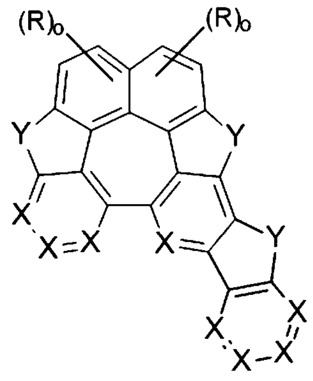

formule (IId)

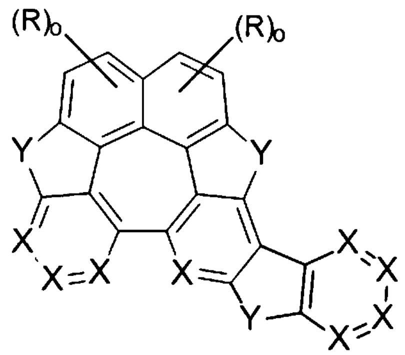

formule (IIe)

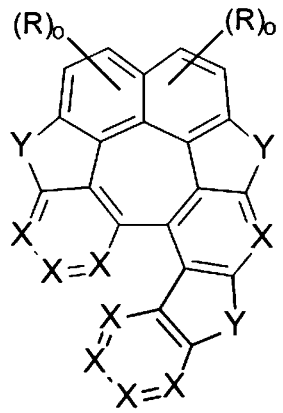

formule (IIf),

dans laquelle les radicaux X, Y et R ont la signification indiquée dans la revendication 1, l'indice m, identique ou différent, est égal à 0, 1, 2, 3 ou 4, de préférence à 0, 1, 2 ou 3, de préférence à 0, 1 ou 2, de manière préférée à 0 ou 1, et l'indice o, identique ou différent, est égal à 0, 1 ou 2, de préférence à 0 ou 1, la somme des indices o ou o et m étant de préférence égale à 1 ou 2, de manière préférée à 1.

**3.** Dispositif électronique selon la revendication 1 ou 2, **caractérisé en ce que** le composé comprend au moins une structure de formules (IIIa), (IIIb), (IIIc), (IIId), (IIIe) et/ou (IIIf),

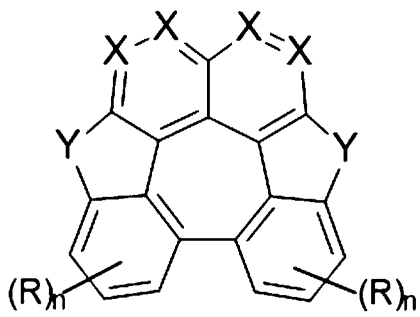

formule (IIIa)

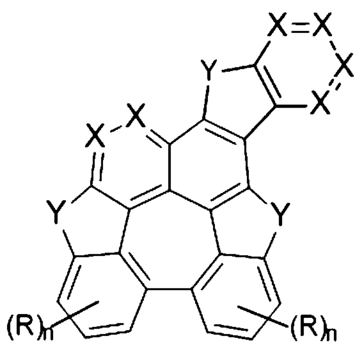

formule (IIIb)

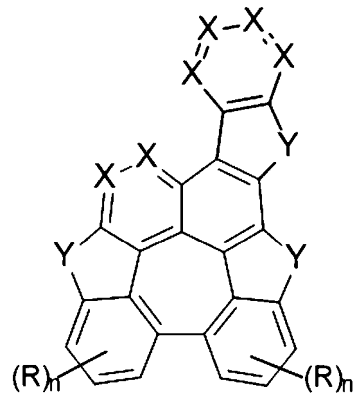

formule (IIIc)

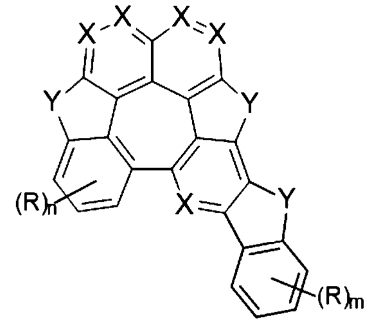

formule (IIId)

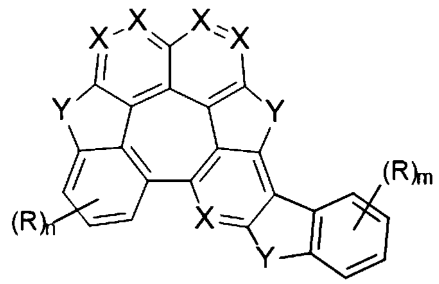

formule (IIIe)

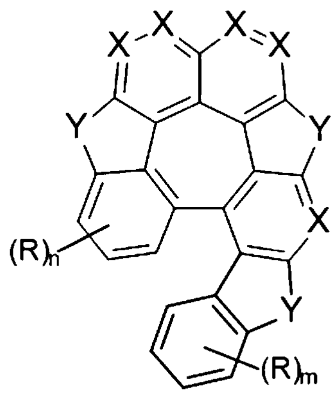

formule (IIIf),

dans laquelle les radicaux X, Y et R ont la signification indiquée dans la revendication 1, l'indice m, identique ou différent, est égal à 0, 1, 2, 3 ou 4, de préférence à 0, 1, 2 ou 3, de préférence à 0, 1 ou 2, de manière préférée à 0 ou 1, et l'indice n, identique ou différent, est égal à 0, 1, 2 ou 3, de préférence à 0, 1 ou 2, de manière préférée à 0 ou 1, la somme des indices n, respectivement n et m, étant de préférence égale à 1 ou 2, de manière préférée à 1.

4. Dispositif électronique selon une ou plusieurs des revendications précédentes, **caractérisé en ce que** le composé comprend au moins une structure de formule (IVa), (IVb), (IVc), (IVd), (IVe) et/ou (IVf),

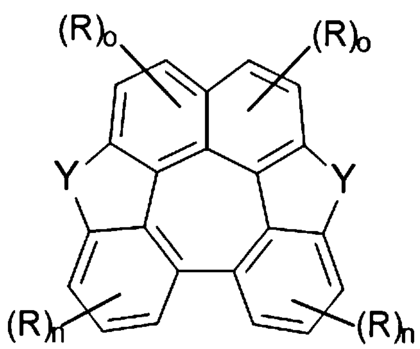

formule (IVa)

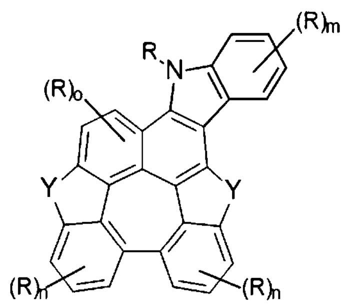

formule (IVb)

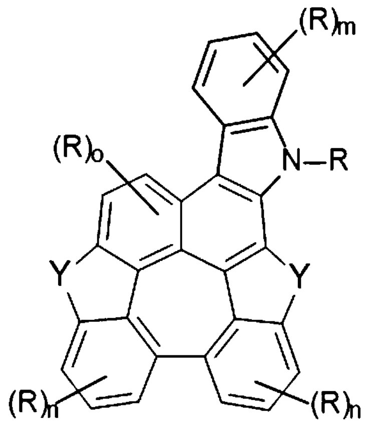

formule (IVc)

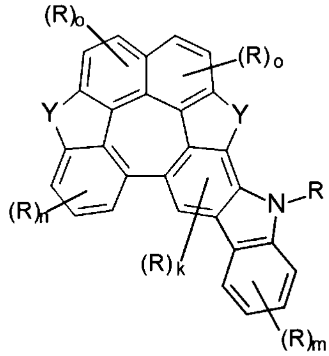

formule (IVd)

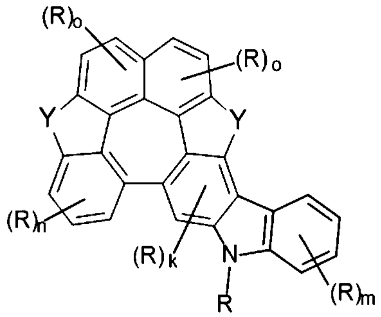

formule (IVe)

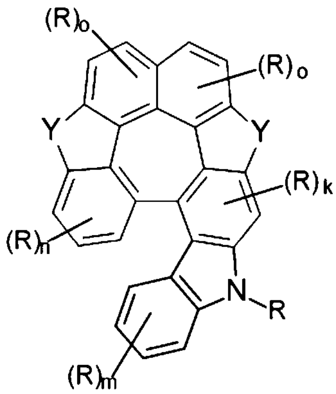

formule (IVf),

dans laquelle les radicaux Y et R sont tels que définis dans la revendication 1, l'indice k, identique ou différent, est 0 ou 1, de préférence 0, l'indice o, identique ou différent, est 0, 1 ou 2, de préférence 0 ou 1, l'indice n, identique ou différent, est 0, 1, 2 ou 3, de préférence 0, 1 ou 2, de préférence 0 ou 1, et l'indice m, identique ou différent, est 0, 1, 2, 3 ou 4, de préférence 0, 1, 2 ou 3, de préférence 0, 1 ou 2, de préférence 0 ou 1, dans lequel la somme des indices k, m, n et o est de préférence 1 ou 2, de préférence 1.

**5.** Dispositif électronique selon l'une ou plusieurs des revendications précédentes 2 à 4, **caractérisé en ce que** la somme des indices k, m, n et o est au plus égale à 6, de préférence au plus égale à 4, de manière particulièrement préférée au plus égale à 2, de préférence au moins égale à 1 et de manière particulièrement préférée exactement égale à 1.

**6.** Dispositif électronique selon une ou plusieurs des revendications précédentes, **caractérisé en ce que** le composé comprend au moins une structure de formules (Va), (Vb), (Vc), (Vd), (Ve), (Vf), (Vg), (Vh), (Vi), (Vj) et/ou (Vk),

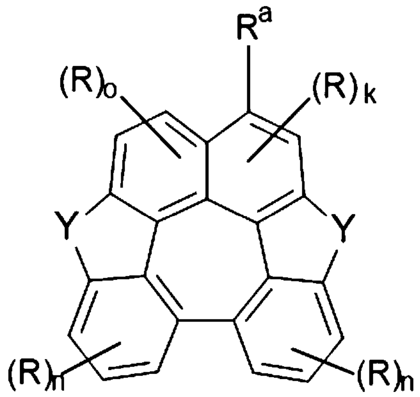

formule (Va)

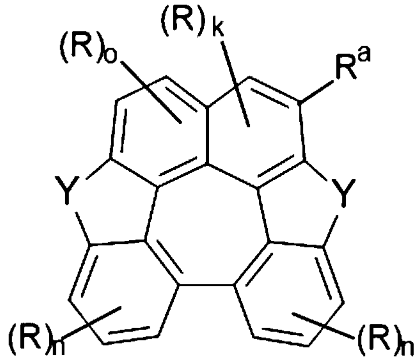

formule (Vb)

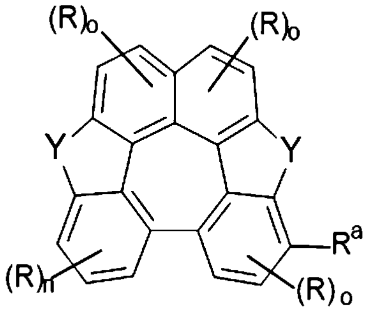

formule (Vc)

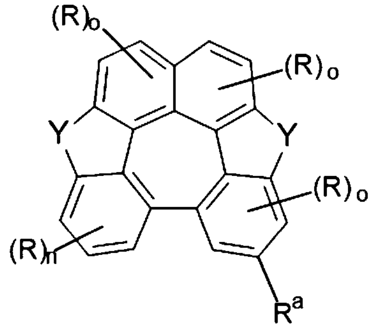

formule (Vd)

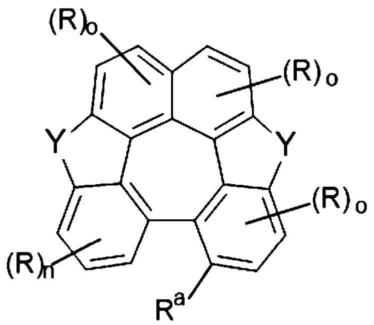

formule (Ve)

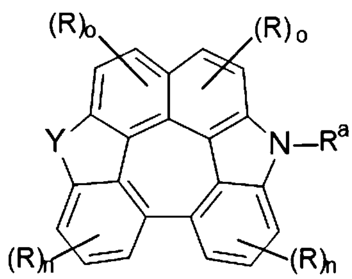

formule (Vf)

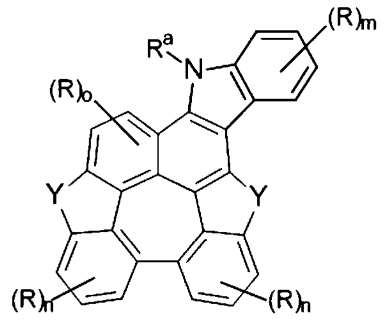

formule (Vg)

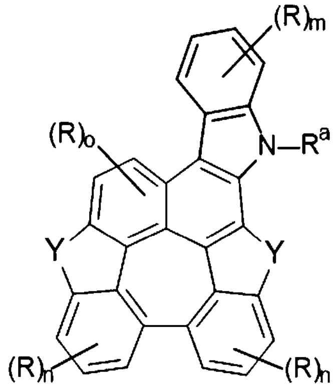

formule (Vh)

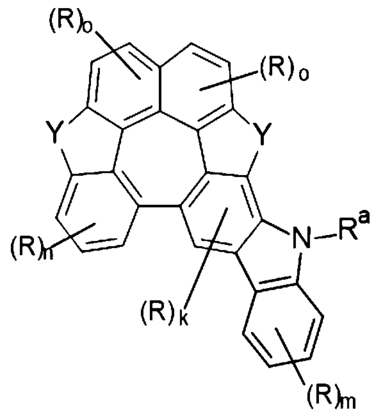

formule (Vi)

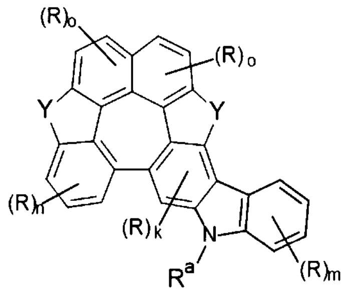

formule (Vj)

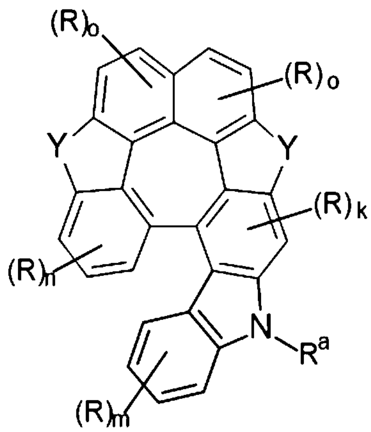

formule (Vk),

dans laquelle les radicaux Y et R ont la signification indiquée dans la revendication 1, et dans laquelle on a en outre :

$R^a$ est à chaque occurrence identique ou différente F, CN, $N(Ar)_2$, $N(R^1)_2$, $Si(Ar)_3$, $Si(R^1)_3$, $B(Ar)_2$, $B(R^1)_2$, un groupe alkyle ou alcoxy à chaîne droite ayant de 1 à 20 atomes de carbone ou un groupe alkyle ou alcoxy ramifié ou cyclique ayant de 3 à 40 atomes de carbone, dans lequel le groupe alkyle ou alcoxy peut être substitué dans chaque cas par un ou plusieurs radicaux $R^1$, ou un système cyclique aromatique ou hétéroaromatique

comportant 5 à 60 atomes cycliques aromatiques, qui peut être substitué dans chaque cas par un ou plusieurs radicaux $R^1$ ; les radicaux $R^a$ pouvant également former un système cyclique avec un radical R, dans lequel les radicaux Ar et $R^1$ ont la signification donnée dans la revendication 1 ;
k est identique ou différent de 0 ou 1, de préférence 0,
o est identique ou différent de 0, 1 ou 2, de préférence 0 ou 1 ;
n est identique ou différent de 0, 1, 2 ou 3, de préférence de 0, 1 ou 2, de préférence encore de 0 ou 1 ; et
m est identique ou différent de 0, 1, 2, 3 ou 4, de préférence de 0, 1 ou 2, de préférence encore de 0 ou 1.

**7.** Dispositif électronique selon la revendication 6, **caractérisé en ce que** la somme des indices k, m, n et o est au plus égale à 6, de préférence au plus égale à 4, de manière particulièrement préférée au plus égale à 2, de manière particulièrement préférée au plus égale à 1, et de manière tout à fait préférée à 0.

**8.** Dispositif électronique selon au moins l'une des revendications précédentes, **caractérisé en ce qu'**au moins l'un des radicaux R et/ou $R^a$ est choisi dans le groupe constitué par les fluorènes, les indénofluorènes, les spirobifluènes, les carbazoles, les indénocarbazoles, indolocarbazoles, spirocarbazoles, pyrimidines, triazines, lactames, triarylamines, dibenzofuranes, dibenzothiènes, imidazoles, benzimidazoles, benzoxazoles, benzothiazoles, 5-arylphénanthridine-6-ones, 9,10-déhydrophénanthrènes, fluoranthènes, anthracènes, benzanthracènes, fluoroadènes.

**9.** Dispositif électronique selon au moins l'une des revendications précédentes, **caractérisé en ce que** l'interconnexion comprend un groupe de transport de trous, de préférence l'un des groupes R et/ou $R^a$ comprenant un groupe de transport de trous.

**10.** Dispositif électronique selon l'une au moins des revendications précédentes, **caractérisé en ce que** l'un des groupes R et/ou $R^a$ représente un groupe de transport de trous.

**11.** Dispositif électronique selon au moins l'une des revendications précédentes, **caractérisé en ce que** l'un des groupes R et/ou $R^a$ représente un groupe de transport d'électrons.

**12.** Composé selon la formule (Ib), (Ic), (Id), (Ie) ou (If),

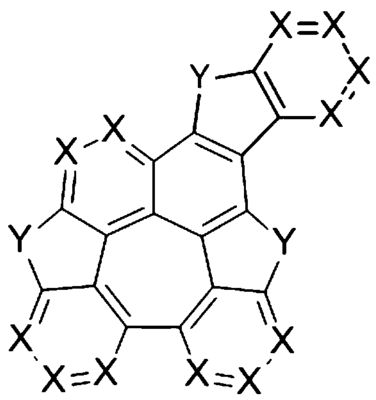

formule (Ib)

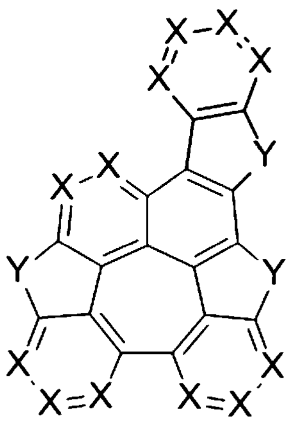

formule (Ic)

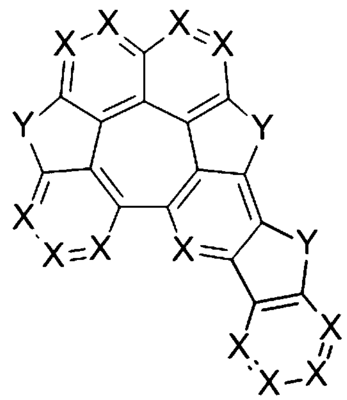

formule (Id)

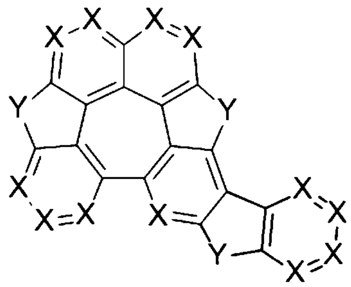

formule (Ie)

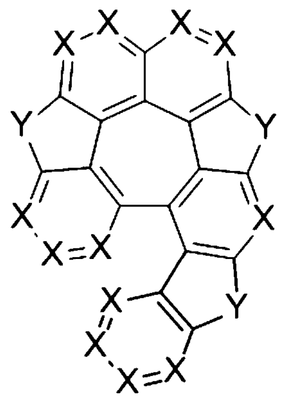

formule (If),

dans laquelle les radicaux X et Y ont la signification donnée dans la revendication 1.

**13.** Composé selon la formule (Ia),

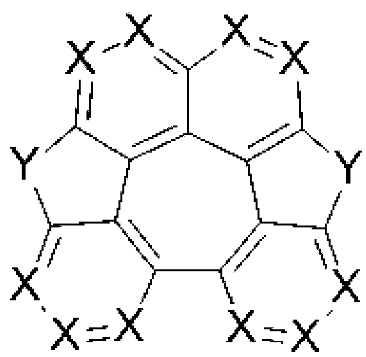

formule (Ia),

dans laquelle

X est, à chaque occurrence, identique ou différent de CR, dans lequel au maximum 2 des groupes CR que représente X ne sont pas identiques au groupe CH ;

Y est, à chaque occurrence, identique ou différent, un pont choisi parmi N(Ar), O, S, B(R) et C=O, de manière particulièrement préférée N(Ar), O et S;

R est, à chaque occurrence, identique ou différent, H, D, F, CN, $N(Ar)_2$, $N(R^1)_2$, $Si(Ar)_3$, $Si(R^1)_3$, $B(Ar)_2$, $B(R^1)_2$, un groupe alkyle ou alcoxy à chaîne droite ayant de 1 à 40 atomes de carbone ou un groupe alkyle ou alcoxy ramifié ou cyclique ayant de 3 à 20 atomes de carbone, dans lequel le groupe alkyle ou alcoxy peut être substitué dans chaque cas par un ou plusieurs radicaux $R^1$, ou un système cyclique aromatique ou hétéroaromatique comportant 5 à 60 atomes cycliques aromatiques, qui peut être substitué dans chaque cas par un ou plusieurs radicaux $R^1$ ;

Ar est à chaque occurrence identique ou différente un système cyclique aromatique ou hétéroaromatique avec 5 à 60 atomes cycliques aromatiques, qui peut être substitué par un ou plusieurs radicaux $R^1$, où deux radicaux Ar, qui sont liés au même atome Si, N ou B peuvent également être pontés ensemble par une liaison simple ou un pont choisi parmi $B(R^1)$, $C(R^1)_2$, $Si(R^1)_2$, C=O, $C=NR^1$, $C=C(R^1)_2$, O, $N(R^1)$, $P(R^1)$ et $P(=O)R^1$;

$R^1$ est à chaque occurrence identique ou différente H, D, F, CN, $N(Ar^1)_2$, $N(R^2)_2$, $B(Ar^1)_2$, $B(R^2)_2$, $Si(Ar^1)_3$, $Si(R^2)_3$, un groupe alkyle, ou alcoxy à chaîne droite ayant de 1 à 40 atomes de carbone ou un groupe alkyle, ou alcoxy ramifié ou cyclique ayant de 3 à 40 atomes de carbone, qui peut être substitué dans chaque cas par un ou plusieurs radicaux $R^2$, dans lesquels un ou plusieurs atomes de H peuvent être remplacés par D, ou un système cyclique aromatique ou hétéroaromatique comportant 5 à 60 atomes cycliques aromatiques, qui peut être substitué dans chaque cas par un ou plusieurs radicaux $R^2$ ;

$Ar^1$ est, à chaque occurrence, identique ou différente, un système cyclique aromatique ou hétéroaromatique comportant de 5 à 30 atomes cycliques aromatiques, qui peut être substitué par un ou plusieurs radicaux non aromatiques $R^2$, où deux radicaux $Ar^1$, qui sont liés au même atome Si, N ou B, peuvent également être pontés ensemble par une liaison simple ou un pont choisi parmi $B(R^2)$, $C(R^2)_2$, $Si(R^2)_2$, C=O, $C=NR^2$, $C=C(R^2)_2$, O, $N(R^2)$, $P(R^2)$ et $P(=O)R^2$;

$R^2$ est choisi à chaque occurrence, de manière identique ou différente, dans le groupe constitué par H, D, F, CN, un radical hydrocarboné aliphatique ayant de 1 à 20 atomes de carbone ou un système cyclique aromatique ou hétéroaromatique ayant de 5 à 30 atomes de cycle aromatique, dans lequel un ou plusieurs atomes de H peuvent être remplacés par D et qui peut être substitué par un ou plusieurs groupes alkyle ayant chacun de 1 à 4 atomes de carbone.

**14.** Oligomère, polymère ou dendrimère contenant un ou plusieurs composés selon la revendication 12 ou 13, dans lequel, au lieu d'un atome d'hydrogène ou d'un substituant, une ou plusieurs liaisons des composés sont présentes avec le polymère, l'oligomère ou le dendrimère.

**15.** Composition contenant au moins un composé selon la revendication 12 ou 13, ou un oligomère, un polymère ou un dendrimère selon la revendication 14, et au moins un autre composé choisi dans le groupe constitué par des émetteurs fluorescents, des émetteurs phosphorescents, des émetteurs présentant une TADF (thermally activated delayed fluorescence), des matériaux hôtes, des matériaux de transport d'électrons, des matériaux d'injection d'électrons, des matériaux de transport de trous, des matériaux d'injection de trous, des matériaux de blocage d'électrons et des matériaux de blocage de trous, des matériaux à large bande interdite et des n-dotands.

**16.** Formulation contenant au moins un composé selon la revendication 12 ou 13 ou un oligomère, polymère ou dendrimère selon la revendication 14 ou une composition selon la revendication 15 et au moins un solvant.

**17.** Utilisation d'un composé selon la revendication 12 ou 13, d'un oligomère, d'un polymère ou d'un dendrimère selon la revendication 14 ou d'une composition selon la revendication 15 dans un dispositif électronique en tant qu'émetteur, de préférence émetteur fluorescent, émetteur présentant une TADF (thermally activated delayed fluorescence), matériau hôte, matériau de transport d'électrons, matériau d'injection d'électrons, matériau de transport de trous, matériau d'injection de trous, matériau de blocage d'électrons, matériau de blocage de trous et/ou matériau à large bande interdite, de manière particulièrement préférée comme émetteur fluorescent (émetteur singulet), matériau hôte, matériau de transport de trous et/ou matériau de transport d'électrons.

**18.** Procédé de préparation d'un composé selon la revendication 12 ou 13 ou d'un oligomère, polymère et/ou dendrimère selon la revendication 14, **caractérisé en ce que**, dans une réaction de couplage, un composé comprenant une structure hétérocyclique est associé à un composé comprenant au moins un groupe aromatique ou hétéroaromatique.

**19.** Dispositif électronique selon une ou plusieurs des revendications 1 à 11, contenant au moins un composé selon la revendication 12 ou 13, un oligomère, un polymère ou un dendrimère selon la revendication 14 ou une composition

selon la revendication 15, dans lequel le dispositif électronique est choisi dans le groupe constitué par les dispositifs organiques électroluminescents, des circuits intégrés organiques, des transistors organiques à effet de champ, des transistors organiques à couches minces, des transistors organiques émettant de la lumière, des cellules solaires organiques, des détecteurs optiques organiques, des photorécepteurs organiques, des dispositifs organiques d'extinction de champ, des cellules électrochimiques émettant de la lumière ou des diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2010151006 A1
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- WO 2018011186 A
- WO 2018001990 A
- WO 2018019687 A
- WO 2018019688 A
- WO 2018041769 A
- WO 2018054798 A
- WO 2018069196 A
- WO 2018069197 A
- WO 2018069273 A
- WO 2018178001 A
- WO 2018177981 A
- WO 2019020538 A
- WO 2019115423 A
- WO 2019158453 A
- WO 2019179909 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- WO 2013041176 A
- WO 2007063754 A
- WO 2008056746 A
- WO 2010136109 A
- WO 2011000455 A
- WO 2013056776 A
- EP 1617710 A
- EP 1617711 A
- EP 1731584 A
- JP 2005347160 A
- WO 2007137725 A
- WO 2005111172 A
- WO 2006117052 A
- WO 2010015306 A
- WO 2011057706 A
- WO 2011060859 A
- WO 2011060877 A
- EP 652273 A
- WO 2009062578 A
- WO 2010054729 A
- WO 2010054730 A

- WO 2011042107 A
- WO 2011060867 A
- WO 2011088877 A
- WO 2012143080 A
- WO 2012048781 A
- WO 2015169412 A
- WO 2016015810 A
- WO 2016023608 A
- WO 2017148564 A
- WO 2017148565 A
- JP 3139321 B
- WO 2011116865 A
- WO 2011137951 A
- WO 2013064206 A
- WO 2014094963 A
- WO 2015192939 A
- WO 2010108579 A
- WO 2009124627 A
- WO 2005053051 A
- WO 2009030981 A
- WO 2004058911 A

**In der Beschreibung aufgeführte Nicht-Patentliteratur**

- **NATALIYA N KARAUSH-KARMAZIN et al.** *New Journal of Chemistry*, 2019, vol. 43 (30)
- **CARLOS A. MANZANO et al.** *Environmental Science & Technology*, 2017, vol. 51 (10)
- **D. M. KOLLER et al.** *Nature Photonics*, 2008, vol. 1-4
- **M. S. ARNOLD et al.** *Appl. Phys. Lett.*, 2008, vol. 92, 053301
- **S. H. SMITROVICH et al.** *Org. Lett*, 2004, vol. 6 (4), 533